(19) Europäisches Patentamt
European Patent Office
Office européen des brevets

(11) Veröffentlichungsnummer: **0 122 389**
**B1**

(12) # EUROPÄISCHE PATENTSCHRIFT

(45) Veröffentlichungstag der Patentschrift: **05.08.87**

(21) Anmeldenummer: **84101469.9**

(22) Anmeldetag: **13.02.84**

(51) Int. Cl.⁴: **C 07 C 121/00,** C 07 C 69/75, C 07 C 13/28, C 07 C 43/162, C 07 C 43/215, C 07 D 319/06, C 09 K 19/08, C 09 K 19/34, C 07 C 49/223, C 07 C 49/225 // C09K19/42, C07D317/72, C07D303/04, C07D303/14, C07D303/18, C07D303/16, C07C61/35

(54) **Flüssigkristallkomponenten mit einer Alkenylseitenkette.**

(30) Priorität: **16.03.83 CH 1436/83**
**22.03.83 CH 1539/83**
**19.01.84 CH 226/84**

(43) Veröffentlichungstag der Anmeldung:
**24.10.84 Patentblatt 84/43**

(45) Bekanntmachung des Hinweises auf die Patenterteilung:
**05.08.87 Patentblatt 87/32**

(84) Benannte Vertragsstaaten:
**CH DE GB LI**

(56) Entgegenhaltungen:
**EP - A - 0 014 885**
**DE - A - 3 006 666**

**CHEMICAL ABSTRACTS, Band 94, 1981, Seite 714, Zusammenfassung Nr. 139275c Columbus, Ohio, US K. PRAEFCKE et al.: "Liquid crystal compounds. VIII. 1,2-Bis (trans-4-alkylcyclohexyl)ethanes,-ethenes and ethynes - new liquid crystal compounds"**
**Mol. Cryst. Liq. Cryst., Band 94, 1983, Gordon and Breach, Science Publishers, Inc. US H. TAKATSU et al.: "Synthesis and Some Physical Properties of Phenyl Cyclohexyl Thylenes", Seiten 255-266**

(73) Patentinhaber: **F. HOFFMANN-LA ROCHE & CO. Aktiengesellschaft, CH-4002 Basel (CH)**

(72) Erfinder: **Petrzilka, Martin, Dr., Schwarzackerstrasse 54, Kaiseraugst (CH)**
Erfinder: **Schadt, Martin, Dr., Liestalerstrasse 77, Seltisberg (CH)**

(74) Vertreter: **Zimmermann, Hans, Dr. et al, Grenzacherstrasse 124 Postfach 3255, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 122 389 B1

ACTORUM AG

**Beschreibung**

Die vorliegende Erfindung betrifft neue Flüssigkristallkomponenten mit einer trans-1-Alkenyl- oder einer trans-3-Alkenyl-Seitenkette, nämlich die Verbindungen der allgemeinen Formel

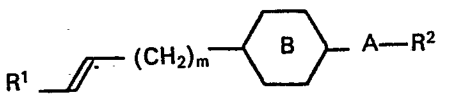

I

worin $R^1$ Wasserstoff oder geradkettiges Alkyl bedeutet; $R^2$–CN, –R, –COR, –COOR oder an einem aromatischen Ring auch –OR, –OOCR oder Fluor darstellt und R Alkyl bezeichnet;A für eine Gruppe mit 1 bis 4 sechsgliedrigen Ringen steht, wobei diese Ringe untereinander und mit Ring B je über eine einfache Kovalenzbindung direkt verknüpft oder an einer oder gegebenenfalls 2 Stellen auch über –COO–, –OOC– oder $–CH_2CH_2–$ verknüpft sind; die sechsgliedrigen Ringe in A und Ring B 1,4-Phenylen oder trans-1,4-Cyclohexylen bedeuten oder einer dieser Ringe auch einen trans-2,5-disubstituierten m-Dioxanring, einen 2,5-disubstituierten Pyrimidinring oder einen 3,6-disubstituierten Pyridazinring bedeutet, und von benachbarten trans-1,4-Cyclohexylen-Ringen höchstens je zwei über eine einfache Kovalenzbindung direkt verknüpft sind; und m die Zahl 2 oder, sofern Ring B trans-1,4-Cyclohexylen oder einen trans-2,5-disubstituierten m-Dioxanring bedeutet, auch die Zahl 0 bezeichnet.

Die Erfindung betrifft ferner die Herstellung der Verbindungen der obigen Formel I, flüssigkristalline Gemische, welche Verbindungen der obigen Formel I enthalten, sowie die Verwendung für elektrooptische Zwecke.

Der obige Ausdruck «Alkyl» umfasst geradkettige Alkylgruppen, wie Methyl, Äthyl, Propyl, Butyl, Pentyl, Hexyl, Heptyl, Octyl, Nonyl, Decyl, Undecyl, Dodecyl und dergleichen, sowie verzweigtkettige Gruppen, insbesondere 1-Methylalkyl und 2-Methylalkyl, wie Isopropyl, Isobutyl, sec-Butyl, 1-Methylbutyl, 2-Methylbutyl, 1-Methylpentyl, 2-Methylpentyl, 1-Methylhexyl, 2-Methylhexyl und dergleichen.

Ring B steht für 1,4-Phenylen, trans-1,4-Cyclohexylen, einen trans-2,5-disubstituierten m-Dioxanring, einen 2,5-disubstituierten Pyrimidinring oder einen 3,6-disubstituierten Pyridazinring. Gruppe A enthält 1 bis 4 sechsgliedrige Ringe. Die Ringe in Gruppe A können 1,4-Phenylen und/oder trans-1,4-Cyclohexylen bedeuten. Falls Ring B für 1,4-Phenylen oder trans-1,4-Cyclohexylen steht, kann einer der Ringe in Gruppe A auch einen trans-2,5-disubstituierten m-Dioxanring, einen 2,5-disubstituierten Pyrimidinring oder einen 3,6-disubstituierten Pyridazinring bedeuten. In Formel I gegebenenfalls vorhandene trans-1,4-Cyclohexylen-Ringe können höchstens je mit einem weiteren trans-1,4-Cyclohexyle-Ring über eine einfache Kovalenzbindung direkt verknüpft sein.

Der obige Ausdruck «aromatischer Ring» bezeichnet einen Benzol-, Pyrimidin- oder Pyridazinring. Der Ausdruck «heterocyclischer Ring» bedeutet im Rahmen der vorliegenden Erfindung einen Pyrimidin-, Pyridazin- oder m-Dioxanring.

Flüssige Kristalle haben in den letzten Jahren vor allem als Dielektrika in Anzeigevorrichtungen stark an Bedeutung gewonnen, da die optischen Eigenschaften solcher Substanzen durch eine angelegte Spannung beeinflusst werden können. Elektro-optische Vorrichtungen auf der Basis von Flüssigkristallen sind dem Fachmann gut bekannt und können auf verschiedenen Effekten beruhen, wie beispielsweise der dynamischen Streuung, der Deformation aufgerichteter Phasen (DAP-Typ), dem Schadt-Helfrich-Effekt (Drehzelle), dem Gast/Wirt-Effekt (Guest/Host-Effekt) oder einem cholesterisch-nematischen Phasenübergang (Phase-Change-Effekt).

Damit Flüssigkristalle als Dielektrika für elektrooptische Anzeigevorrichtungen geeignet sind, müssen sie jedoch einer Reihe von Anforderungen genügen. Beispielsweise müssen sie eine gute chemische Stabilität gegenüber Umwelteinflüssen, wie z.B. Wärme, Feuchtigkeit, Luft und elektromagnetische Strahlung im infraroten, sichtbaren und ultravioletten Bereich besitzen. Ferner sollten sie farblos sein, kurze Ansprechzeiten und nicht zu hohe Viskosität aufweisen, einen guten Kontrast ergeben und im ganzen Temperaturbereich, in welchem die Flüssigkristallzelle betrieben werden soll, eine nematische bzw. cholesterische Mesophase besitzen. Weitere Eigenschaften müssen je nach verwendetem Zellentyp unterschiedliche Bedingungen erfüllen, beispielsweise sollten Flüssigkristalle, die in Drehzellen verwendet werden, eine grosse positive Anisotropie der Dielektrizitätskonstante ($\Delta\varepsilon=\varepsilon_{\parallel}-\varepsilon_{\perp}$>, wobei $\varepsilon_{\parallel}$ die Dielektrizitätskonstante entlang der Moleküllängsachse und $\varepsilon_{\perp}$ die Dielektrizitätskonstante senkrecht dazu bedeuten), und Flüssigkristalle, die in Guest/Host-Zellen verwendet werden, eine grosse positive oder negative Anisotropie der Dielektrizitätskonstante aufweisen. In beiden Fällen ist zudem eine niedrige Schwellenspannung und ein möglichst geringe Leitfähigkeit erwünscht.

Da es im allgemeinen nicht möglich ist alle gewünschten und zum Teil widersprüchlichen Eigenschaften mit einer einzigen Verbindung zu erreichen, wird meist versucht, durch Mischen mehrerer Komponenten die Eigenschaften für die jeweiligen Anwendungen zu optimieren. Hierbei ist jedoch wichtig, dass die Komponenten untereinander keine chemischen Reaktionen eingehen und gut mischbar sind. Ferner sollten die gebildeten Mischungen mindestens bei Temperaturen, bei denen die Flüssigkristallzelle betrieben werden soll, keine smektischen Mesophasen aufweisen.

Es sind bereits eine Reihe von flüssigkristallinen Verbindungen und Dotierungsmitteln für Flüssigkristallmischungen bekannt, die als Flügelgruppen beispielsweise Alkyl-, Alkoxy-, Alkanoyloxy-, Alkoxycarbonyl- und Cyanogruppen aufweisen.

Es wurde nun gefunden, dass die erfindungsgemässen Verbindungen eine ausreichende Stabilität gegenüber elektromagnetischer Strahlung besitzen und dass die trans-1-Alkenyl- bzw. trans-3-Alkenyl-Seitenkette einen günstigen Einfluss auf das Mesophasenverhalten ausübt. Die erfindungsgemässen Verbindungen sind ferner gut mischbar mit bekannten Flüssigkristallen und besitzen auch die übrigen, oben genannten, erforderlichen Eigenschaften. Je nach Bedeutung des Ringes B und der Gruppe A und $R^2$ decken die erfindungsgemässen Verbindungen ein breites Spektrum verschiedenster elektro-optischer Anwendungsmöglichkeiten, Beispielsweise besitzen die Verbindungen, welche einen Pyrimidinring und/oder eine Cyanogruppe enthalten, eine positive Anistropie der Dielektrizitätskonstante ($\Delta\varepsilon=\varepsilon_{\parallel}-\varepsilon_{\perp}$, wobei $\varepsilon_{\parallel}$ die Dielektrizitätskonstante entlang der Moleküllängsachse und $\varepsilon$ die Dielektrizitätskonstante senkrecht dazu bedeuten), während ein Pyridazinring den Verbindungen eine negative dielektrische Anisotropie verleiht. Die Verbindungen, welche keine Cyanogruppe und keinen Pyrimidin- oder Pyridazinring enthalten, sind hingegen niederviskose Verbindungen mit kleinem Absolutbetrag der dielektrischen Anisotropie. Die Verbindungen, welche einen m-Dioxanring enthalten, sind vor allem zur Erzielung niedriger Schwellenspannungen und kurzer Ansprechzeiten von Interesse. Der erfindungsgemässen Verbindungen sind zum grössten Teil selbst flüssigkristallin und besitzen zum Teil sehr grosse Mesophasebereiche. Diejenigen erfindungsgemässen Verbindungen, welche nur monotrope oder virtuelle Klärpunkte besitzen, wie beispielsweise die Verbindungen der Formel I, worin A 1,4-Phenylen oder einen 3,6-disubstituierten Pyridazinring, Ring B trans-1,4-Cyclohexylen und $R^2$ Alkyl oder Alkoxy bedeuten, sind vor allem als Dotierungsmittel für Flüssigkristallmischungen geeignet. Durch die vorliegende Erfindung wird somit die Auswahl an geeigneten Flüssigkristallkomponenten beträchtlich erweitert, was inbesondere die weitere Optimierung von Mischungen erleichtert.

Eine bevorzugte Gruppe erfindungsgemässer Verbindungen sind die Verbindungen der allgemeinen Formel

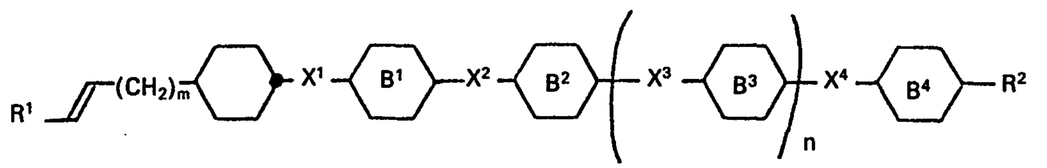

V

worin die Ringe $B^1$, $B^2$, $B^3$ und $B^4$ 1,4-Phenylen oder trans-1,4-Cyclohexylen bedeuten; n für die Zahl 0 oder 1 steht; $X^1$, $X^2$, $X^3$ und $X^4$ einfache Kovalenzbindungen oder eine oder zwei dieser Gruppen auch $-COO-$, $-OOC-$ oder $-CH_2CH_2-$ bezeichnen; von benachbarten trans-1,4-Cyclohexylen-Ringen höchstens je zwei über eine einfache Kovalenzbindung direkt verknüpft sind; und $R^1$, $R^2$, R und m die obigen Bedeutungen haben.

Vorzugsweise steht m in Formel V für die Zahl 0. Besonders bevorzugte Verbindungen der Formel V sind diejenigen, worin $X^1$ eine einfache Kovalenzbindung, $-COO-$ oder $-CH_2CH_2-$ bezeichnet, die Gruppen $X^2$, $X^3$ und $X^4$ einfache Kovalenzbindungen darstellen, die Ringe $B^1$, $B^2$ und $B^3$ für 1,4-Phenylen stehen und $R^2$ Cyano, Alkyl oder an einem aromatischen Ring auch Alkoxy bedeuten. Vorzugsweise steht ferner Ring $B^4$ für trans-1,4-Cyclohexylen, wenn $R^2$ Alkyl bedeutet, und für 1,4-Phenylen, wenn $R^2$ Cyano bedeutet.

Im Vordergrund des Interesses stehen jedoch im allgemeinen die bicyclischen und die tricyclischen Verbindungen, d.h. die Verbindungen der Formel I, worin Gruppe A einen oder zwei sechsgliedrige Ringe aufweist. Bevorzugte erfindungsgemässe Verbindungen sind daher die Verbindungen der allgemeinen Formel

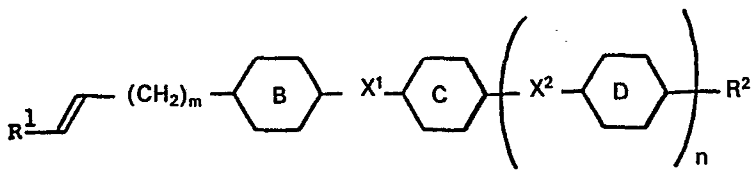

Ia

worin die Ringe B, C und D 1,4-Phenylen oder trans-1,4-Cyclohexylen bedeuten oder einer dieser Ringe auch einen trans-2,5-disubstituierten m-Dioxanring, einen 2,5-disubstituierten Pyrimidinring oder einen 3,6-disubstituierten Pyridazinring bedeutet; eines der Symbole $X^1$ und $X^2$ eine einfache Kovalenzbindung und das andere $-COO-$, $-OOC-$, $-CH_2CH_2-$ oder, sofern mindestens einer der Ringe B, C und D von trans-1,4-Cyclohexylen verschieden ist, auch eine einfache Kovalenzbindung bezeichnet; n für die Zahl 0 oder 1 steht; und $R^1$, $R^2$, R und m die obigen Bedeutungen haben.

Ring B in den Formeln I und Ia steht vorzugsweise für trans-1,4-Cyclohexylen oder einen trans-2,5-disubstituierten m-Dioxanring. In Formel Ia steht ferner n bevorzugt für die Zahl 0. m steht im allgemeinen bevorzugt für die Zahl 0, wenn Ring B trans-1,4-Cyclohexylen oder einen trans-2,5-disubstituierten m-Dioxanring bedeutet.

Bevorzugte, unter Formel I und Ia fallende Verbindungen sind die Verbindungen der allgemeinen Formel

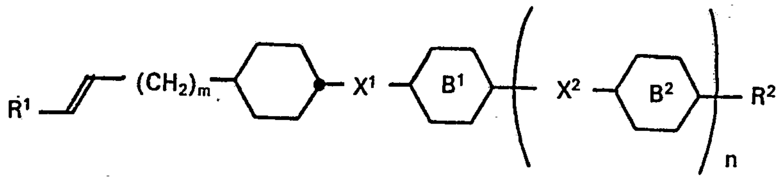
II

worin die Ringe $B^1$ und $B^2$ 1,4-Phenylen oder trans-1,4-Cyclohexylen bedeuten; eines der Symbole $X^1$ und $X^2$ eine einfache Kovalenzbindung und das andere $-COO-$, $-OOC-$, $-CH_2CH_2-$ oder, sofern mindestens einer der Ringe $B^1$ und $B^2$ von trans-1,4-Cyclohexylen verschieden ist, auch eine einfache Kovalenzbindung bezeichnet; n für die Zahl 0 oder 1 steht; und $R^1$, $R^2$, R und m die obigen Bedeutungen haben, die Verbindungen der allgemeinen Formel

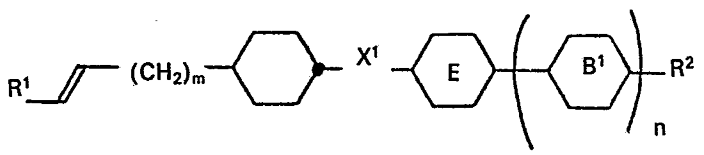
III

worin Ring E einen 2,5-disubstituierten Pyrimidinring und Ring $B^1$ 1,4-Phenylen oder trans-1,4-Cyclohexylen bedeutet; $X^1$ eine einfache Kovalenzbindung, $-COO-$, $-OOC-$ oder $-CH_2CH_2-$ bezeichnet; n für die Zahl 0 oder 1 steht; und $R^1$, $R^2$, R und m die obigen Bedeutungen haben, die Verbindungen der allgemeinen Formel

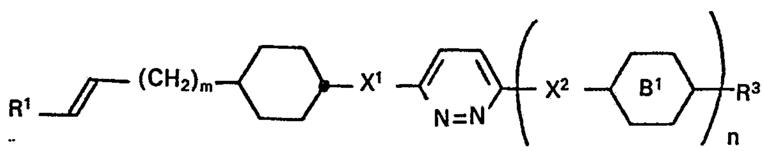
IV

worin Ring $B^1$ 1,4-Phenylen oder trans-1,4-Cyclohexylen bedeutet; $X^1$ und $X^2$ einfache Kovalenzbindungen oder eine dieser Gruppen auch $-COO-$, $-OOC-$ oder $-CH_2CH_2-$ bezeichnen; n für die Zahl 0 oder 1 steht; $R^3$ eine der Gruppen $-R$, $-COR$, $-COOR$ oder an einem aromatischen Ring auch $-OR$ oder $-OOCR$ darstellt; und $R^1$, R und m die obigen Bedeutungen haben, und die Verbindungen der allgemeinen Formel

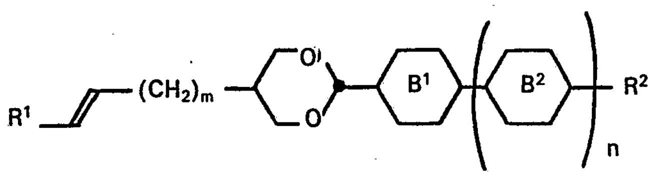
Ib

worin die Ringe $B^1$ und $B^2$ 1,4-Phenylen oder trans-1,4-Cyclohexylen bedeuten; n für die Zahl 0 oder 1 steht; und $R^1$, $R^2$, R und m die obigen Bedeutungen haben.

Besonders bevorzugte Verbindungen der Formel II sind diejenigen, worin $X^1$ eine einfache Kovalenzbindung, $-COO-$, $-OOC-$ oder $-CH_2CH_2-$ und $X^2$ eine einfache Kovalenzbindung bezeichnen, Ringe $B^1$ und $B^2$ 1,4-Phenylen darstellen und $R^2$ Cyano, Alkyl oder Alkoxy bedeutet. Vorzugsweise bedeutet ferner in Formel II n die Zahl 0 undoder $X^1$ eine einfache Kovalenzbindung.

Besonders bevorzugte Verbindungen der Formel III sind diejenigen, worin $X^1$ in Stellung 5 des Pyrimidinringes E steht und eine einfache Kovalenzbindung bezeichnet. Bevorzugte Reste $R^2$ sind Alkyl, Alkoxy und insbesondere Cyano. Vorzugsweise steht n für die Zahl 1 und Ring $B^1$ für 1,4-Phenylen.

Besonders bevorzugte Verbindungen der Formel IV sind diejenigen, worin $X^1$ und $X^2$ einfache Kovalenzbindungen oder eine dieser Gruppen auch $-CH_2CH_2-$ bezeichnen und $R^3$ Alkyl oder an einem aromatischen Ring auch Alkoxy bedeuten und insbesondere diejenigen, worin $X^1$ eine einfache Kovalenzbindung und n die Zahl 0 bezeichnen.

Besonders bevorzugte Verbindungen der Formel Ib sind diejenigen, worin n die Zahl 0 und $R^2$ Cyano, Alkyl oder an einem aromatischen Ring auch Alkoxy bezeichnen. Vorzugsweise steht Ring $B^1$ für 1,4-Phenylen.

Rest R in obigen Definitionen von $R^2$ und $R^3$ umfasst zweckmässig Alkylreste mit 1 bis 12 Kohlenstoffatomen, insbesondere geradkettige Alkylreste mit 1 bis 12 Kohlenstoffatomen.

Ferner sind grundsätzlich diejenigen Verbindungen der Formel I bevorzugt, worin $R^2$ Cyano, Alkyl oder an einem aromatischen Ring auch Alkoxy bedeutet. Bevorzugte Alkyl- und Alkanoylgruppen $R^2$ bzw. $R^3$ sind diejenigen mit 3 bis 7 Kohlenstoffatomen und bevorzugte Alkoxy-, Alkanoyloxy- und Alkoxycarbonylgruppen $R^2$ bzw. $R^3$ sind diejenigen mit 2 bis 6 Kohlenstoffatomen.

Rest $R^1$ in den obigen Formeln I, Ia, Ib, II, III, IV und V bedeutet vorzugsweise Wasserstoff oder geradkettiges $C_1$–$C_{10}$-Alkyl, insbesondere Wasserstoff oder geradkettiges $C_1$–$C_5$-Alkyl, wenn m für die Zahl 0 steht, und vorzugsweise Wasserstoff oder geradkettiges $C_1$–$C_8$-Alkyl, insbesondere Wasserstoff oder geradkettiges $C_1$–$C_3$-Alkyl, wenn m für die Zahl 2 steht. Im Rahmen der vorliegenden Erfindung umfasst daher der Ausdruck «trans-1-Alkenyl» insbesondere die Gruppen Vinyl, trans-1-Propenyl, trans-1-Butenyl, trans-1-Pentenyl, trans-1-Hexenyl, trans-1-Heptenyl, trans-1-Octenyl, trans-1-Nonenyl, trans-1-Decenyl, trans-1-Undecenyl und trans-1-Dodecenyl und der Ausdruck «trans-3-Alkenyl» umfasst insbesondere die Gruppen 3-Butenyl, trans-3-Pentenyl, trans-3-Hexenyl, trans-3-Heptenyl, trans-3-Octenyl, trans-3-Nonenyl, trans-3-Decenyl, trans-3-Undecenyl und trans-3-Dodecenyl.

Weitere, bevorzugte Gruppen erfindungsgemässer Verbindungen und Beispiele bevorzugter Verbindungen sind die in den Reaktionsschemata und den Synthesebeispielen erwähnten, unter Formel I fallenden Verbindungen.

Die Verbindungen der Formel I können erfindungsgemäss dadurch hergestellt werden, dass man

a) zur Herstellung der Verbindungen der Formel I, worin $R^1$ primäres Alkyl mit mindestens 2 Kohlenstoffatomen bedeutet, $R^2$ –CN, –R oder an einem aromatischen Ring auch –OR oder Fluor darstellt und die in A vorkommenden Ringe untereinander und mit Ring B je über eine einfache Kovalenzbindung direkt verknüpft oder an einer oder gegebenenfalls 2 Stellen auch über $-CH_2CH_2-$ verknüpft sind, eine Verbindung der allgemeinen Formel

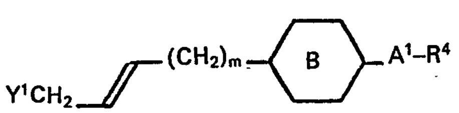
VI

worin $Y^1$ eine Abgangsgruppe bezeichnet; $R^4$ –CN, –R oder an einem aromatischen Ring auch –OR oder Fluor darstellt und R Alkyl bedeutet; $A^1$ für eine Gruppe mit 1 bis 4 sechsgliedrigen Ringen steht, wobei diese Ringe untereinander und mit Ring B je über eine einfache Kovalenzbindung direkt verknüpft oder an einer oder gegebenenfalls 2 Stellen auch über $-CH_2CH_2-$ verknüpft sind; die sechsgliedrigen Ringe in A und Ring B 1,4-Phenylen oder trans-1,4-Cyclohexylen bedeuten oder einer dieser Ringe auch einen trans-2,5-disubstituierten m-Dioxanring, einen 2,5-disubstituierten Pyrimidinring oder einen 3,6-disubstituierten Pyridazinring bedeutet, und von benachbarten trans-1,4-Cyclohexylen-Ringen höchstens je zwei über eine einfache Kovalenzbindung direkt verknüpft sind; und m die Zahl 2 oder, sofern Ring B trans-1,4-Cyclohexylen oder einen trans-2,5-disubstituierten m-Dioxanring bedeutet, auch die Zahl 0 bezeichnet, in Gegenwart von Dilithiumtetrachlorkuprat oder Dilithiumtetrabromkuprat mit Alkylmagnesiumhalogenid umsetzt, oder

b) zur Herstellung der Verbindungen der Formel I, worin $R^1$ Methyl bedeutet, $R^2$ –CN, –R oder an einem aromatischen Ring auch –OR oder Fluor darstellt und die in A vorkommenden Ringe untereinander und mit Ring B je über eine einfache Kovalenzbindung direkt verknüpft oder an einer oder gegebenenfalls 2 Stellen auch über $-CH_2CH_2-$ verknüpft sind, eine Verbindung der Formel VI reduziert, oder

c) zur Herstellung der Verbindungen der Formel I, worin $R^2$ –COR darstellt und die in A vorkommenden Ringe untereinander und mit Ring B je über eine einfache Kovalenzbindung direkt verknüpft oder an einer oder gegebenenfalls 2 Stellen auch über $-CH_2CH_2-$ verknüpft sind, eine Verbindung der allgemeinen Formel

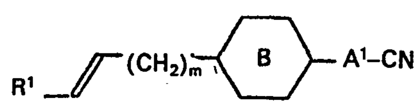
VII

worin $R^1$ Wasserstoff oder geradkettiges Alkyl bezeichnet und $A^1$, m und Ring B die in Formel VI gegebenen Bedeutungen haben, in Gegenwart von Dilithiumtetrachlorkuprat oder Dilithiumtetrabromkuprat mit Alkylmagnesiumhalogenid umsetzt, oder

d) zur Herstellung der Verbindungen der Formel I, worin A mindestens eine Gruppe –COO– oder –OOC– enthält und/oder $R^2$ –COOR oder –OOCR darstellt, eine Verbindung der allgemeinen Formel

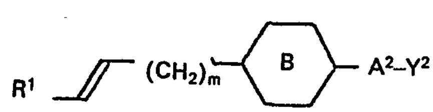
VIII

mit einer Verbindung der allgemeinen Formel

$Y^2$–$A^3$–$R^5$ IX

worin $R^1$ Wasserstoff oder geradkettiges Alkyl bedeutet; eine der Gruppen $Y^2$ und $Y^3$ –COOH oder –COCl und die andere –OH darstellt; $A^2$ und $A^3$ je

für eine Gruppe mit 1 bis 4 sechsgliedrigen Ringen stehen oder eine der Gruppen $A^2$ und $A^3$ auch fehlen kann, mit der Massgabe, dass $A^2$ und $A^3$ zusammen höchstens 4 Ringe enthalten; Ring B gegebenenfalls mit in $A^2$ vorhandenen sechsgliedrigen Ringen und, sofern $A^2$ und/oder $A^3$ mehrere sechsgliedrige Ringe aufweisen, diese Ringe untereinander je über eine einfache Kovalenzbindung direkt verknüpft sind oder eine oder, sofern $A^3$ fehlt, gegebenenfalls auch 2 dieser Verknüpfungen über $-COO-$, $-OOC$ oder $-CH_2CH_2-$ erfolgen; die sechsgliedrigen Ringe in $A^2$ und $A^3$ und Ring B 1,4-Phenylen oder trans-1,4-Cyclohexylen bedeuten oder einer dieser Ringe auch einen trans-2,5-disubstituierten m-Dioxanring, einen 2,5-disubstituierten Pyrimidinring oder einen 3,6-disubstituierten Pyridazinring bedeutet, und von benachbarten trans-1,4-Cyclohexylen-Ringen höchstens je zwei über eine einfache Kovalenzbindung direkt verknüpft sind; m die Zahl 2 oder, sofern Ring B trans-1,4-Cyclohexylen oder einen trans-2,5-disubstituierten m-Dioxanring bedeutet auch die Zahl 0 bezeichnet; und $R^5$, wenn $A^3$ für eine Gruppe mit 1 bis 4 sechsgliedrigen Ringen steht, $-CN$, $-R$, $-COR$, $-COOR$ oder an einem aromatischen Ring auch $-OR$, $-OOCR$ oder Fluor darstellt oder $R^5$, wenn $A^3$ fehlt, $-R$ darstellt und R Alkyl bezeichnet, verestert, oder

e) zur Herstellung der Verbindungen der Formel I, worin $R^2$ $-CN$, $-R$ oder an einem aromatischen Ring auch $-OR$ oder Fluor darstellt und die in A vorkommenden Ringe untereinander und mit Ring B je über eine einfache Kovalenzbindung direkt verknüpft oder an einer oder gegebenenfalls 2 Stellen auch über $-CH_2CH_2-$ verknüpft sind, eine Verbindung der allgemeinen Formel

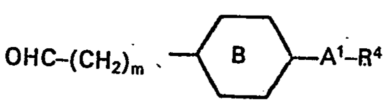

X

worin $A^1$, $R^4$, m und Ring B die in Formel VI gegebenen Bedeutungen haben, in Gegenwart einer Base mit Alkyltriphenylphosphoniumhalogenid umsetzt, oder

f) zur Herstellung der Verbindungen der Formel I, worin Ring B oder einer der sechsgliedrigen Ringe in A einen trans-2,5-disubstituierten m-Dioxanring bedeutet, eine Verbindung der allgemeinen Formel

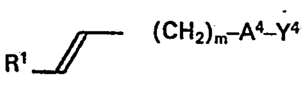

XXXV

mit einer Verbindung der allgemeinen Formel

$$Y^5-A^5-R^2 \qquad XXXVI$$

worin $R^1$ Wasserstoff oder geradkettiges Alkyl bedeutet; $R^2$ $-CN$, $-R$, $-COR$, $-COOR$ oder an einem aromatischen Ring auch $-OR$, $-OOCR$ oder Fluor darstellt und R Alkyl bezeichnet; eine der Gruppen $Y^4$ und $Y^5$ $-CH(CH_2OH)_2$ und die andere $-CHO$ oder eine Acetalgruppe bedeutet; $A^4$ und $A^5$ je für eine Gruppe mit 1 bis 4 1,4-Phenylen- oder trans-1,4-Cyclohexylen-Ringen stehen oder eine der Gruppen $A^4$ und $A^5$ auch fehlen kann, mit der Massgabe, dass $A^4$ und $A^5$ zusammen höchstens 4 Ringe enthalten; $Y^4$ und $Y^5$ gegebenenfalls mit in $A^4$ bzw. in $A^5$ vorhandenen sechsgliedrigen Ringen sind, sofern $A^4$ und/oder $A^5$ mehrere sechsgliedrige Ringe aufweisen, diese Ringe untereinander je über eine einfache Kovalenzbindung direkt verknüpft sind oder eine oder 2 dieser Verknüpfungen über $-COO-$, $-OOC-$ oder $-CH_2CH_2-$ erfolgen; von benachbarten trans-1,4-Cyclohexylen-Ringen höchstens je zwei über eine einfache Kovalenzbindung direkt verknüpft sind; und m die Zahl 2 bedeutet oder, wenn $A^4$ fehlt oder die Gruppe $R^1-CH=CH-(CH_2)_m-$ an einem trans-1,4-Cyclohexylen-Ring steht, auch die Zahl 0 bedeutet, umsetzt.

Die Umsetzung einer Verbindung der Formel VI mit Alkylmagnesiumhalogenid (Verfahrensvariante a) kann in an sich bekannter Weise durchgeführt werden. Der Ausdruck «Halogenid» steht hierbei für Chlorid, Bromid oder Jodid, vorzugsweise für Bromid. Bevorzugter Katalysator ist Dilithiumtetrachlorokuprat. $Y^1$ bedeutet irgendeine in derartigen Reaktionen übliche Abgangsgruppe, beispielsweise Alkanoyloxy, Alkylsulfonyloxy, Arylsulfonyloxy oder Halogen, wie Acetoxy, Methansulfonyloxy, Benzolsulfonyloxy, Tosyloxy, Naphthalinsulfonyloxy, Brom, Jod und dergleichen. Die Umsetzung wird zweckmässig in einem Äther, wie Dimethoxyäthan, Diäthylenglykoldimethyläther, Tetrahydrofuran, Dioxan, Diäthyläther und dergleichen, ausgeführt. Bevorzugtes Lösungsmittel ist Tetrahydrofuran oder ein Gemisch von Tetrahyforuan und Diäthyläther. Temperatur und Druck können in weiten Grenzen gehalten werden. Im allgemeinen werden jedoch Atmosphärendruck und eine Temperatur zwischen etwa −80 °C und Raumtemperatur angewendet. Bevorzugt ist eine Reaktionstemperatur von unter etwa 0 °C und besonders bevorzugt etwa −15 °C.

Die Reduktion einer Verbindung der Formel VI gemäss Verfahrensvariante b) kann nach an sich bekannten Methoden erfolgen, beispielsweise durch Umsetzung einer Verbindung der Formel VI, worin $Y^1$ Jod bedeutet, mit Triphenylphosphoniumjodid und durch Umsetzung einer Verbindung der Formel VI, worin $Y^1$ Acetoxy bedeutet, mit methanolischer Kaliumhydroxid-Lösung und Triphenylphosphin-Jod. Derartige Reaktion sind z.B. in Chem. Ber. 109, 1586 (1976) beschrieben.

Die Umsetzung einer Verbindung der Formel VII mit Alkylmagnesiumhalogenid (Verfahrensvariante c) kann in analoger Weise zu Verfahrensvariante a) durchgeführt werden. Zweckmässigerweise wird jedoch im allgemeinen bei etwas höherer Temperatur, vorzugsweise bei etwa 0 °C bis Rückflusstemperatur gearbeitet.

Die Veresterung einer Verbindung der Formel VIII mit einer Verbindung der Formel IX (Verfahrensvariante d) kann in an sich bekannter Weise erfolgen. Die Veresterung der Säurechloride (welche aus den Carbonsäuren z.B. durch Erhitzen mit Thionylchlorid erhältlich sind) kann beispielsweise in Diäthyläther, Tetrahydrofuran, Dimethylformamid, Benzol, Toluol, Tetrachlorkohlenstoff, Pyridin und dergleichen erfolgen. Die Veresterung einer Carbonsäure erfolgt vorzugsweise in Gegenwart von 4-(Dimethylamino)pyridin und N,N'-Dicyclohexylcarbodiimid oder in Gegenwart von Oxalylchlorid und Dimethylformamid. Temperatur und Druck dieser Veresterungsreaktionen sind nicht kritisch und im allgemeinen werden Atmosphärendruck und eine Temperatur zwischen etwa −30 °C und der Siedetemperatur des Reaktionsgemisches angewendet.

Die Umsetzung einer Verbindung der Formel X mit Alkyltriphenylphosphoniumhalogenid in Gegenwart einer Base, z.B. Kaliumcarbonat, (Verfahrensvariante e) kann ebenfalls in an sich bekannter Weise erfolgen. Der Ausdruck «Halogenid» steht hier für Chlorid, Bromid oder Jodid, vorzugsweise für Bromid. Die Reaktion wird zweckmässig in einem inerten organischen Lösungsmittel, beispielsweise einem Äther, wie Diäthyläther, Tetrahydrofuran, Dioxan und dergleichen durchgeführt. Temperatur und Druck sind nicht kritisch; im allgemeinen wird jedoch bei Atmosphärendruck und einer Temperatur von Raumtemperatur bis Rückflusstemperatur gearbeitet.

Die Umsetzung einer Verbindung der Formel XXXV mit einer Verbindung der Formel XXXVI (Verfahrensvariante f) kann in an sich bekannter Weise erfolgen. Zweckmässigerweise erfolgt die Umsetzung des Diols mit dem Aldehyd oder einem geeigneten Acetal hiervon (z.B. Dimethylacetal) in einem inerten organischen Lösungsmittel in Gegenwart einer katalytischen Menge einer organischen oder anorganischen Säure. Bevorzugte Lösungsmittel sind Kohlenwasserstoffe, insbesondere aromatische Kohlenwasserstoffe, wie Benzol, Toluol, Xylol und dergleichen. Bevorzugte Säuren sind trockener Chlorwasserstoff und Sulfonsäuren, insbesondere p-Toluolsulfonsäure. Temperatur und Druck sind nicht kritisch, vorzugsweise wird jedoch bei Rückflusstemperatur und Atmosphärendruck gearbeitet. Die Dioxane werden im allgemeinen als cis/trans-Gemische erhalten, aus denen die reinen trans-Verbindungen leicht durch Umkristallisation gewonnen werden können. Gewünschtenfalls können die Mutterlaugen mit Säure wieder in das cis/trans-Gleichgewichtsgemisch übergeführt und erneut umkristallisiert werden.

Bei der Herstellung von Verbindungen der Formel I, worin m die Zahl 0 und Ring B trans-1,4-Cyclohexylen bedeuten, nach Verfahrensvariante e) wird der Cyclohexanring fast ausschliesslich (ca. 98%) in trans-Konfiguration erhalten, auch wenn von einem cis/trans-Isomerengemisch (Verbindung der Formel X, worin m Zahl 0 und Ring B cis-1,4-Cyclohexylen oder trans-1,4-Cyclohexylen bedeuten) ausgegangen wird. Dieses Verfahren bietet somit auch eine ausgezeichnete Möglichkeit, um durch anschliessende katalytische Hydrierung in an sich bekannter Weise, z.B. mit Palladium, Platin oder Raney-Nickel, entsprechende trans-4-Alkylcyclohexyl-Verbindungen herzustellen.

Die Verbindungen der Formeln IX und XXXVI sind bekannte oder Analoge bekannter Verbindungen.

Die Verbindungen der Formel VI, VIII, X und XXXV sind neu. Die Herstellung der Verbindungen der Formeln I, VI, VII, VIII, X und XXXV wird anhand repräsentativer Beispiele in den folgenden Reaktionsschemata 1–11 veranschaulicht, worin $R^6$ geradkettiges Alkyl, $R^7$ Wasserstoff oder geradkettiges Alkyl, Ts p-Tosyl und Ring $B^1$ 1,4-Phenylen oder trans-1,4-Cyclohexylen bezeichnen und $R^1$, $R^4$ und R die obigen Bedeutungen haben. Die hierbei benötigten Ausgangsmaterialien sind bekannte oder Analoge bekannter Verbindungen.

Schema 1

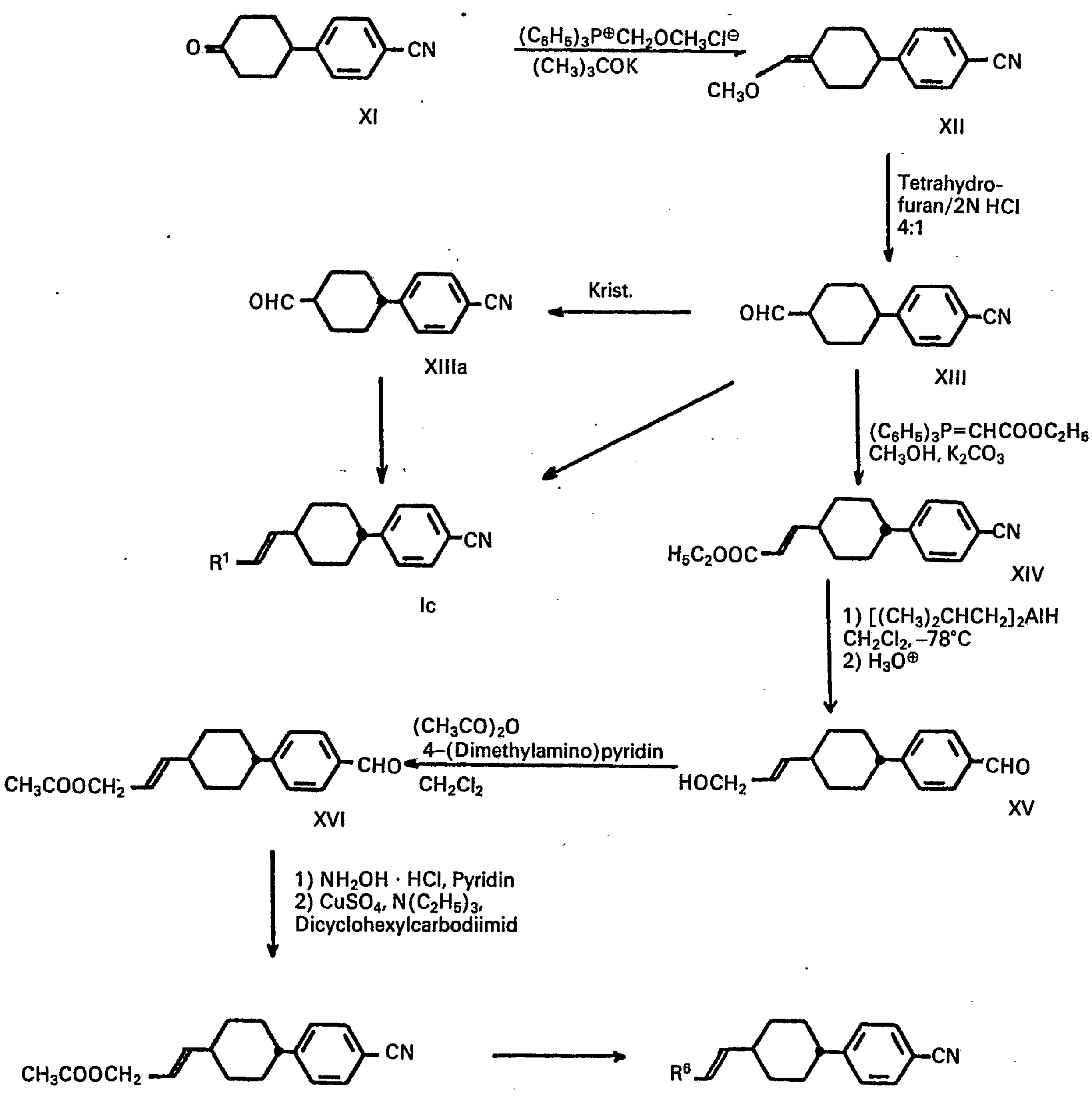


XI, XII, XIII, XIIIa, Ic, XIV, XV, XVI, XVII, Id

Schema 2

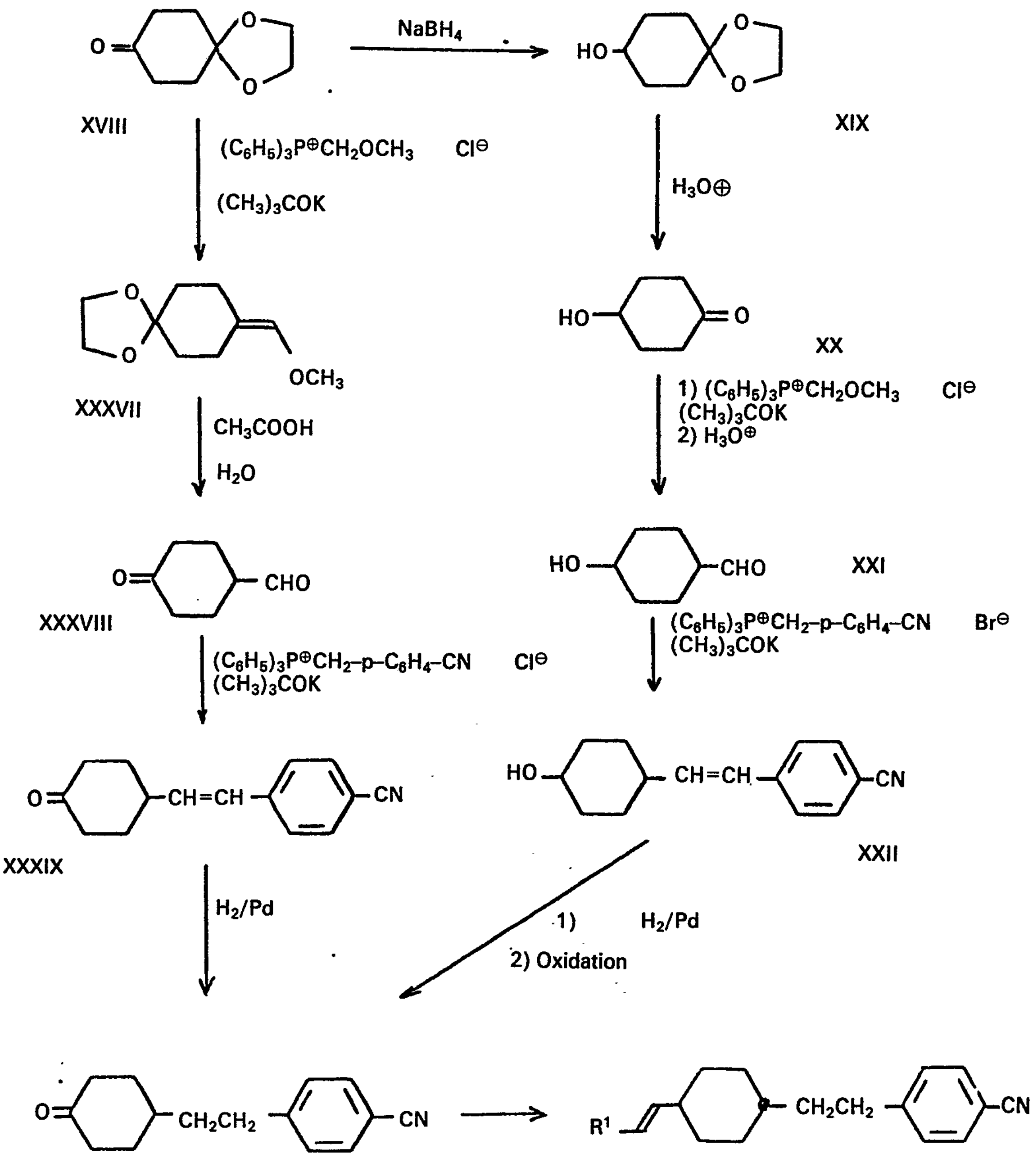


XVIII XIX XXXVII XX XXXVIII XXI XXXIX XXII XXIII Ie

Schema 3

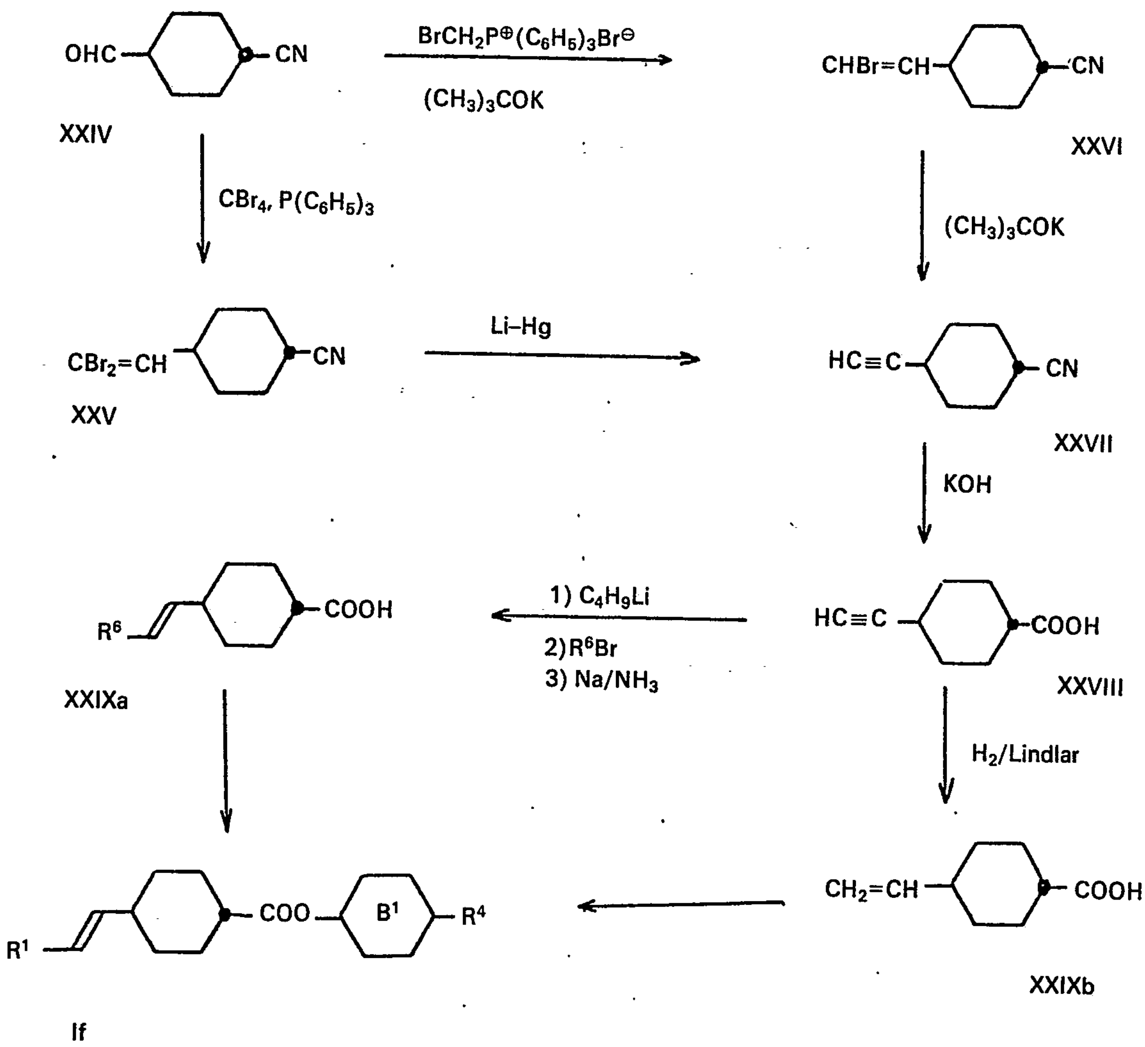
BrCH$_2$P$^{\oplus}$(C$_6$H$_5$)$_3$Br$^{\ominus}$
(CH$_3$)$_3$COK
CBr$_4$, P(C$_6$H$_5$)$_3$
(CH$_3$)$_3$COK
Li–Hg
KOH
1) C$_4$H$_9$Li
2) R$^6$Br
3) Na/NH$_3$
H$_2$/Lindlar

Schema 4

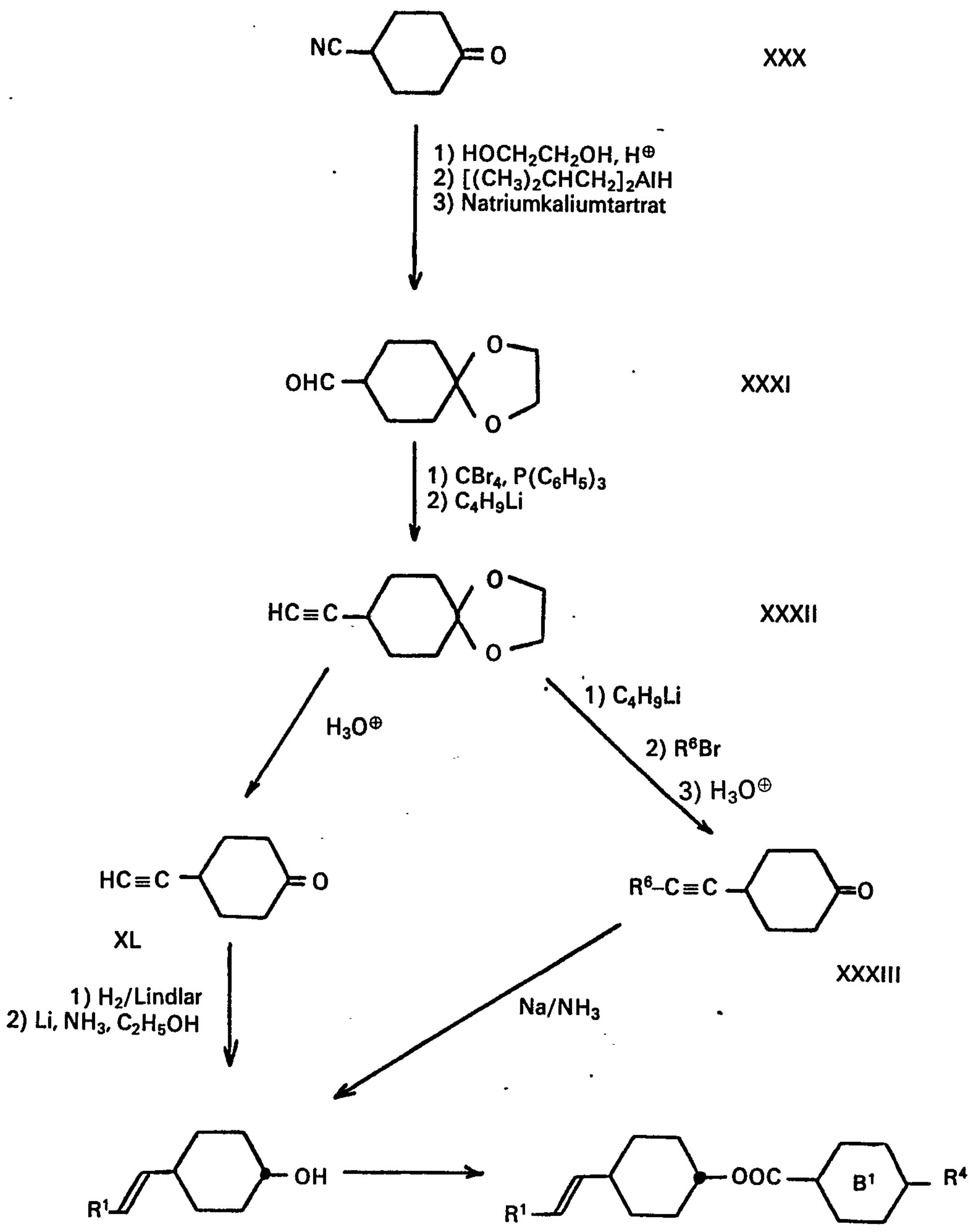


XXX

XXXI

XXXII

XL

XXXIII

XXXIV

Ig

Schema 5

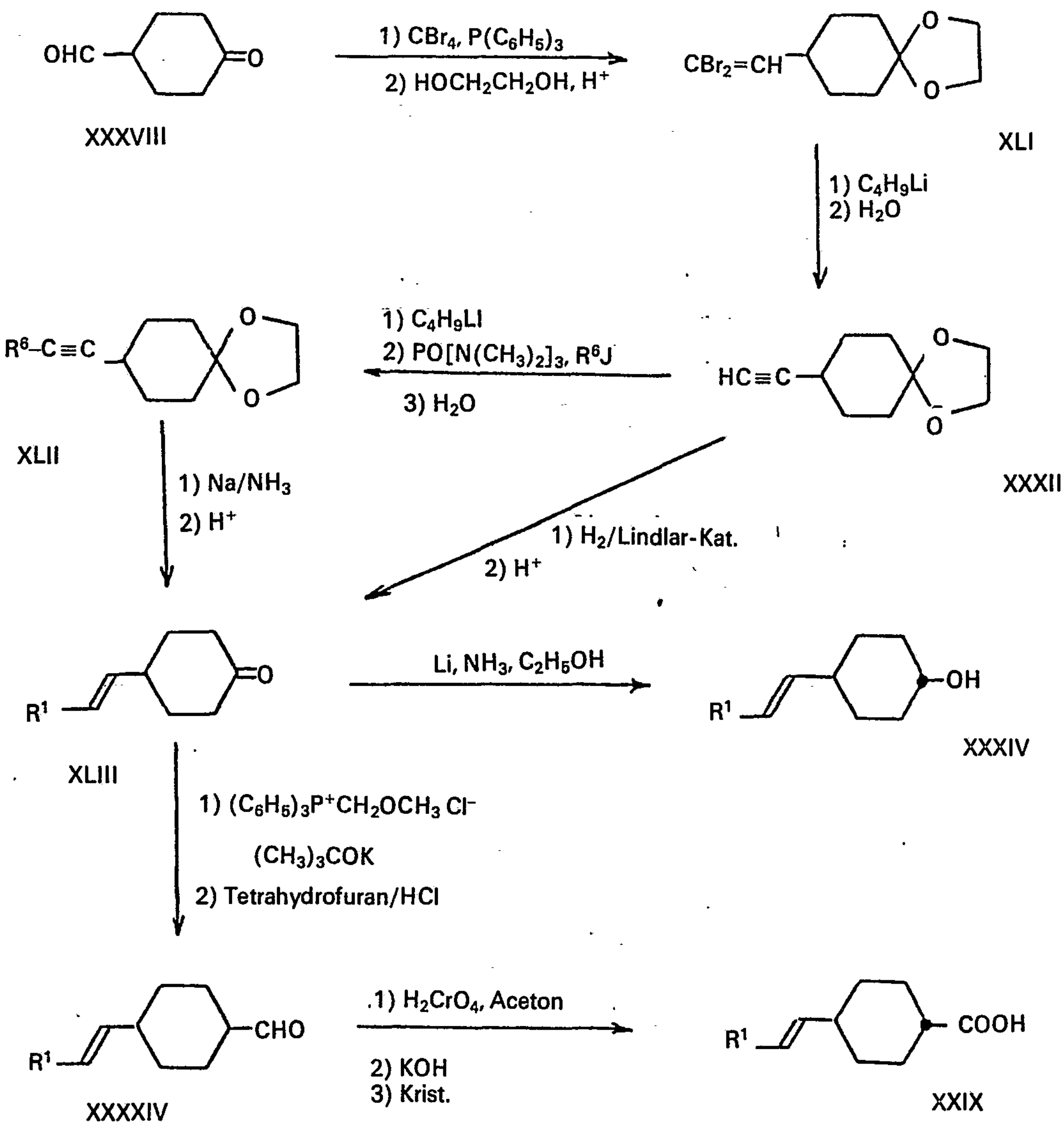
XXXVIII
1) $CBr_4$, $P(C_6H_5)_3$
2) $HOCH_2CH_2OH$, $H^+$
XLI
1) $C_4H_9Li$
2) $H_2O$
1) $C_4H_9Li$
2) $PO[N(CH_3)_2]_3$, $R^6J$
3) $H_2O$
XLII
XXXII
1) $Na/NH_3$
2) $H^+$
1) $H_2$/Lindlar-Kat.
2) $H^+$
Li, $NH_3$, $C_2H_5OH$
XLIII
XXXIV
1) $(C_6H_5)_3P^+CH_2OCH_3$ $Cl^-$
$(CH_3)_3COK$
2) Tetrahydrofuran/HCl
1) $H_2CrO_4$, Aceton
2) KOH
3) Krist.
XXXXIV
XXIX

Schema 6

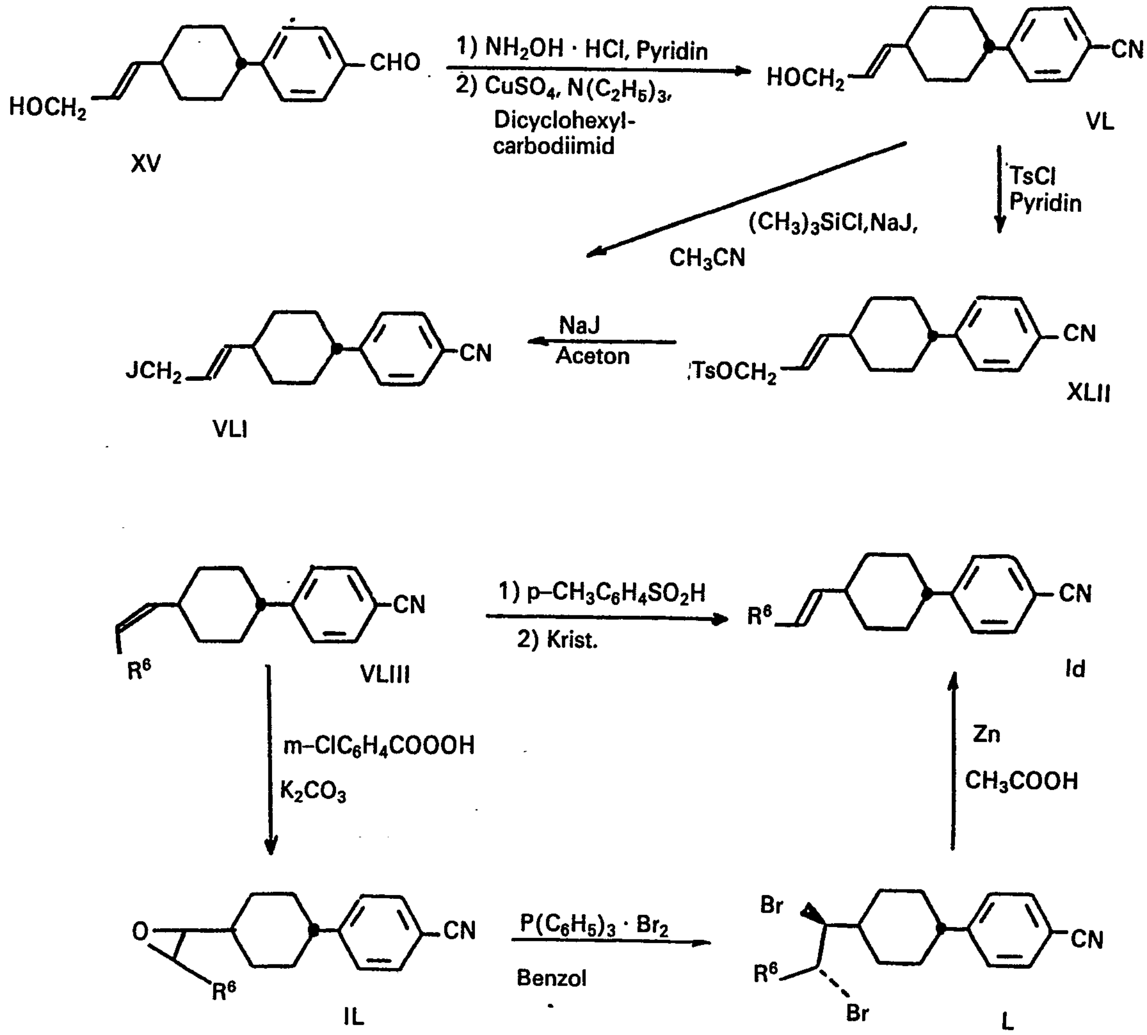


XV VL XLII VLI VLIII Id IL L

Schema 7

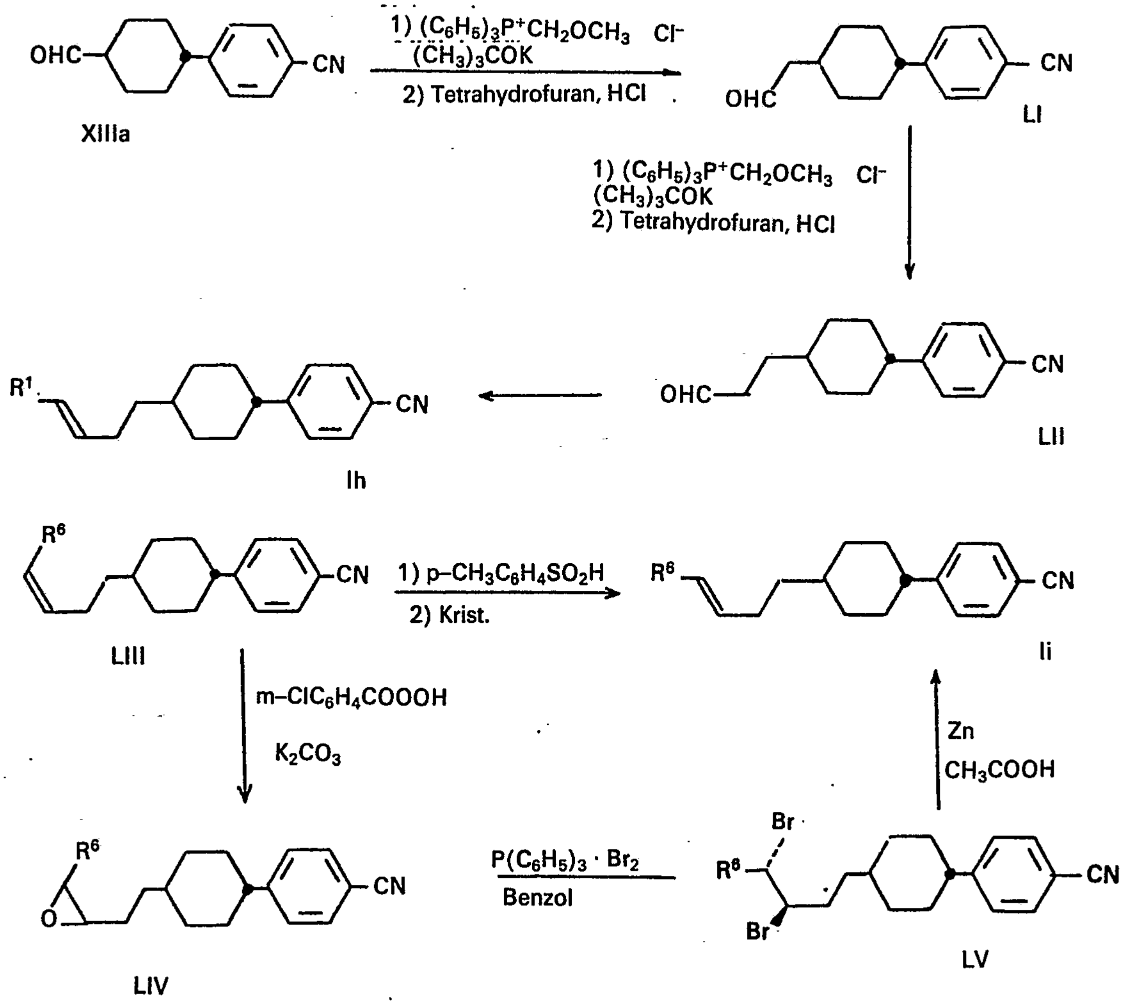
1) $(C_6H_5)_3P^+CH_2OCH_3$ $Cl^-$
$(CH_3)_3COK$
2) Tetrahydrofuran, HCl
XIIIa
LI
1) $(C_6H_5)_3P^+CH_2OCH_3$ $Cl^-$
$(CH_3)_3COK$
2) Tetrahydrofuran, HCl
LII
Ih
1) $p-CH_3C_6H_4SO_2H$
2) Krist.
LIII
Ii
$m-ClC_6H_4COOOH$
$K_2CO_3$
Zn
$CH_3COOH$
$P(C_6H_5)_3 \cdot Br_2$
Benzol
LIV
LV

Schema 8

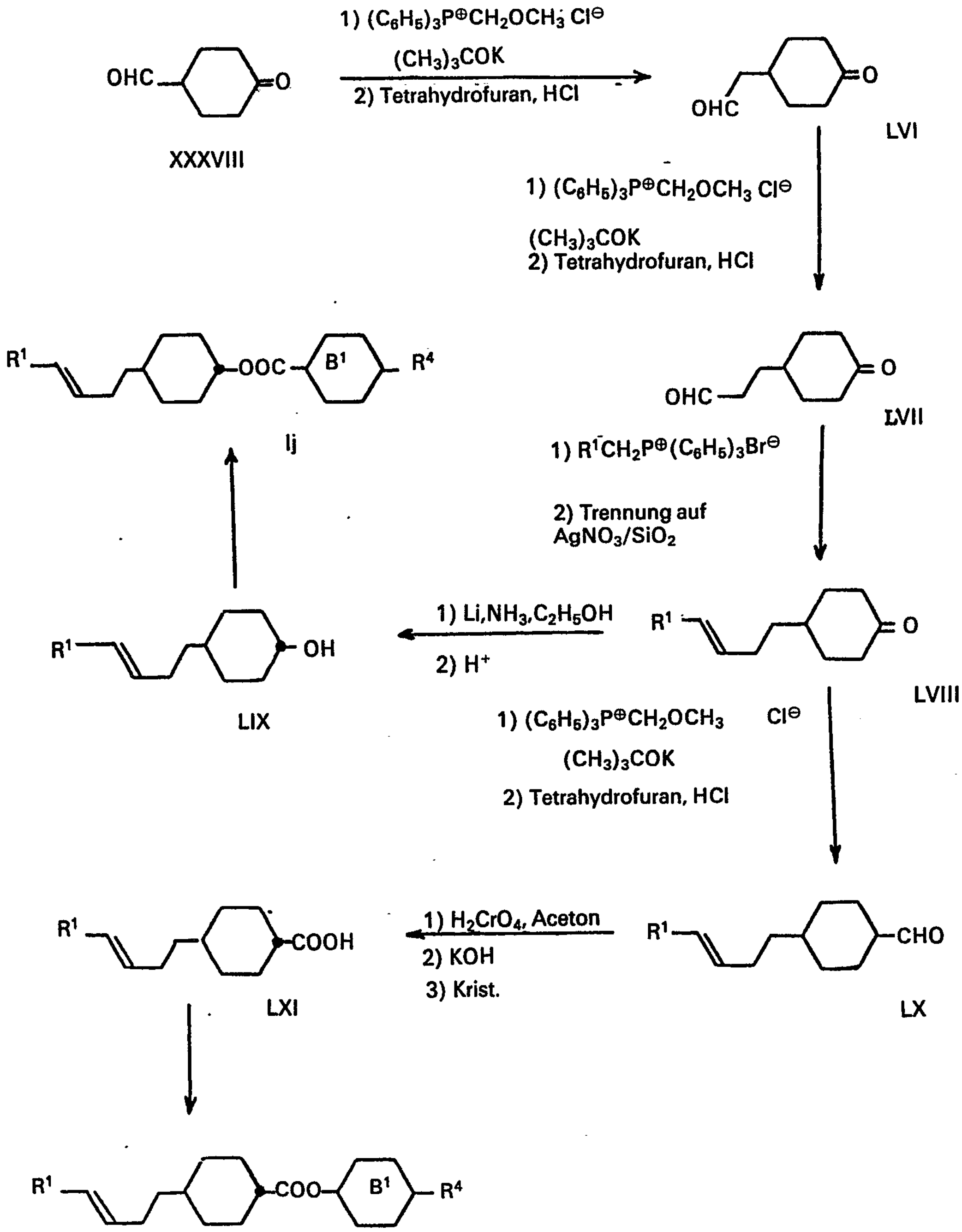


XXXVIII

LVI

LVII

Ij

LVIII

LIX

LX

LXI

Ik

Schema 9

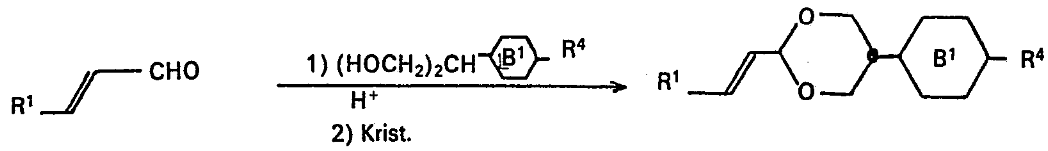


LXII

II

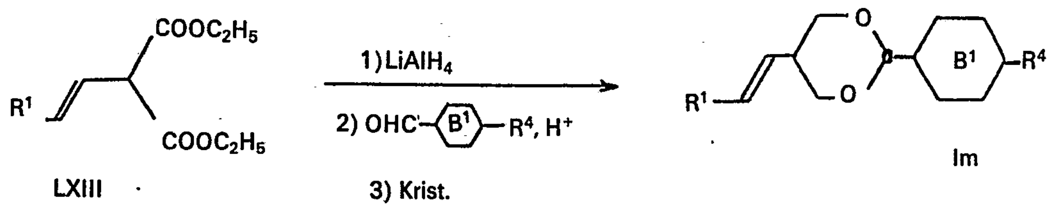


LXIII

Im

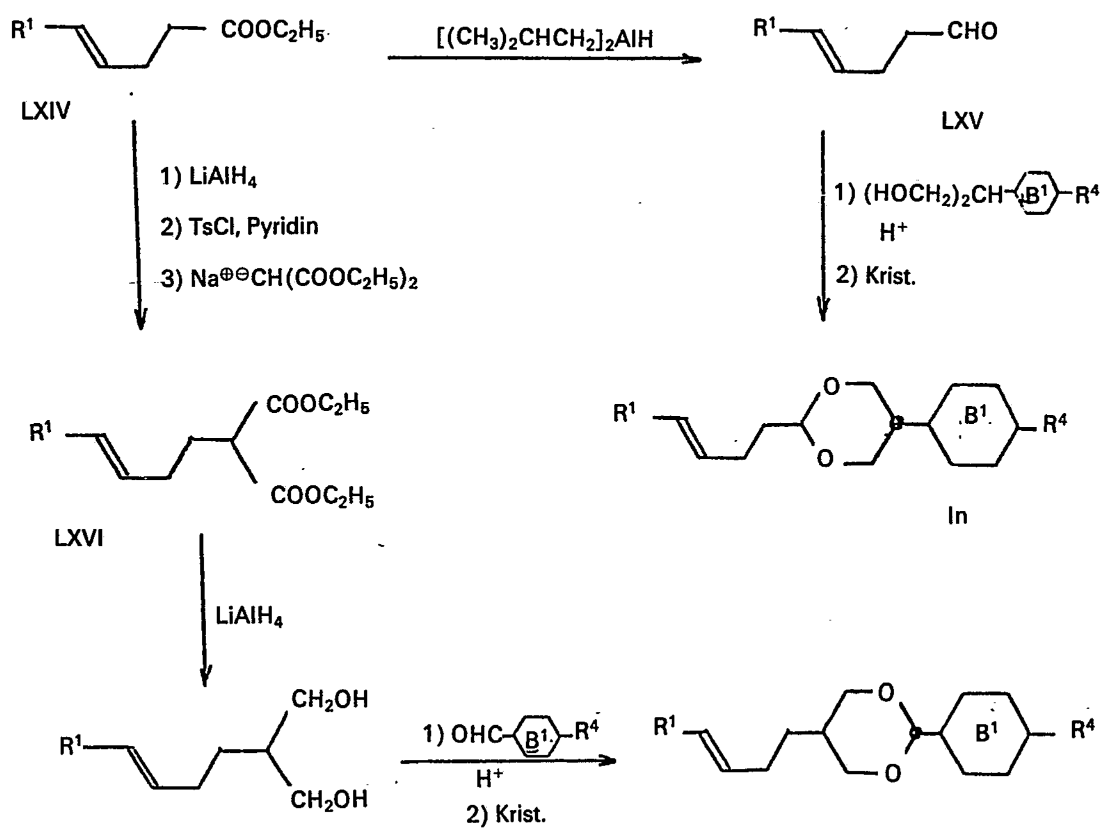


LXIV

LXV

LXVI

In

LXVII

Io

Schema 10

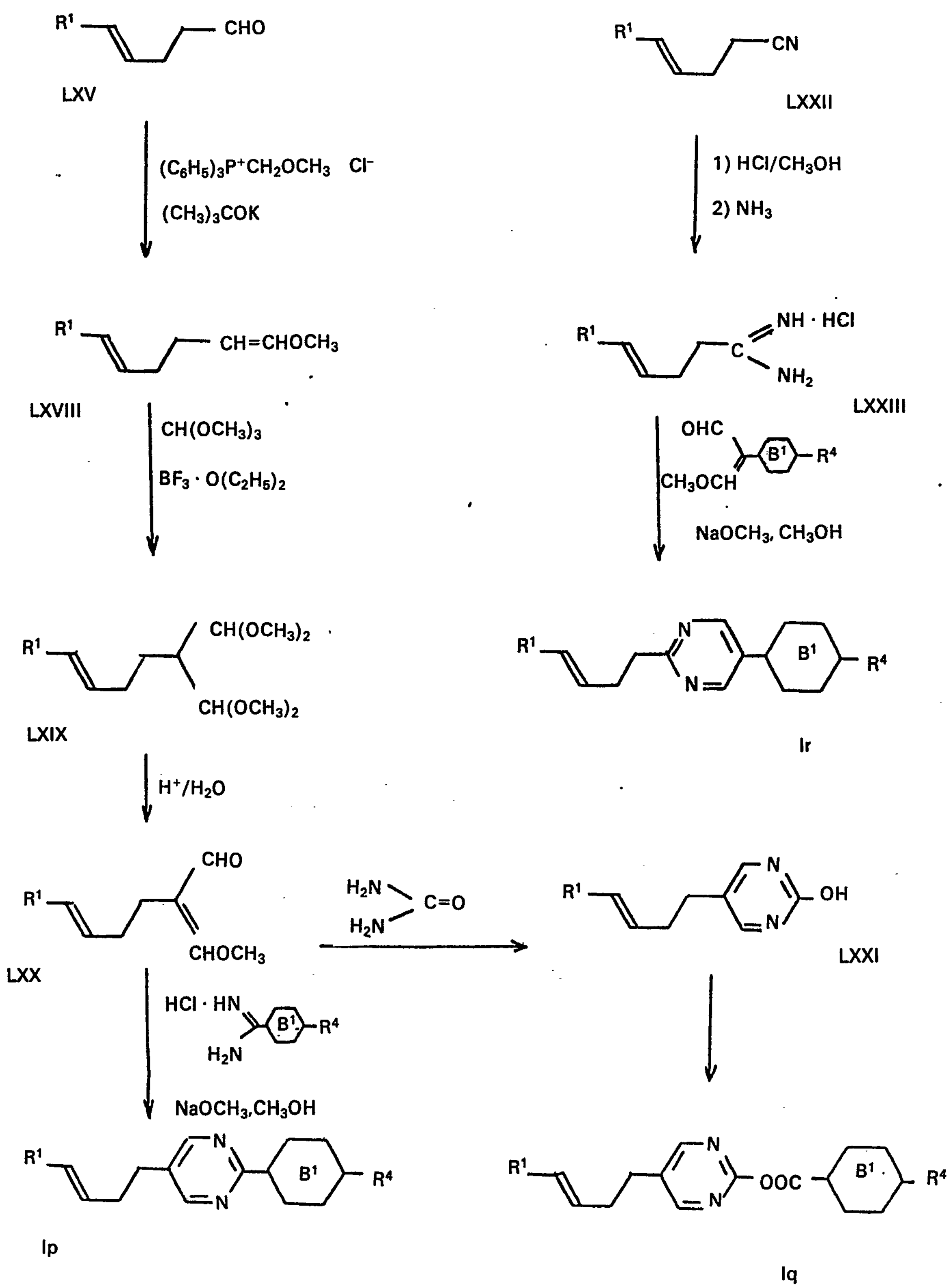


LXV, LXXII, LXVIII, LXXIII, LXIX, Ir, LXX, LXXI, Ip, Iq

Schema 11

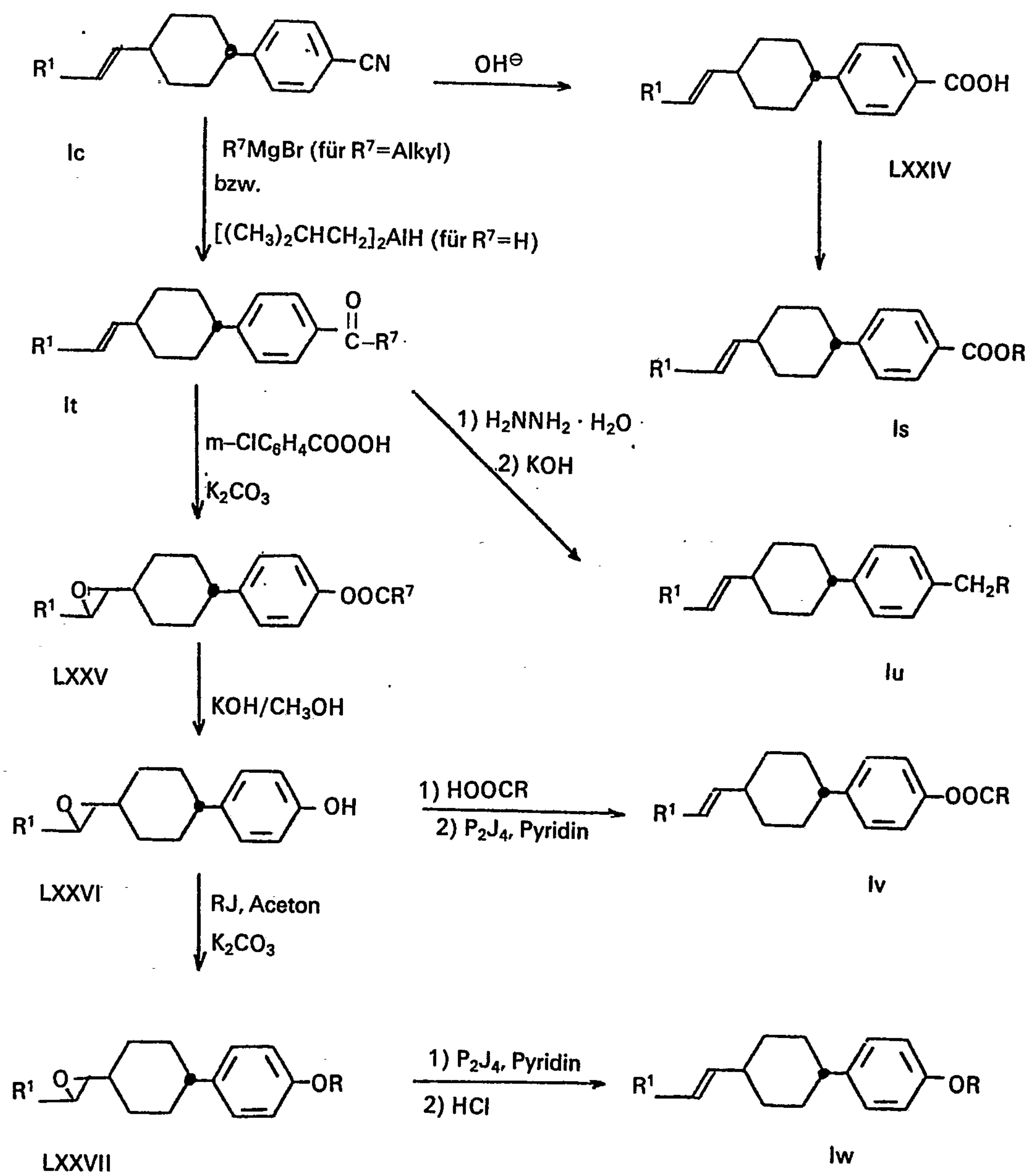


Ic LXXIV It Is LXXV Iu LXXVI Iv LXXVII Iw

Wird anstelle der Cyanoverbindung der Formel XI eine entsprechende Alkyl-, Alkoxy- oder Fluorverbindung gemäss Schema 1 umgesetzt, so kann die vorletzte Stufe (XVI→XVII) unterbleiben, da Alkyl, Alkoxy und Fluor bei der Reduktion mit Diisobutylaluminiumhydrid nicht angegriffen werden. Die Einführung anderer Abgangsgruppen $Y^1$ (anstelle der Acetoxygruppe in Formel XVII oder analogen Verbindungen) kann nach an sich bekannten Methoden erfolgen. Weitere Beispiele sind in Schema 6 angegeben.

Bei der Herstellung der Verbindungen der Formeln Ic und Ih, worin $R^1$ geradkettiges Alkyl bedeutet, gemäss Schema 1 bzw. 7 (Verfahrensvariante e) entsteht im allgemeinen ein Gemisch bestehend aus cis-Alkenyl- und trans-Alkenyl-Verbindung. Derartige Gemische können auf mit Silbernitrat beschichtetem Kieselgel chromatographisch getrennt werden. Gewünschtenfalls können die cis-Alkenyl-Verbindungen (oder Gemische mit überwiegend cis-Alkenyl-Verbindung) gemäss Schema 6 bzw. 7 in die entsprechenden trans-Alkenyl-Verbindungen übergeführt werden.

Die Umsetzung XXIII → Ie gemäss Schema 2 kann in analoger Weise zu Schema 1 erfolgen.

Die Herstellung von Säuren und Alkoholen der Formel VIII und die Einführung von trans-3-Alkenylgruppen wird anhand der Schemata 3–5, 7 und 8 illustriert. Weitere Säuren der Formel VIII können auch durch Verseifung von Nitrilen der Formel VII erhalten werden.

Die Herstellung von Verbindungen der Formel I, worin einer der Ringe einen Dioxan-, Pyrimidin-oder Pyridazinring bedeutet und/oder Ring B für 1,4-Phenylen steht, ergibt sich sinngemäss aus dem oben Gesagten unter Brücksichtigung der bekannten Methoden zur Herstellung derartiger Ringsysteme.

Bei der Herstellung von Verbindungen der Formel I, welche eine Estergruppe oder einen Dioxanring aufweisen, wird im allgemeinen mit Vorteil die Veresterung bzw. die Bildung des Dioxanringes erst am Schluss durchgeführt. Beispiele zur Herstellung erfindungsgemässer Dioxane sind in Schema 9 angegeben. Die erfindungsgemässen Pyrimidine und Pyridazine können dadurch erhalten werden, dass zuerst nach bekannten Methoden das Ringsystem aufgebaut und nachträglich die Alkenylgruppe eingeführt wird oder, dass bei der Bildung des heterocyclischen Ringes eine Verbindung eingesetzt wird, welche bereits die Alkenylgruppe aufweist. Die letztere Methode ist in Schema 10 für einige Pyrimidine näher veranschaulicht.

Verbindungen der Formel I, worin $R^2$ –OOCR bedeutet, können ebenfalls aus Verbindungen der Formel I, worin $R^2$ –COR bedeutet, hergestellt werden durch Baeyer-Villiger-Oxidation mit Persäure, z.B. Perbenzoesäure, und anschliessender Rückführung des dabei gleichzeitig gebildeten Epoxides in die Doppelbindung nach der in Tetrahedron 36, 557 (1980) beschriebenen Methode.

Schemata 1, 2, 6 und 7 gelten sinngemäss, wenn die Cyanogruppe durch Alkyl, Alkoxy oder Fluor ersetzt wird. Es kann jedoch von Vorteil sein, Verbindungen der Formel I mit verschiedenen Bedeutungen für $R^2$ nach einem gemeinsamen Reaktionsschema herzustellen und die gewünschten Reste $R^2$ in an sich bekannter Weise erst nachträglich einzuführen. Beispiele für diese Variante sind in Schema 11 angegeben.

Die zur Herstellung der erfindungsgemässen Fluorverbindungen ($R^2$ = Fluor) benötigten Ausgangsmaterialien sind bekannte oder Analoge bekannter Verbindungen oder können in an sich bekannter Weise aus entsprechenden Aminen erhalten werden durch Diazotierung mit Natriumnitrit und Salzsäure, Überführung in das Diazonium-tetrafluoroborat mit Natriumtetrafluoroborat und anschliessendes Erhitzen. Die Aminen sind beispielsweise ausgehend von den entsprechenden Carbonsäuren durch Hofmann-, Curtius- oder Schmidt-Abbau erhältlich.

Die Herstellung der Verbindungen der Formel I, worin A für die Gruppe mit 2 bis 4 sechsgliedrigen Ringen steht, (bzw. die Herstellung der entsprechenden in den Verfahrensvarianten a)–f) benötigten Ausgangsmaterielien) kann in analoger Weise zur oben näher beschriebenen Herstellung der bicyclischen Verbindungen erfolgen.

Aufgrund der günstigen Eigenschaften der Verbindungen der Formel I und ihrer guten Mischbarkeit können die erfindungsgemässen Mischungen auch lediglich aus zwei oder mehreren Verbindungen der Formel I bestehen. Die erfindungsgemässen Mischungen enthalten daher zweckmässig etwa 1–100 Gew.-%, vorzugsweise etwa 5–70 Gew.-% und besonders bevorzugt etwa 10–50 Gew.-% an Verbindungen der Formel I. Neben einer oder mehreren Verbindungen der Formel I enthalten die erfindungsgemässen Mischungen, gegebenenfalls, vorzugsweise eine oder mehrere Verbindungen der allgemeinen Formeln

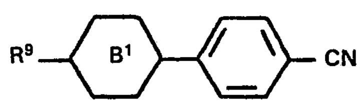
LXXX

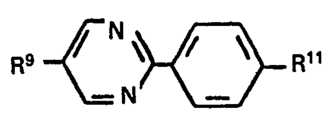
LXXXI

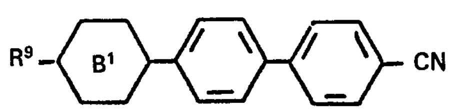
LXXXII

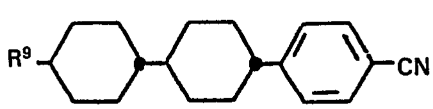
LXXXIII

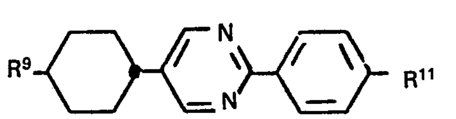
LXXXIV

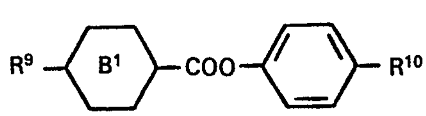
LXXXV

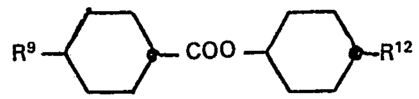

LXXXVI

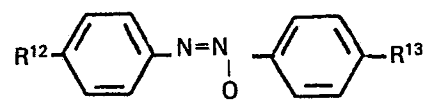

LXXXVII

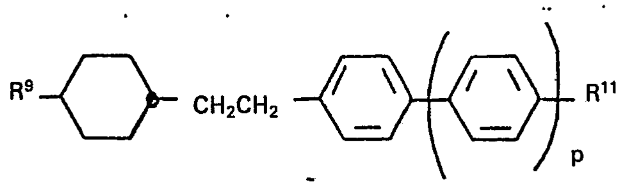

LXXXVIII

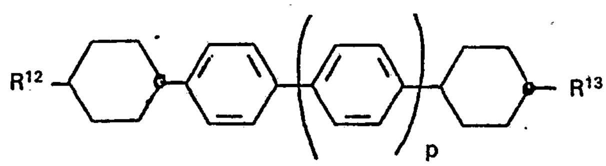

LXXXIX

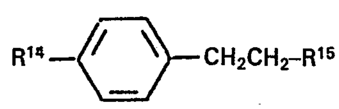

XC

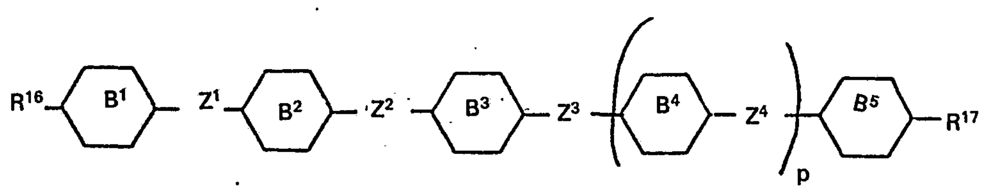

XCI

worin Ring $B^1$ 1,4-Phenylen oder trans-1,4-Cyclohexylen, $R^9$ geradkettiges $C_2$–$C_7$-Alkyl, $R^{10}$ Cyano oder geradkettiges $C_1$–$C_6$-Alkoxy, $R^{11}$ Cyano oder geradkettiges $C_1$–$C_6$-Alkyl oder $C_1$–$C_7$-Alkoxy und $R^{12}$ und $R^{13}$ geradkettiges $C_1$–$C_7$-Alkyl bezeichnen; p für 0 oder 1 steht; $R^{14}$ trans-4-Alkylcyclohexyl, 4'-Alkyl-4-biphenylyl, p-(trans-4-Alkylcyclohexyl)phenyl, 2-(trans-4-Alkylcyclohexyl)äthyl oder p-[2-(trans-4-Alkylcyclohexyl)-äthyl]phenyl und $R^{15}$ trans-4-Alkylcyclohexyl bedeuten, oder $R^{14}$ trans-4-Alkylcyclohexyl und $R^{15}$ p-(trans-4-Alkylcyclohexyl) phenyl, p-[2-(trans-4-Alkylcyclohexyl) äthyl]phenyl oder 4'-(trans-4-Alkylcyclohexyl) -4-biphenylyl bedeuten, oder $R^{14}$ p-Alkylphenyl und $R^{15}$ p-[2-(trans-4-Alkylcyclohexyl) äthyl]phenyl bedeuten, und die Alkylreste in den Substituenten $R^{14}$ und $R^{15}$ geradkettiges $C_1$–$C_7$-Alkyl bezeichnen; eine der Gruppen $Z^1$ und $Z^2$ –COO– oder–OOC– und die übrigen der Gruppen $Z^1$, $Z^2$, $Z^3$ und $Z^4$ einfache Kovalenzbindungen darstellen, oder eine dieser Gruppen auch $-CH_2CH_2-$ darstellt; die Ringe $B^1$ und $B^5$ in Formel XCI eine Gruppe der Formel

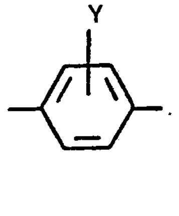

XCII

oder trans-1,4-Cyclohexylen bezeichnen; die Ringe $B^2$, $B^3$ und $B^4$ eine Gruppe der Formel XCII oder, sofern sie nicht mit mindestens einem der beiden andern dieser Ringe durch eine einfache Kovalenzbindung verknüpft sind, auch trans-1,4-Cyclohexylen darstellen; Y Wasserstoff oder an einem der Ringe der Formel XCII, welcher nicht mit einem weiteren Ring über eine einfache Kovalenzbindung verknüpft ist, auch Fluor, Chlor oder Methyl bedeutet; $R^{16}$ und $R^{17}$ geradkettiges $C_1$–$C_7$-Alkyl oder an einem Ring der Formel XCII auch geradkettiges $C_1$–$C_7$-Alkoxy bezeichnen.

Die Verbindungen der Formeln LXXX–XC sind bekannte oder Analoge bekannter Verbindungen.

Die Ester der Formel XCI sind neu, können jedoch nach an sich bekannten Veresterungsmethoden erhalten werden. Die hierbei benötigten Ausgangsmaterialien sind bekannte oder Analoge bekannter Verbindungen und können nach bekannten Methoden hergestellt werden.

Die erfindungsgemässen Mischungen können ferner geeignete, optisch aktive Verbindungen, beispielsweise optisch aktive Biphenyle, und/oder dichroitische Farbstoffe, beispielsweise Azo-, Azoxy- oder Anthrachinon-Farbstoffe, enthalten. Der Anteil solcher Verbindungen wird durch die Löslichkeit und die gewünschte Ganghöhe (pitch), Farbe, Extinktion und dergleichen bestimmt. Vorzugsweise beträgt der Anteil optisch aktiver Verbindungen höchstens etwa 4 Gew.-% und der Anteil dirchroitischer Farbstoffe höchstens etwa 10 Gew.-%.

Die Herstellung der erfindungsgemässen flüssigkristallinen Gemische kann in an sich bekannter Weise erfolgen, z.B. durch Erhitzen einer Mischung der Bestandteile auf eine Temperatur knapp oberhalb des Klärpunktes und anschliessendes Abkühlen.

Die Herstellung elektro-optischer Vorrichtungen, welche als Dielektrikum eine erfindungsgemässe Mischung enthalten, kann ebenfalls in an sich bekannter Weise erfolgen, z.B. durch Evakuieren einer geeigneten Zelle und Einbringen der Mischung in die evakuierte Zelle.

Beispiele bevorzugter erfindungsgemässer Gemische sind die folgenden Mischungen 1–4. $V_{10}$ bezeichnet die Schwellenspannung für 10% Transmission, η die Viskosität (bulk viscosity) und $t_{on}$ bzw. $t_{off}$ die Einschaltzeit und die Ausschaltzeit (in einer Drehzelle bei 2,5·$V_{10}$ und Kippwinkel 0°). Die Messungen wurden bei 22 °C durchgeführt.

Mischung 1

| | |
|---|---|
| 19,0 Gew.-% | 4-Äthoxy-1-[2-(trans-4-pentylcyclohexyl)-äthyl]benzol, |
| 3,5 Gew.-% | 4-Cyano-4''-pentyl-p-terphenyl, |
| 3,5 Gew.-% | 4-Cyano-4'-(trans-4-pentylcyclohexyl)-biphehyl, |
| 3,0 Gew.-% | p-[trans-4-(2-trans-4-Pentylcyclohexyl)-äthyl)cyclohexyl]-benzonitril, |
| 8,0 Gew.-% | 1-[2-(trans-4-Butylcyclohexyl) äthyl]-4-(trans-4- pentylcyclohexyl)benzol, |
| 23,0 Gew-% | p-[trans-4-(trans-1-Butenyl) cyclohexyl]-benzonitril, |
| 40,0 Gew.-% | p-[trans-4-(trans-1-Pentenyl) cyclohexyl]-benzonitril; |

Smp. < −20 °C, Klp. 78 °C, nematisch; $V_{10}$ = 2,18 V, $t_{on}$=23 ms, $t_{off}$ = 41 ms.

Mischung 2

| | |
|---|---|
| 10,0 Gew.-% | p-(5-Butyl-2-pyrimidinyl) benzonitril, |
| 4,0 Gew.-% | p-(5-Heptyl-2-pyrimidinyl)benzonitril, |
| 9,0 Gew.-% | trans-4-Butylcyclohexancarbonsäure- p-pentyloxyphenylester, |
| 13,0 Gew.-% | trans-4-Pentylcyclohexancarbonsäure- p-propyloxyphenylester, |
| 21,0 Gew.-% | 4-Äthoxy-1-[2-(trans-4-propylcyclohexyl) -äthyl]benzol, |
| 4,0 Gew.-% | 4-Cyano-4'-(trans-4-pentylcyclohexyl) biphenyl, |
| 6,0 Gew.-% | p-[5-(trans-4-Äthylcyclohexyl)-2-pyrimidin]benzonitril, |
| 6,5 Gew.-% | p-[trans-4-(trans-1-Propenyl)-cyclohexyl]-benzonitril, |
| 26,5 Gew.-% | p-[trans-4-(trans-1-Butenyl) cyclohexyl]-benzonitril; |

Smp. < −20 °C, Klp. 60 °C, nematisch; η = 26,5 cP, $V_{10}$ = 1,50 V.

Mischung 3

| | |
|---|---|
| 17,5 Gew.-% | p-(5-Butyl-2-pyrimidinyl)benzonitril, |
| 10.0 Gew.-% | p-(5-Heptyl-2-pyrimidinyl)benzonitril, |
| 6,5 Gew.-% | p-Äthylbenzoesäure-p'-cyanophenylester, |
| 8,0 Gew.-% | 4-cyano-4'-(trans-4-pentylcyclohexyl) biphenyl, |
| 10,0 Gew.-% | p-[5-(trans-4-Äthylcyclohexyl)-2-pyrimidinyl]-benzonitril, |
| 14,5 Gew.-% | p-[trans-4-(trans-1-Propenyl)-cychexyl] -benzonitril, |
| 9,5 Gew.-% | p-[trans-5-(trans-1-Pentenyl)-m-dioxan-2-yl]-benzonitril, |
| 24,0 Gew.-% | p-[trans-5-(trans-1-Hexenyl)-m-dioxan-2-yl]-benzonitril; |

Smp. ca. −10 °C, Klp. 80,4 °C, nematisch; $V_{10}$ = 1,31 V.

Mischung 4

| | |
|---|---|
| 16,3 Gew.-% | p-(5-Butyl-2-pyrimidinyl)benzonitril, |
| 9,3 Gew.-% | p-(5-Heptyl-2-pyrimidinyl)benzonitril, |
| 6,1 Gew.-% | p-Äthylbenzoesäure-p'-cyanophenylester, |
| 7,0 Gew.-% | 4-Äthyl-1-[2-(trans-4-propylcyclohexyl) -äthyl]benzol, |
| 7,4 Gew.-% | 4-Cyano-4'-(trans-4-pentylcyclohexyl)biphenyl, |
| 9,3 Gew.-% | p-[5-(trans-4-Äthylcyclohexyl)-2-pyrimidinyl]-benzonitril, |
| 13,5 Gew.-% | p-[trans-4-(trans-1-Propenyl)-cyclohexyl] -benzonitril, |
| 8,8 Gew.-% | p-[trans-5-(trans-1-Pentenyl)-m-dioxan-2-yl]-benzonitril, |
| 22,3 Gew.-% | p-[trans-5-(trans-1-Hexenyl)m-dioxan-2-yl]-benzonitril; |

Smp. < −20 °C, Klp. 72 °C, nematisch; $V_{10}$ = 1,29 V.

Die Herstellung der erfindungsgemässen Verbindungen wird durch die folgenden Beispiele weiter veranschaulicht. C bedeutet eine kristalline, S eine smektische, $S_B$ eine smektisch B, N eine nematische und I die isotrope Phase.

Beispiel 1

In einem Sulfierkolben mit Thermometer, Tropftrichter und Serumkappe wurde unter Argonbegasung eine Lösung von 7,1 mMol Äthylmagnesiumbromid (hergestellt aus 172 mg Magnesium und 530 μl Äthylbromid) in 20 ml absolutem Tetrahydrofuran bei −78 °C vorgelegt und der Reihe nach mit 3,6 ml einer 0,48M Lösung von Dilithiumtetrachlorokuprat in absolutem Tetrahydrofuran und mit einer Lösung von 500 mg p-[trans-4-(3-Acetoxy-trans-1-propenyl) cyclohexyl] benzonitril in 10 ml absolutem Tetrahydrofuran versetzt. Nach beendeter Zugabe wurde das Reaktionsgemisch auf −15 °C erwärmt, bei dieser Temperatur noch 1,5 Stunden gerührt, anschliessend mit 20 ml gesättigter Ammoniumchlorid-Lösung versetzt und noch 1 Stunde bei Raumtemperatur gerührt. Die nunmehr tiefblaue, wässrige Phase wurde abgetrennt und noch zweimal mit je 50 ml Diäthyläther extrahiert. Die organischen Phasen wurden zweimal mit je 50 ml Wasser gewaschen, über Magnesiumsulfat getrocknet und eingeengt. Niederdruckchromatographie (0,5 bar) des Rückstandes (0,4 g) an Kieselgel mit Essigester/Petroläther (Vol. 3:97) ergab 375 mg rohes p-[trans-4- (trans-1-Pentenyl) cyclohexyl] benzonitril, welches gemäss gaschromatographischer Analyse zu 8,5% mit p-[trans-4-(trans-1-Pentenyl) cyclohexyl] propiophenon und zu 4,0% mit p-[trans-4-(1-Vinylpropyl) cyclohexyl] benzonitril verunreinigt war. Behandlung dieses Rohproduktes mit einem Überschuss Natriumborhydrid in Methanol bei 0 °C (zwecks Reduktion des Propiophenons), Aufarbeitung, Niederdruckchromatographie (0,5 bar) an Kieselgel mit Essigester/Petroläther (Vol. 1:9) und schliesslich Umkristallisation aus Methanol bei −78 °C ergab p-[trans-4-(trans-1-Pentenyl) cyclohexyl] benzonitril in 98,6%iger Reinheit; Smp. (C–N) 15,5 °C, Klp. (N–I) 57,0 °C.

Bei der chromatographischen Trennung des Rohproduktes an Kieselgel mit Essigester/Petroläther (Vol. 19) wurde ferner als zweite Fraktion 1-(1-Hydroxypropyl)-4-[trans-4-(trans-1-Pentenyl) cyclohexyl] benzol erhalten, welches in Aceton bei 0 °C gelöst und durch Zugabe von Chromsäure (bis die orange Farbe bestehen blieb) wieder zum Keton oxidiert wurde. Der Überschuss an Chromsäure wurde mit Isopropanol zerstört. Nach Aufarbeitung und Umkristallisation wurde schliesslich reines p-[trans-4-(trans-1-Pentenyl) cyclohexyl] propiophenon mit Smp. (C–N) 60,5 °C und Klp. (N–I) 75,0 °C erhalten.

Das als Ausgangsmaterial verwendete p-[trans-4-(3-Acetoxy-trans-1- propenyl) cyclohexyl] benzonitril wurde wie folgt hergestellt:

a) In einem Sulfierkolben mit Thermometer, mechanischem Rührer, Tropftrichter und Festsubstanzzugaberohr wurden unter Argonbegasung 10,4 g Triphenyl-methoxymethyl- phosphoniumchlorid in 60 ml t-Butylmethyläther aufgeschlämmt und bei −10 °C innert 10 Minuten mit 3,6 g festem Kalium-t-butylat versetzt. Nach beendeter Zugabe wurde noch während 30 Minuten bei −10 °C bis 0 °C gerührt und dann das tieforange, heterogene Reaktionsgemisch bei 0 °C tropfenweise mit einer Lösung von 4,2 g 4-(p-Cyanophenyl) cyclohexanon in 50 ml absolutem Tetrahydrofuran versetzt. Anschliessend wurde das Reaktionsgemisch noch 2 Stunden bei Raumtemperatur gerührt, dann auf 500 ml Hexan gegossen und filtriert. Niederdruckchromatographie (0,5 bar) des eingeengten Rückstandes (7,1 g) an Kieselgel mit Essigester/Petroläther (Vol. 5:95) ergab 4,5 g (94%) p-[4-(Methoxymethylen) cyclohexyl] benzonitril als farbloses Öl; Reinheit 95%, Rf-Wert (Essigester/Petroläther Vol. 1:9) 0,30.

b) In einem Rundkolben wurde ein Gemisch von 4,2 g p-[4-(Methoxymethylen) cyclohexyl] benzonitril und 100 ml Tetrahydrofuran/2N Salzsäure (Vol. 4:1) während 30 Minuten zum Rückfluss erhitzt. Anschliessend wurde das Reaktionsgemisch auf 100 ml Wasser gegossen und dreimal mit je 100 ml Diäthyläther extrahiert. Die organischen Phasen wurden noch einmal mit 100 ml Wasser gewaschen, über Magnesiumsulfat getrocknet und eingeengt. Es resultierten 3,9 g (100%) 4-(p-Cyanophenyl) cyclohexancarboxaldehyd als farbloses Öl, welches ohne weitere Reinigung in der nächsten Stufe eingesetzt wurde; trans/cis-Verhältnis ca. 3:1, Rf-Wert (Essigester/Petroläther Vol. 3:7) 0,41. Durch Kristallisation aus Hexan konnte reiner trans-4-(p-Cyanophenyl) cyclohexancarboxaldehyd erhalten werden; Smp. 57,1 °C.

c) In einem Sulfierkolben mit Festsubstanzzugaberohr wurde unter Argonbegasung ein Gemisch von 3,9 g des oben erhaltenen 4-(p-Cyanophenyl) cyclohexancarboxaldehyds und 272 mg gemahlenem Kaliumcarbonat in 60 ml Äthanol bei Raumtemperatur vorgelegt und innert 15 Minuten mit 7,6 g festem Äthoxycarbonylmethylen-triphenylphosphoran versetzt. Anschliessend wurde das Reaktionsgemisch noch 2 Stunden bei Raumtemperatur gerührt, dann am Rotationsverdampfer von Äthanol befreit, in 100 ml Wasser aufgenommen und dreimal mit je 100 ml Methylenchlorid extrahiert. Die organischen Phasen wurden noch zweimal mit je 100 ml Wasser gewaschen, über Magnesiumsulfat getrocknet und eingeengt. Niederdruckchromatographie (0,5 bar) des Rückstandes (12 g) an Kieselgel mit Toluol/Petroläther/Essigester (Vol. 5:4:1) ergab 5,2 g einer kristallinen Masse, welche nach Kristallisation aus 500 ml Hexan 3,9 g (75%) trans-3-[trans-4-(p-Cyanophenyl) cyclohexyl] acrylsäure-äthylester als farblose Kristalle mit Smp. 125 °C lieferte.

d) In einem Sulfierkolben mit Thermometer und Serumkappe wurde unter Argonbegasung eine Lösung von 1,0 g trans-3-[trans-4-(p-Cyanophenyl) cyclohexyl]acrylsäure-äthylester in 25 ml Methylenchlorid bei −78 °C vorgelegt und innert 10 Minuten mit 15,0 ml einer 0,84M Lösung von Diisobutylaluminiumhydrid in Toluol versetzt. Nach beendeter Zugabe wurde das Reaktionsgemisch auf −10 °C erwärmt und noch 30 Minuten bei dieser Temperatur gerührt, bevor es auf 100 ml 0,2N Schwefelsäure gegossen und zweimal mit je 50 ml Methylenchlorid extrahiert wurde. Die organischen Phasen wurden mit 50 ml Wasser gewaschen, über Magnesiumsulfat getrocknet und eingeengt. Der Rückstand (ca. 850 mg) wurde in 30 ml Methylenchlorid gelöst und der Reihe nach mit 0,5 ml Essigsäureanhydrid und 45 mg 4-(Dimethylamino)pyridin versetzt. Das Reaktionsgemisch wurde 1 Stunde bei Raumtemperatur gerührt, dann auf 50 ml gesättigte, wässrige Kupfersulfatlösung gegossen und zweimal mit je 50 ml Methylenchlorid extrahiert. Die organischen Phasen wurden noch zweimal mit je 50 ml Wasser gewaschen, über Magnesiumsulfat getrocknet und eingeengt. Niederdruckchromatographie (0,5 bar) des Rückstandes (0,95 g) an Kieselgel mit Essigester/Petroläther

(Vol. 1:9) ergab 720 mg (71%) p-[trans-4- (3-Acetoxy-trans-1- propenyl) cyclohexyl] benzaldehyd; Reinheit 99,9%, Rf-Wert (Essigester/ Petroläther Vol. 1:9) 0,31.

e) In einem Sulfierkolben mit mechanischem Rührer wurde unter Argonbegasung eine Lösung von 513 mg Hydroxylammoniumchlorid in 5 ml Wasser vorgelegt und bei Raumtemperatur mit einer Lösung von 2,0 g p-[trans-4-(3-Acetoxy-trans-1-propenyl) cyclohexyl] benzaldehyd in 10 ml Pyridin versetzt. Das Reaktionsgemisch wurde 1 Stunde gerührt und dann der Reihe nach mit 350 mg Kupfersulfat-pentahydrat und einer Lösung von 2,1 ml Triäthylamin in 10 ml Methylenchlorid versetzt. Nachdem die zunächst tintenblaue Farbe des Kupfer-Pyridin-Komplexes nach olivgrün umgeschlagen war, wurde eine Lösung von 1,74 g Dicyclohexylcarbodiimid in 20 ml Methylenchlorid zugesetzt. Anschliessend wurde das Reaktionsgemisch noch während 3 Stunden bei Raumtemperatur gerührt und dann filtriert. Das Filtrat wurde auf 100 ml Wasser gegossen und dreimal mit je 100 ml Methylenchlorid extrahiert. Die organischen Phasen wurden noch zweimal mit je 50 ml Wasser gewaschen, über Magnesiumsulfat getrocknet und eingeengt. Niederdruckchromatographie (0,5 bar) des Rückstandes (2,75 g) an Kieselgel mit Toluol/Essigester (Vol. 9:1) ergab 2,26 g (114%) p-[trans-4-(3-Acetoxy-trans-1-propenyl) cyclohexyl] benzonitril als farblose Kristalle, welche als einzige Verunreinigung noch etwas Dicyclohexylcarbodiimid enthielten. Dieses Material wurde ohne zusätzliche Reinigung weiterverwendet. Rf-Wert (Toluol/Essigester Vol. 9:1) 0,33.

In analoger Weise wurde folgende Verbindung hergestellt:

p-[trans-4-(trans-1-Butenyl) cyclohexyl] benzonitril, Smp. (C–N) 44,2 °C, Klp. (N–I) 49,5 °C.

Beispiel 2

In einem Rundkolben mit Rückflusskühler wurde unter Argonatmosphäre ein Gemisch von 235 mg p-[trans-4-(trans-1-Pentenyl) cyclohexyl] propiophenon (hergestellt nach Beispiel 1), 0,161 ml Hydrazinhydrat, 5 ml Diäthylenglykol und 5 ml Äthanol während 30 Minuten zum Rückfluss erhitzt. Dann wurde das Reaktionsgemisch mit 195 mg festem Kaliumhydroxid versetzt und anschliessend sukzessive, unter Abdestillation des Äthanols, auf 200 °C erwärmt und während 2 Stunden bei dieser Temperatur gehalten. Das abgekühlte Reaktionsgemisch wurde auf 50 ml Wasser gegossen und dreimal mit je 50 ml Petroläther extrahiert. Die organischen Phasen wurden noch dreimal mit je 50 ml Wasser gewaschen, über Magnesiumsulfat getrocknet und eingeengt. Niederdruckchromatographie (0,5 bar) des Rückstandes mit Hexan auf einer kurzen Säule mit Kieselgel ergab 185 mg (83%) 4-Propyl-1-[trans-4-(trans-1-pentenyl) cyclohexyl]-benzol als farblose Flüssigkeit; Reinheit 98,6%, Smp. (C–I) 7,0 °C.

Beispiel 3

In einem Rundkolben mit Rückflusskühler wurde unter Argonbegasung ein Gemisch von 3,8 g 4-(p-Cyanophenyl) cyclohexancarboxaldehyd (hergestellt nach Beispiel 1), 10,3 g Propyltriphenylphosphoniumbromid und 12,3 g Kaliumcarbonat in 200 ml Dioxan während 25 Stunden zum Rückfluss erhitzt. Anschliessend wurde das abgekühlte Reaktionsgemisch filtriert, eingeengt, in 150 ml Wasser aufgenommen und dreimal mit je 150 ml Diäthyläther extrahiert. Die organischen Phasen wurden noch zweimal mit je 100 ml Wasser gewaschen, über Magnesiumsulfat getrocknet und eeingeengt. Niederdruckchromatographie (0,5 bar) des Rückstandes (10,5 g) mit Essigester/Petroläther (Vol. 3:97) ergab 2,35 g (55%) farblose, semikristalline Masse, welche gemäss gaschromatographischer Analyse aus 80,3 Gew.-% p-[trans-4-(cis-1-Butenyl)-cyclohexyl]benzonitril, 17,5 Gew.-% p-[trans-4-(trans-1Butenyl)cyclohexyl] benzonitril und 2,2 Gew.-% p-[cis-4-(cis-1-Butenyl) cyclohexyl] benzonitril bestand. Durch zusätzliche Niederdruckchromatographie (0,5 bar) dieses Materials an mit 10% Silbernitrat beschichtetem Kiesegel unter Verwendung von Essigester/ Petroläther (Vol. 3:97) sowie durch anschliessende Kristallisation aus Methanol bei −78 °C konnte reines p-[trans-4-(trans-1-Butenyl)cyclohexyl] benzonitril isoliert werden, Smp. (C–N) 44,2 °C, Klp. (N–I) 49,5 °C.

Beispiel 4 (Ausgangsmaterial)

a) In einem Sulfierkolben mit Thermometer wurden unter Argonbegasung 4,0 g 4-(p-Cyanophenyl) cyclohexancarboxaldehyd (hergestellt nach Beispiel 1) in 50 ml 0,1N methanolischer Kaliumhydroxid-Lösung gelöst und die Lösung bei 0 °C innert 20 Minuten portionenweise mit 711 mg Natriumborhydrid versetzt. Anschliessend wurde das Reaktionsgemisch noch 10 Minuten bei 0 °C gerührt, dann nach Neutralisation mit 1N Salzsäure am Rotationsverdampfer eingeengt, in 200 ml Wasser aufgenommen und dreimal mit je 100 ml Methylenchlorid extrahiert. Die organischen Phasen wurden zweimal mit je 100 ml Wasser gewaschen, über Magnesiumsulfat getrocknet und eingeengt. Einmalige Kristallisation des Rückstandes (4,2 g) aus 90 ml Essigester/Petroläther (Vol. 1:2) bei 0 °C ergab 3,27 g (77%) p-[trans-4-(Hydroxymethyl) cyclohexyl] benzonitril als farblose Kristalle mit Smp. 109,8 °C; Reinheit 99,4%.

b) In einem Sulfierkolben mit mechanischem Rührer, Thermometer und Tropftrichter wurde unter Argonbegasung eine Lösung von 3,0 g p-[trans-4-(Hydroxymethyl) cyclohexyl] benzonitril in 5 ml Pyridin bei 0 °C vorgelegt und innert 5 Minuten mit einer Lösung von 4,4 g p-Tosylchlorid in 10 ml Pyridin versetzt. Nach beendeter Zugabe wurde das Kühlbad entfernt und das Reaktionsgemisch bei Raumtemperatur während 15 Stunden gerührt. Das Reaktionsgemisch wurde am Rotationsverdampfer vom Pyridin befreit und der Rückstand in 100 ml Wasser aufgenommen

und dreimal mit je 100 ml Methylenchlorid extrahiert. Die organischen Phasen wurden noch zweimal mit je 100 ml Wasser gewaschen, über Magnesiumsulfat getrocknet und eingeengt. Niederdruckchromatographie (0,5 bar) des Rückstandes (5,0 g) an Kieselgel mit Essigester/Petroläther (Vol. 1:4) ergab 4,35 g (95%) p-[trans-4- (p-Tosyloxymethyl) cyclohexyl] benzonitril als farblose Kristalle; Rf-Wert (Essigester/Petroläther Vol. 3:7) 0,38.

c) In einem Rundkolben mit Rückflusskühler wurde unter Argonbegasung ein Gemisch von 3,8 g p-[trans-4-(p-Tosyloxymethyl) cyclohexyl] benzonitril, 2,3 g Natriumjodid und 100 ml Aceton während 15 Stunden zum Rückfluss erhitzt. Nach Filtration und Einengen des Reaktionsgemisches am Rotationsverdampfer wurde der Rückstand in 100 ml Wasser aufgenommen und dreimal mit je 100 ml Diäthyläther extrahiert. Die organischen Phasen wurden noch zweimal mit je 100 ml Wasser gewaschen, über Magnesiumsulfat getrocknet und eingeengt. Niederdruckchromatographie (0,5 bar) des Rückstandes an Kieselgel mit Toluol ergab 3,19 g (95%) p-[trans-4-(Jodmethyl) cyclohexyl] benzonitril als farblose Kristalle; Rf-Wert (Essigester/Petroläther Vol. 1:9) 0,30.

Beispiel 5

In einem Sulfierkolben mit Tropftrichter und Thermometer wurde unter Argonbegasung eine Suspension von 2,51 g Methyltriphenylphosphoniumbromid in 80 ml absolutem Tetrahydrofuran bei −20 °C vorgelegt und mit 7,6 ml einer ca. 0,8M Lösung von Butyllithium in Hexan versetzt. Nach 30 Minuten Rühren bei −20 °C wurde zu dem gelben Reaktionsgemisch eine Lösung von 1,0 g trans-4-(p-Cyanophenyl) cyclohexancarboxaldehyd in 10 ml absolutem Tetrahydrofuran bei −20 °C innert 5 Minuten zugetropft, wobei die gelbe Farbe verschwand. Nun wurde noch 30 Minuten bei −20 °C nachgerührt und dann der Kolbeninhalt auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Niederdruckchromatographie (0,5 bar) des Rückstandes (2,3 g) an Kieselgel mit Essigester/Petroläther (Vol. 3:97) ergab 897 mg (91%) p-(trans-4-Vinylcyclohexyl) benzonitril als farblose Kristalle; Reinheit 99,4%. Durch zusätzliche kristallisation aus 22 ml Methanol wurde p-(trans-4-Vinylcyclohexyl) benzonitril in einer Reinheit von 99,95% erhalten; Smp. (C–I) 56,4 °C, Klp. 28,5 °C.

Beispiel 6

In einem Sulfierkolben mit Thermometer, mechanischem Rührer, Tropftrichter und Festsubstanzzugaberohr wurde unter Argonbegasung eineSuspension von 3,6 g Butyltriphenylphosphoniumbromid in 40 ml t-Butylmethyläther bei Raumtemperatur vorgelegt, mit 1,01 g Kalium-t-butylat versetzt und noch 1 Stunde bei Raumtemperatur gerührt. Anschliessend wurde das tieforange, heterogene Reaktionsgemisch auf −60 °C abgekühlt und innert 15 Minuten mit einer Lösung von 1,28 g trans-4-(p-Cyanophenyl) cyclohexancarboxaldehyd in 10 ml t-Butylmethyläther versetzt. Das Reaktionsgemisch wurde noch 60 Minuten unter langsamem Erwärmen auf −30 °C gerührt, dann auf 100 ml Wasser gegossen und dreimal mit je 50 ml Diäthyläther extrahiert. Die organischen Phasen wurden einmal mit 50 ml Wasser gewaschen, über Magnesiumsulfat getrocknet und eingeengt. Niederdruckchromatographie (0,5 bar) des Rückstandes (3,45 g) an Kieselgel mit Essigester/Petroläther (Vol. 3:97) ergab 1,52 g (99%) p-[trans-4-(1-Pentenyl) cyclohexyl] benzonitril (trans-1-Pentenyl/cis-1-Pentenyl-Verhältnis ca. 5:95) als farbloses Öl; Rf-Wert (Essigester/Petroläther Vol. 3:97) 0,19.

In analoger Weise wurden folgende Verbindungen hergestellt:

p-[trans-4-(1-Propenyl) cyclohexyl]benzonitril,
p-[trans-4-(1-Butenyl) cyclohexyl]benzonitril,
p-[trans-4-(1-Hexenyl) cyclohexyl]benzonitril,
p-[trans-4-(1-Heptenyl)cyclohexyl] benzonitril.

Beispiel 7

In einem Rundkolben mit Magnetrührer und Rückflusskühler wurde unter Argonbegasung ein Gemisch von 3,79 g p-[trans-4-(1-Hexenyl) cyclohexyl] benzonitril (hergestellt gemäss Beispiel 6; trans-1-Hexenyl/cis-1-Hexenyl-Verhältnis ca. 5:95) und 758 mg Benzolsulfinsäure in 50 ml 1,4-Dioxan 15 Stunden unter Rückfluss gekocht. Anschliessend wurde nochmals 379 mg Benzolsulfonsäure zugegeben und das Gemisch weitere 4 Stunden zum Rückfluss erhitzt. Dann wurde das abgekühlte Reaktionsgemisch auf 50 ml 1N Natronlauge gegossen und dreimal mit je 100 ml Hexan extrahiert. Die organischen Phasen wurden zweimal mit je 50 ml Wasser gewaschen, über Magnesiumsulfat getrocknet und eingeengt. Dreimalige Kristallisation des quantiativ zurückerhaltenen, äquilibrierten Olefingemisches (trans-1-Hexenyl/cis-1- Verhätnis 80,4:19,6) aus Methanol ergab schliesslich 1,74 g (46%) p-[trans-4- (trans-1-Hexenyl) cyclohexyl] benzonitril (enthaltend 0,3% cis-1-Hexenyl-Isomer) mit Smp. (C–N) 14,3 °C und Klp. (N–I) 39,5 °C. Die Mutterlaugen wurden nicht aufgearbeitet. Gewünschtenfalls können diese aber wiederum äquilibriert und kristallisiert werden.

In analoger Weise wurden folgende Verbindungen hergestellt:

p-[trans-4-(trans-1-Propenyl)cyclohexyl] benzonitril; Smp. (C–N) 66,3 °C, Klp. (NNI) 73,0 °C,

p-[trans-4-(trans-1-Butenyl)cyclohexyl] benzonitril; Smp. (C–N) 45,1 °C, Klp. (N–I) 51,8 °C,

p-[trans-4-(trans-1-Pentenyl)cyclohexyl] benzonitril; Smp. (C–N) 15,6 °C, Klp. (N–I) 58,5 °C,

p-[trans-4-(trans-1-Heptenyl)cyclohexyl] benzonitril; Smp. (C–N) 17,9 °C, Klp. (N–I) 49,2 °C.

Beispiel 8

In einem Sulfierkolben mit mechanischem Rührer und Thermometer wurde unter Argonbegasung ein Gemisch von 2,75 g p-[trans-4-(erythro-1,2-Dibrompentyl) cyclohexyl] benzonitril und 20 ml Eisessig bei Raumtemperatur mit 2,42 g Zinkpulver versetzt und dann 2 Stunden gerührt, wobei sich das Reaktionsgemisch auf 33 °C erwärmte und das Edukt allmählich in Lösung ging. Anschliessend wurde das Reaktionsgemisch auf 100 ml Wasser gegossen und dreimal mit je 100 ml Petroläther extrahiert. Die organischen Phasen wurden zweimal mit je 100 ml Wasser und einmal mit 50 ml gesättigter Natriumhydrogencarbonat-Lösung gewaschen, über Magnesiumsulfat getrocknet und eingeengt. Hierbei wurden 1,43 g (99%) p-[trans-4-(trans-1-Pentenyl) cyclohexyl] benzonitril in einer Reinheit von 99,5% erhalten; Smp. (C–N) 15,6 °C, Klp. (N–I) 58,5 °C.

Das als Ausgangsmaterial verwendete p-[trans-4-(erythro-1,2-Dibrompentyl) cyclohexyl] benzonitril wurde wie folgt hergestellt:

a) In einem Sulfierkolben mit Thermometer, Tropftrichter und mechanischem Rührer wurde unter Argonbegasung ein Gemisch von 1,51 g 90%-iger m-Chlorperbenzoesäure und 3,0 g gemahlenem Kaliumcarbonat in 60 ml Methylenchlorid bei 0 °C vorgelegt und innert 15 Minuten mit einer Lösung von 2,0 g p-[trans-4-(1-Pentenyl) cyclohexyl] benzonitril (hergestellt nach Beispiel 6; trans-1-Pentenyl-/cis-1-Pentenyl-Verhältnis ca. 5:95) in 20 ml Methylenchlorid versetzt. Anschliessend wurde das Kühlbad entfernt und das Reaktionsgemisch nach insgesamt 75 Minuten und 105 Minuten nochmals mit je 0,75 g 90%-iger m-Chlorperbenzoesäure versetzt. Das Reaktionsgemisch wurde noch 60 Minuten bei Raumtemperatur gerührt, dann auf 50 ml 10%-ige (Gew./Vol.) Natriumthiosulfat-Lösung gegossen und dreimal mit je 100 ml Methylenchlorid extrahiert. Die organischen Phasen wurden mit 50 ml gesättigter Natriumhydrogencarbonat-Lösung gewaschen, über Magnesiumsulfat getrocknet und eingeengt. Hierbei wurden 2,1 g (98%) p-[trans-4-(1,2-Epoxypentyl) cyclohexyl]benzonitril(trans-1,2-Epoxypentyl/cis-1,2-Epoxypentyl-Verhältnis ca. 5:95) als farbloses Öl erhalten; Rf-Werte (Essigester/Petroläther Vol. 10:90): trans-1,2-Epoxypentyl-Isomer 0,17, cis-1,2-Epoxypentyl-Isomer 0,14.

b) In einem Rundkolben mit Tropftrichter wurde unter Argonbegasung eine Lösung von 2,46 g Triphenylphosphin in 30 ml Methylenchlorid bei 0 °C vorgelegt und tropfenweise solange mit einer ca. 1M Lösung vonBrom in Methylenchlorid versetzt, bis eine schwach gelbe Farbe bestehen blieb. Anschliessend wurde die Lösung am Rotationsverdampfer vorsichtig eingeengt und am Hochvakuum nachgetrocknet. Der resultierende, kristalline Rückstand wurde in 30 ml Benzol aufgeschlämmt, mit einer Lösung von 2,1 g p-[trans-4-(1,2-Epoxypentyl) cyclohexyl] benzonitril in 10 ml Benzol versetzt und 3 Stunden zum Rückfluss erhitzt. Filtration der warmen Reaktionslösung an Kieselgel mit Toluol ergab 3,0 g kristallines Rohprodukt, welches nach Niederdruckchromatographie (0,5 bar) an Kieselgel mit Hexan/Toluol (Vol. 1:1) 2,61 g (81%) fast reines p-[trans-4-(erythro-1,2-Dibrompentyl) cyclohexyl] benzonitril als farblose Kristalle lieferte. Durch Umkristallisation aus 90 ml Petroläther/Essigester (Vol. 2:1) wurden schliesslich 2,09 g (65%) sehr reines erythro-Dibromid erhalten; Smp. 140,9 °C.

In analoger Weise wurden folgende Verbindungen hergestellt:

p-[trans-4-(trans-1-Propenyl)cyclohexyl] benzonitril; Smp. (C–N) 66,3 °C, Klp. (N–I) 73,0 °C,

p-[trans-4-(trans-1-Butenyl)cyclohexyl] benzonitril; Smp. (C–N) 45,1 °C, Klp. (N–I) 51,8 °C,

p-[trans-4-(trans-1-Hexenyl)cyclohexyl] benzonitril; Smp. [C–N) 14,4 °C, Klp. (N–I) 39,2 °C,

p-[trans-4-(trans-1-Heptenyl)cyclohexyl] benzonitril; Smp.(C–N) 17,9 °C, Klp. (N–I) 49,2 °C.

Beispiel 9

In analoger Weise zu den Beispielen 1, 5, 6 und 8 wurde 4-[2-(p-Cyanophenyl) äthyl] cyclohexanon in trans-4- [2-(p-Cyanophenyl)äthyl] cyclohexancarboxaldehyd übergeführt und dieser zu p-[2-(trans-4-(trans-1-Alkenyl)cyclohexyl)-äthyl] benzonitrilen weiter umgesetzt.

Das als Ausgangsmaterial verwendete 4-[2-(p-Cyanophenyl)-äthyl]cyclohexanon wurde wie folgt hergestellt:

a) In einem Sulfierkolben wurden unter Rühren und Stickstoffbegasung 149 g Methoxymethyltriphenylphosphoniumchlorid und 860 ml t-Butylmethyläther bei Raumtemperatur vorgelegt, die Suspension auf −10 °C gekühlt und innert 10 Minuten mit 51,6 g Kalium-t-butylat versetzt. Die Suspension wurde noch 30 Minuten bei −10 °C bis 0 °C gerührt und dann innert 45 Minuten bei 0 °C tropfenweise mit einer Lösung von 47,3 g 4,4-Äthylendioxycyclohexanon in 720 ml Tetrahydrofuran versetzt. Die orange Suspension wurde noch 2 Stunden bei Raumtemperatur weiter gerührt, dann auf 5 l Hexan gegossen, 10 Minuten gerührt und genutscht. Das Filtrat wurde im Vakuum eingeengt und das erhaltene gelbbräunliche Öl (104,1 g) mit 500 ml Hexan versetzt und genutscht. Das Filtrat wurde im Vakuum eingeengt, wobei 61,7 g gelbbräunliches Öl erhalten wurden. Chromatographische Trennung dieses Rohproduktes an Kieselgel mit Methylenchlorid/Aceton (Vol. 98:2 und 95:5) ergab schliesslich 53,5 g 1,1-Äthylendioxy-4- (methoxymethylen) cyclohexan als farbloses Öl.

In einem Rundkolben wurde unter Stickstoffbegasung ein Gemisch von 28,2 g 1,1-Äthylendioxy-4-(methoxymethylen)-cyclohexan, 770 ml Eisessig und 385 ml Wasser 1 Stunde zum Rückfluss erhitzt. Danach wurde die gelbliche klare Lösung auf Raumtemperatur gekühlt, mit 800 ml Wasser verdünnt und dreimal mit je 700 ml Methylenchlorid extrahiert. Die organischen Phasen wurden zweimal mit je 500 ml 10%-iger (Gew./

Vol.) Natriumcarbonat-Lösung gewaschen, über Natriumsulfat getrocknet, filtriert und eingeengt. Chromatographische Trennung der erhaltenen bräunlichen Flüssigkeit (18,5 g) an Kieselgel mit Methylenchlorid als Eluens ergab schliesslich 16,7 g 4-Formylcyclohexanon als bräunliche Flüssigkeit.

c) In einem Sulfierkolben wurden unter Rühren und Stickstoffbegasung 63,3 g p-Cyanobenzyltriphenylphosphoniumchlorid, 17,2 g Kalium-t-butylat und 195 ml Äthylenglykoldimethyläther vorgelegt, wobei die Innentemperatur bis auf 44 °C anstieg. Die braune Suspension wurde auf 0 °C gekühlt und innert 2 Minuten mit einer Lösung von 16,7 g 4-Formylcyclohexanon in 100 ml Äthylenglykoldimethyläther versetzt. Danach wurde das Kühlbad entfernt und das Reaktionsgemisch noch 3,5 Stunden bei Raumtemperatur gerührt. Anschliessend wurde die Suspension auf 500 ml Wasser gegossen und dreimal mit je 600 ml Methylenchlorid extrahiert. Die organischen Phasen wurden zweimal mit je 500 ml 10%-iger (Gew./Vol.) Kochsalzlösung gewaschen, über Natriumsulfat getrocknet, filtriert und eingeengt, wobei 76,9 g einer bräunlichen Paste zurückblieben. Chromatographische Trennung dieses Rohproduktes an Kieselgel mit Methylenchlorid als Eluens ergab 33,0 g 4-[2-(p-Cyanophenyl)vinyl]cyclohexanon als gelbbräunliches Öl.

d) In einem Rundkolben mit Magnetrührer wurde ein Gemisch von 33,0 g 4-[2-(p-Cyanophenyl)vinyl]cyclohexanon, 520 ml Toluol, 260 ml Äthanol und 3,2 g Palladium/Kohle (5%) bei Raumtemperatur vorgelegt und das Gemisch bis zum Stillstand der Wasserstoffaufnahme hydriert. Anschliessend wurde die schwarze Suspension genutscht (Nachwaschen mit Toluol) und das Filtrat im Vakuum eingeengt. Das erhaltene, leicht trübe, gelbliche Öl (34,1 g) wurde an Kieselgel chromatographisch getrennt. Methylenchlorid/Hexan (Vol. 1:1), Methylenchlorid/Hexan (Vol. 8:2) und Methylenchlorid eluierten 25,6 g gelbliches Öl, welches aus t-Butylmethyläther kristallisiert wurde. Hierbei wurden 22,6 g 4-[2-(p-Cyanophenyl) äthyl] cyclohexanon als farblose Kristalle mit Smp. 62,5–64,3 °C erhalten.

Auf diese Weise wurden folgende Verbindungen hergestellt:

p-[2-(trans-4-(trans-1-Propenyl)cyclohexyl)-äthyl] benzonitril; Smp. (C–I) 61,3 °C, Klp. (N–I) 54,2 °C,

p-[2-(trans-4-(trans-1-Butenyl)cyclohexyl)-äthyl] benzonitril; Smp. (C–I) 42,6 °C, Klp. (N–I) 39,7 °C,

p-[2-(trans-4-(trans-1-Pentenyl)cyclohexyl)-äthyl] benzonitril; Smp. (C–N) 25,1 °C, Klp. (N–I) 47,5 °C,

p-[2-(trans-4-(trans-1-Hexenyl)cyclohexyl)-äthyl] benzonitril; Smp.(C–N) 16,8 °C bzw. 19,7 °C (2 Modifikationen), Klp. (N–I) 34,6 °C,

p-[2-(trans-4-(trans-1-Heptenyl)cyclohexyl) äthyl]benzonitril; Smp. (C–N) 31,6 °C, Klp. (N–I) 43,6 °C.

Beispiel 10

Ein Gemisch von 200 mg trans-4-(trans-1-Pentenyl) cyclohexanol, 245,9 mg trans-4-Pentylcyclohexancarbonsäure, 293 mg Dicyclohexylcarbodiimid, 14,16 mg 4-(Dimethylamino) pyridin und 3 ml Methylenchlorid wurde 25 Stunden bei Raumtemperatur gerührt. Anschliessend wurde das Reaktionsgemisch mit Diäthyläther verdünnt, der ausgefallene Harnstoff abfiltriert und das Filtrat eingeengt. Der Rückstand wurde in 40 ml Methylenchlorid aufgenommen und mit 5%-iger Salzsäure, Natriumhydrogencarbonat-Lösung und Kochsalzlösung gewaschen. Die wässrigen Phasen wurden mit Methylenchlorid nachextrahiert. Die organischen Phasen wurden über Magnesiumsulfat getrocknet, filtriert und eingeengt. Niederdruckchromatographie des erhaltenen, weissen Rückstandes (450 mg) an Kieselgel mit Diäthyläther/Petroläther (Vol. 3:97) ergab 348 mg weisse, durchsichtige Nadeln, welche aus 20 ml Methanol umkristallisiert wurden. Hierbei wurden 289 mg trans-4-Pentylcyclohexancarbonsäure-trans-4-(trans-1-pentenyl) cyclohexylester erhalten; Smp. (C–S) 39,8 °C, Klp. (S–I) 66,5 °C.

Das als Ausgangsmaterial verwendete trans-4-(trans-1-Pentenyl) cyclohexanol wurde wie folgt hergestellt:

a) 261,2 g Methoxymethyl-triphenylphosphoniumchlorid wurden in 550 ml t-Butylmethyläther aufgeschlämmt und bei −10 °C mit 90,53 g Kalium-t-butylat versetzt. Das Kühlbad wurde entfernt und das Gemisch noch 1 Stunde bei Raumtemperatur gerührt. Anschliessend wurde die Suspension bei −10 °C langsam mit einer Lösung von 70 g 4,4-Äthylendioxycyclohexanon in 350 ml Tetrahydrofuran versetzt, noch 1 Stunde bei Raumtemperatur gerührt, dann mit Wasser versetzt und dreimal mit Diäthyläther extrahiert. Die organischen Phasen wurden zweimal mit Wasser gewaschen und die wässrigen Phasen mit Diäthyläther nachextrahiert. Die vereinigten organischen Phasen wurden über Magnesiumsulfat getrocknet und eingeengt. Der erhaltene, kristalline Rückstand wurde in Essigester gelöst, mit Petroläther verdünnt, filtriert und vom Lösungsmittel befreit. Destillation des erhaltenen, gelben Öles (100 g) ergab im Hauptlauf (71 °C/ 0,20–0,17 Torr) 75,28 g (91,17%) 1,1-Äthylendioxy-4- (methoxymethylen) cyclohexan als klare, farblose Flüssigkeit.

b) Ein Gemisch von 10,55 g 1,1-Äthylendioxy-4-(methoxymethylen) cychexan, 130 ml Wasser und 200 ml Eisessig wurde 1 Stunde zum Rückfluss erhitzt. Anschliessend wurde am Rotationsverdampfer das Lösungsmittel abdestilliert und das Destillat zweimal mit Methylenchlorid extrahiert. Der Destillationsrückstand (gelbes Öl) wurde mit 200 ml Wasser verdünnt, mit Natriumcarbonat-Lösung neutralisiert und dreimal mit Methylenchlorid extrahiert. Die organischen Phasen wurden mit gesättigter Natriumcarbonat-Lösung gewaschen und die wässrigen Phasen mit Methylenchlorid nachextrahiert. Die organischen Phasen wurden über Magnesiumsulfat getrocknet und eingedampft. Destillation des zurückbleibenden, gelben Öles ergab bei 70 °C/0,15 Torr 6,7 g (93%) 4-Formylcyclohexanon.

c) Eine Lösung von 36,72 g Triphenylphosphin in 200 ml Methylenchlorid wurde bei −20 °C langsam mit 23,22 g Tetrabromkohlenstoff versetzt und noch 10 Minuten gerührt. Anschliessend wurde das Gemisch mittels einer Kanüle zu einer auf −60 °C gekühlten LÖsung von 6,30 g 4-Formylcyclohexanon in 100 ml Methylenchlorid zugetropft. Das Reaktionsgemisch wurde noch 15 Minuten bei −60 °C gerührt und dann in Wasser/Methylenchlorid verteilt. Die wässrigen Phasen wurden noch zweimal mit Methylenchlorid extrahiert. Die organischen Phasen wurden zweimal mit Wasser gewaschen, über Magnesiumsulfat getrocknet und eingedampft. Niederdruckchromatographie des erhaltenen hellgelben Öles (16 g) an Kieselgel mit Essigester/Petroläther (Vol. 10:90) ergab 12,08 g (85,8%) 4-(2,2-Dibromvinyl) cyclohexanon als hellgelbe Flüssigkeit.

d) Ein Gemisch von 2 g 4-(2,2-Dibromvinyl)-cyclohexanon, 3,43 g Äthylenglykol, 0,202 g p-Troluolsulfonsäure und 240 ml Benzol wurde unter Wasserabscheidung 5 Stunden am Rückfluss gekocht. Anschliessend wurde das Reaktionsgemisch mit Kaliumcarbonat versetzt, kurz gerührt und über Nacht stehen gelassen. Danach wurde das Gemisch filtriert und das Filtrat am Rotationsverdampfer vom Lösungsmittel befreit. Der Rückstand wurde in 200 ml Methylenchlorid aufgenommen und zweimal mit verdünnter Natronlauge und einmal mit Wasser gewaschen. Die wässrigen Phasen wurden mit Methylenchlorid nachextrahiert. Die organischen Phasen wurden über Mangesiumsulfat getrocknet, filtriert und eingedampft. Hierbei wruden 14 g 1,1-Äthylendioxy-4-(2,2-dibromvinyl) cyclohexan als leicht gelbe, kristallisierende Flüssigkeit erhalten.

e) Eine Lösung von 14 g 1,1-Äthylendioxy-4-(2,2-dibromvinyl) cyclohexan in 70 ml Tetrahydrofuran wurde auf −20 °C gekühlt und bei dieser Temperatur langsam mit 76,62 ml einer 1,4 M Lösung von Butyllithium in Hexan versetzt (exotherme Reaktion). Das Kühlbad wurde entfernt und das Gemisch innert etwa 20 Minuten auf 20 °C erwärmen gelassen. Anschliessend wurde das Reaktionsgemisch mit 150 ml Wasser versetzt und dreimal mit Diäthyläther extrahiert. Die organischen Phasen wurden zweimal mit Wasser gewaschen und die Waschwasser mit Diäthyläther nachextrahiert. Die organischen Phasen wurden über Magnesiumsulfat getrocknet, filtriert und eingedampft. Niederdruckchromatographie der zurückbleibenden, gelben Flüssigkeit (8 g) an Kieselgel mit Essigester/Petroläther (Vol. 7:93) ergab 6,5 g (91%) 4-Äthyl-1,1-äthylendioxycyclohexan als klare Flüssigkeit.

f) Eine Lösung von 6,5 g 4-Äthinyl-1,1-äthylendioxycyclohexan in40 ml Tetrahydrofuran wurde bei −20 °C mit 39,1 ml einer 1,4 M Lösung von Butyllithium in Hexan versetzt. Anschliessend wurde das Gemisch bei 0 °C mit 60 ml Hexamethylphosphorsäuretriamid (kurzer Temperaturanstieg auf 26 °C) und dann tropfenweise mit 6,5 ml Propyljodid versetzt. Das Kühlbad wurde entfernt und das Gemisch auf Raumtemperatur erwärmen gelassen. Hierbei bildete sich ein weisser Niederschlag. Nach 30 Minuten wurde das Reaktionsgemisch mit 150 ml Wasser versetzt und dreimal mit Hexan extrahiert. Die organischen Phasen wurden dreimal mit Wasser gewaschen und die Waschwasser mit Hexan nachextrahiert. Die organischen Phasen wurden über Magnesiumsulfat getrocknet, filtriert und am Rotationsverdampfer vom Lösungsmittel befreit. Niederdruckchromatographie der erhaltenen gelben Flüssigkeit (9 g) an Kieselgel mit Essigester/Petroläther (Vol. 10:90) und Behandlung mit Aktivkohle ergab 6,23 g (76,5%) 1,1-Äthylendioxy-4- (1-pentinyl) cyclohexan als leicht hellgelbe Flüssigkeit.

g) In einem Sulfierkolben mit Magnetrührer wurde eine Lösung von 4,5 g 1,1-Äthylendioxy-4- (1-pentinyl) cyclohexan in 54 ml Tetrahydrofuran tropfenweise mit ca. 50 ml vorkondensiertem Ammoniak versetzt. Anschliessend wurde das Gemisch bei −78 °C innert 7 Stunden portionenweise mit 1,3 g Natrium versetzt. 1,5 Stunden nach der letzten Zugabe wurde das Ammoniak abgedampft und das Reaktionsgemisch mit 25%-iger Salzsäure neutralisiert und über Nacht stehen gelassen. Danach wurde das Reaktionsgemisch dreimal in Wasser/Diäthyläther verteilt. Die wässrigen Phasen wurden mit Diäthyläther nachextrahiert. Die organischen Phasen wurden über Magnesiumsulfat getrocknet und am Rotationsverdampfer vom Lösungsmittel befreit. Die erhaltene, hellgelbe Flüssigkeit (3,8 g) wurde mit 200 ml Aceton und 0,1 ml konzentrierter Schwefelsäure versetzt. Das Gemisch wurde 10 Minuten zum Rückfluss erhitzt, dann mit Wasser versetzt und am Rotationsverdampfer vom Aceton befreit. Der Rückstand wurde dreimal in Methylenchlorid/Wasser verteilt. Die wässrigen Phasen wurden mit Methylenchlorid nachextrahiert. Die organischen Phasen wurden über Magnesiumsulfat getrocknet und eingedampft. Chromatographische Trennung der zurückbleibenden, gelben Flüssigkeit (3,5 g) an Kieselgel mit Essigester/Petroläther (Vol. 7:93) ergab 3,0 g (83,5%) 4-(trans-1-Pentenyl) cyclohexanon als hellgelbe Flüssigkeit.

h) 1,63 g 4-(trans-1-Pentenyl) cyclohexanon wurden in 8 ml Diäthyläther und 14 ml Äthanol gelöst. Anschliessend wurde die Lösung tropfenweise mit ca. 70 ml Ammoniak versetzt und portionenweise solange mit Lithiumdraht versetzt, bis die Farbe des Reaktionsgemisches 1,5 Stunden lang konstant blieb (ca. 1,3 g Lithium). Danach wurde das Ammoniak abgedampft und das Gemisch mit Ammoniumchlorid und Salzsäure sauer gestellt und 3 Tage stehengelassen. Dann wurde das Reaktionsgemisch in Diäthyläther/Wasser verteilt und die wässrigen Phasen mit Diäthyläther nachextrahiert. Die organischen Phasen wurden über Magnesiumsulfat getrocknet, filtriert und am Rotationsverdampfer vom Lösungsmittel befreit. Niederdruckchromatographie

des zurückbleibenden, gelben Öles an Kieselgel mit Essigester/Petroläther (Vol. 10:90) ergab 1,47 g (89,1%) trans-4-(trans-1-Pentenyl) cyclohexanol als leicht gelbes, viskoses Öl.

In analoger Weise wurden folgende Verbindungen hergestellt:

trans-4-Propylcyclohexancarbonsäure-trans-4-(trans-1-pentenyl) cyclohexylester; Smp. (C–S) 23,8 °C, Klp. (S–I) 50,7 °C,

trans-4-Butylcyclohexancarbonsäure-trans-4-(trans-1-pentenyl) cyclohexylester; Smp. (C–S) 26,2 °C, Klp. (S–I) 65,0 °C.

Beispiel 11

Ein Gemisch von 800 mg trans-4- (trans-1-Pentenyl)- cyclohexancarbonsäure, 675,64 mg p-Äthoxyphenol, 1,02 g Dicyclohexylcarbodiimid und 49,79 mg 4-(Dimethylamino) pyridin wurden in 14,5 ml Methylenchlorid aufgeschlämmt und bei Raumtemperatur 20 Stunden gerührt. Anschliessend wurde das Reaktionsgemisch mit Diäthyläther verdünnt, der ausgefallene Harnstoff abfiltriert und das Filtrat eingeengt. Der Rückstand wurde in Hexan aufgenommen und mit verdünnter Salzsäure, Natriumhydrogencarbonat-Lösung und Wasser gewaschen. Die wässrigen Phasen wurden zweimal mit Hexan nachextrahiert. Die organischen Phasen wurden über Magnesiumsulfat getrocknet und eingedampft. Niederdruckchromatographie des erhaltenen Rückstandes an Kieselgel mit Essigester/Petroläther (Vol. 3:97) ergab 1,080 g (83,8%) trans-4-(trans-1-Pentenyl) cyclohexancarbonsäure-p-äthoxyphenylester. Nach Umkristallisation aus 40 ml Hexan wurden schliesslich 880 mg Produkt in Form durchsichtiger Kristalle erhalten; Smp. (C–N) 57,2 °C; Klp. (N–I) 93,1.

Die als Ausgangsmaterial verwendete trans-4-(trans-1-Pentenyl) cyclohexancarbonsäure wurde wie folgt hergestellt:

a) 7 g Methoxymethyl-triphenylphosphoniumchlorid wurden in 35 ml t-Butylmethyläther aufgeschlämmt und bei −20 °C mit 2,43 g Kalium-t-butylat versetzt. Das Gemisch wurde noch 1 Stunde bei Raumtemperatur gerührt, dann bei −20 °C tropfenweise mit einer Lösung von 2 g 4-(trans-1-Pentenyl) cyclohexanon in 18 ml Tetrahydrofuran versetzt und nochmals 1 Stunde bei Raumtemperatur gerührt. Anschliessend wurde das Reaktionsgemisch mit Wasser versetzt und dreimal mit je 50 ml Diäthyläther extrahiert. Die Extrakte wurden mit Wasser gewaschen und die Waschwasser mit Diäthyläther nachextrahiert. Die organischen Phasen wurden über Magnesiumsulfat getrocknet und vom Lösungsmittel befreit. Der Rückstand wurde in Essigester gelöst und die Lösung mit Petroläther verdünnt, durch Filtration vom ausgefallenen Triphenylphosphinoxid befreit und erneut eingeengt. Dieser Vorgang zur Abtrennung von Triphenylphosphinoxid wurde noch zweimal wiederholt und das erhaltene Rohprodukt von 1-(Methoxymethylen) -4- (trans-1-pentenyl) cyclohexan ohne zusätzliche Reinigung weiterverwendet.

b) 2,75 g 1-(Methoxymethylen)-4-(trans-1-pentenyl) cyclohexan (Rohprodukt aus Absatz a) wurden mit 100 ml Tetrahydrofuran/2N Salzsäure (Vol. 4:1) 30 Minuten zum Rückfluss erhitzt und dann über Nacht bei Raumtemperatur gerührt. Anschliessend wurde das Gemisch mit 100 ml Wasser versetzt und dreimal mit je 100 ml Diäthyläther extrahiert. Die Extrakte wurden mit verdünnter Natriumhdrogencarbonat-Lösung und Wasser gewaschen und die wässrigen Phasen mit Diäthyläther nachextrahiert. Die organischen Phasen wurden über Magnesiumsulfat getrocknet und eingedampft. Niederdruckchromatographie der zurückbleibenden gelblichen Flüssigkeit (2,5 g an Kieselgel mit Petroläther und Essigester/Petroläther (Vol. 3:97) ergab 1,65 g (76%) 4-(trans-1-Pentenyl) cyclohexancarboxaldehyd.

c) Eine Lösung von 1,6 g 4-(trans-1-Pentenyl) cyclohexancarboxyldehyd in 120 ml Aceton wurde auf −10 °C gekühlt, tropfenweise mit 8N Chromsäure (ca. 10 ml) versetzt, bis die Farbe des Reaktionsgemisches braunorange blieb, und noch 1 Stunde gerührt. Überschüssige Chromsäure wurde durch Zugabe von Isopropanol reduziert. Anschliessend wurde die grüne Lösung dreimal in Wasser/Methylenchlorid verteilt. Die organischen Extrakte wurden zweimal mit Wasser gewaschen und die Waschwasser mit Methylenchlorid nachextrahiert. Die organischen Phasen wurden über Magnesiumsulfat getrocknet und eingedampft. Der hellbraune kristalline Rückstand (2,01 g) wurde in 20 ml Petroläther teilweise gelöst. Ungelöster Rückstand wurde abfiltriert und das Filtrat eingedampft. Umkristallisation des erhaltenen Rückstandes aus 60 ml Petroläther bei −78 °C ergab 866 mg (49,5%) 4-(trans-1-Pentenyl) cyclohexancarbonsäure als weisse Kristalle.

d) Ein Gemisch von 1,29 g 4-(trans-1-Pentenyl) cyclohexancarbonsäure und 20 ml einer 11%-igen (Gew./Vol.) Lösung von Kaliumhydroxid in Diäthylenglykol wurde unter Argonbegasung 20 Stunden am Rückfluss gekocht. Anschliessend wurde das Reaktionsgemisch mit 25%-iger Salzsäure schwach sauer gestellt und dreimal in Wasser/Methylenchlorid verteilt. Die organischen Extrakte wurden zweimal mit Wasser gewaschen und die Waschwasser mit Methylenchloird nachextrahiert. Die organischen Phasen wurden über Magnesiumsulfat getrocknet und am Rotationsverdampfer vom Lösungsmittel befreit. Umkristallisation des erhaltenen, dunkelbraunen, kristallisierenden Öles (1,17 g) aus 50 ml Petroläther bei −78 °C ergab 0,44 g trans-4-(trans-1-Pentenyl)- cyclohexancarbonsäure als hellbraune Kristalle. Die Mutterlauge enthaltend 0,695 g rohe cis/trans-4-(trans-1-Pentenyl) cyclohexancarbonsäure wurde nicht aufgearbeitet.

Beispiel 12

In einem Sulfierkolben mit mechanischem Rührer wurde unter Argonbegasung eine Suspension von 6,64 g Methyltriphenylphosphoniumbromid

in 80 ml t-Butylmethyläther bei −10 °C innert 3 Minuten mit 2,12 g festem Kalium-t-butylat versetzt. Das Gemisch wurde noch 1 Stunde bei Raumtemperatur gerührt, dann bei 0 °C innert 5 Minuten mit einer Lösung von 3,0 g 3-[trans-4-(p-Cyanophenyl) cyclohexyl] propionaldehyd in 20 ml t-Butylmethyläther versetzt und noch 15 Stunden bei Raumtemperatur gerührt. Anschliessend wurde das Reaktionsgemisch dreimal in Diäthyläther/Wasser verteilt. Die organischen Extrakte wurden zweimal mit Wasser gewaschen, über Magnesiumsulfat getrocknet, filtriert und eingedampft. Zur Abtrennung von Triphenylphosphinoxid wurde der Rückstand in Essigester gelöst und die Lösung mit Petroläther verdünnt, filtriert und eingedampft. Chromatographische Trennung des erhaltenen, hellbraunen Öles (4,38 g) an Kieselgel mit Essigester/Petroläther (Vol. 3:97) ergab 2,83 g weisse Kristalle. Umkristallisation aus Methanol und Aufarbeitung der Mutterlauge ergab schliesslich insgesamt 2,116 g p-[trans-4- (3-Butenyl) cyclohexyl]benzonitril als weisse Kristalle; Smp. (C–N) 49,5 °C, Klp. (N–I) 52,5 °C.

Der als Ausgangsmaterial verwendete 3-[trans-4-(p-Cyanophenyl) cyclohexyl] propionaldehyd wurde wie folgt hergestellt:

a) In einem Sulfierkolben mit mechanischem Rührer wurde unter Argonbegasung eine Suspension von 29,0 g Methoxymethyl-triphenylphosphoniumchlorid in 200 ml t-Butylmethyläther bei −10 °C innert 3 Minuten mit 9,7 g Kalium-t-butylat versetzt. Die orange Suspension wurde noch 1 Stunde bei ca. 0 °C gerührt, dann bei −10 °C innert 10 Minuten tropfenweise mit einer Lösung von 12,0 g trans-4- (p-Cyanophenyl) cyclohexancarboxaldehyd in 90 ml t-Butylmethyläther versetzt und noch 45 Minuten bei 0 °C gerührt. Anschliessend wurde das Reaktionsgemisch dreimal in Diäthyläther/Wasser verteilt. Die organischen Extrakte wurden zweimal mit Wasser gewaschen, über Magnesiumsulfat getrocknet, filtriert und eingedampft. Zur Abtrennung von Triphenylphosphinoxid wurde der Rückstand im Essigester gelöst und die Lösung mit Petroläther verdünnt, filtriert und eingedampft. Chromatographische Trennung des gelblichen, kristallinen Rückstandes (16,3 g) an Kieselgel mit Essigester/Petroläther (Vol. 5:95) ergab 10,1 g (74%) p-[trans-4-(2-Methoxyvinyl) cyclohexyl] benzonitril als weisse Kristalle.

b) Eine Lösung von 10,1 g p-[trans-4-(2-Methoxyvinyl)-cyclohexyl]benzonitril in 200 ml Tetrahydrofuran/2N Salzsäure (Vol. 4:1) wurde unter Rühren 1 Stunde zum Rückfluss erhitzt. Anschliessend wurde das Reaktionsgemisch dreimal in Diäthyläther/Wasser verreilt. Die organischen Extrakte wurden zweimal mit Wasser gewaschen, über Magnesiumsulfat getrocknet, filtriert und eingedampft, wobei 9,8 g 2-[trans-4- (p-Cyanophenyl) cyclohexyl] acetaldehyd als leicht gelblicher, kristalliner Rückstand erhalten wurden.

c) In einem Sulfierkolben mit mechanischem Rührer wurde unter Argonbegasung eine Suspension von 22,2 g Methoxymethyl-triphenylphosphoniumchlorid in 150 ml t-Butylmethyläther bei 0 °C innert 3 Minuten mit 7,4 g festem Kalium-t-butylat versetzt. Die organische Suspension wurde noch 1 Stunde bei 0 °C gerührt und dann innert 10 Minuten tropfenweise mit einer Lösung von 9,8 g 2-[trans-4- (p-Cyanophenyl) cyclohexyl]acetaldehyd in 100 ml Tetrahydrofuran versetzt. Anschliessend wurde die Suspension unter Rühren langsam auf Raumtemperatur erwärmen gelassen. Nach 15 Stunden wurde die Suspension dreimal in Diäthyläther/Wasser verteilt. Die organischen Extrakte wurden zweimal mit Wasser gewaschen, über Magnesiumsulfat getrocknet, filtriert und eingedampft. Zur Abtrennung von Triphenylphosphinoxid wurde der Rückstand in Essigester gelöst und die Lösung mit Petroläther verdünnt, filtriert und eingedampft. Chromatographische Trennung des zurückbleibenden gelblichen Öles (13,7 g) an Kieselgel mit Essigester/Petroläther (Vol. 5:95) ergab 10,5 g (96%) p-[trans-4-(3-Methoxy-2-propenyl) cyclohexyl]- benzonitril] als farbloses Öl.

d) Eine Lösung von 10,5 g p-[trans-4-(3-Methoxy-2-propenyl)- cyclohexyl] benzonitril in 200 ml Tetrahydrofuran/2N Salzsäure (Vol. 4:1) wurde unter Rühren 45 Minuten zum Rückfluss erhitzt. Danach wurde das Reaktionsgemisch dreimal in Diäthyläther/Wasser verteilt. Die organischen Extrakte wurden zweimal mit Wasser gewaschen, über Magnesiumsulfat getrocknet, filtriert und eingedampft. Chromatographische Trennung des weissen, kristallinen Rückstandes (9,9 g) an Kieselgel mit Essigester/Petroläther (Vol. 10:90 und 30:70) ergab schliesslich 9,4 g (95%) 3-[trans-4-(p-Cyanophenyl) cyclohexyl] propionaldehyd als weisse Kristalle.

Beispiel 13

In einem Rundkolben mit Magnetrührer wurde unter Argonbegasung eine Lösung von 9,54 g p-[trans-4-(erythro-3,4-Dibrompentyl) cyclohexyl] benzonitril in 100 ml Eisessig mit 9,8 g Zink-Pulver versetzt. Das Gemisch wurde 30 Minuten bei Raumtemperatur gerührt und dann dreimal in Petroläther/Wasser verteilt. Die organischen Extrakte wurden zweimal mit Wasser gewaschen, über Magnesiumsulfat getrocknet, filtriert und eingeengt. Chromatographische Trennung des öligen Rückstandes (5,56 g) an mit Silbernitrat beschichtetem Kieselgel (hergestellt durch Aufschlämmen von 300 g Kiesegel in 500 ml einer 0,2M Lösung von Silbernitrat in Acetonitril, anschliessende Filtration und Trocknung des Rückstandes) unter Verwendung von Diäthyläther/Hexan (Vol. 1:9) als Eluens ergab 3,2 g Rohprodukt als weisse Kristalle. Nach Umkristallisation aus 80 ml Methanol wurden 1,65 g (28%) p-[trans-4-(trans-3-Pentenyl) cyclohexyl] benzonitril als weisse Kristalle erhalten. Die Mutterlauge und die unreinen Fraktionen aus der chromatographischen Trennung wurden vereinigt und nochmals an mit Silbernitrat beschichteten Kieselgel unter Verwendung von Diäthyläther/Hexan (Vol. 1:9) als Eluens gereinigt. Umkristallisation des erhaltenen, kristallinen Rohproduktes (1,5 g) aus 40 ml Methanol ergab weitere 0,65 g p-[trans-4- (trans-3-Pentenyl)- cyclohexyl] benzonitril als weisse Kristalle; Smp. (C–N) 59,8 °C, Klp. (N–I) 73,7 °C.

Das als Ausgangsmaterial verwendete p-[trans-4-(erythro-3,4-Dibrompentyl) cyclohexyl] benzonitril wurde wie folgt hergestellt:

a) In einem Sulfierkolben mit mechanischem Rührer wurde unter Argonbegasung eine Suspension von 14,8 g Äthyltriphenylphosphoniumbromid in 150 ml t-Butylmethyläther bei −10 °C innert 5 Minuten mit 4,54 g festem Kalium-t-butylat versetzt. Die Suspension wurde noch 1 Stunde bei Raumtemperatur gerührt, dann bei 0 °C innert 5 Minuten tropfenweise mit einer Lösung von 6,4 g 3-[trans-4-(p-Cyanophenyl) cyclohexyl7 propionaldehyd in 40 ml t-Butylmethyläther versetzt und noch 15 Stunden bei Raumtemperatur gerührt. Anschliessend wurde das Reaktionsgemisch dreimal in Diäthyläther/Wasser verteilt. Die organischen Extrakte wurden zweimal mit Wasser gewaschen, über Magnesiumsulfat getrocknet, filtriert und eingeengt. Zur Abtrennung von Triphenylphosphinoxid wurde der Rückstand in Essigester gelöst und die Lösung mit Petroläther verdünnt, filtriert und eingeengt. Chromatographische Trennung des zurückbleibenden gelblichen Öles (8,55 g) an Kieselgel mit Essigester/Petroläther (Vol. 3:97) ergab 5,93 g (89%) p-[trans-4-(3-Pentenyl)cyclohexyl] benzonitril als weisse Kristalle.

b) Eine Lösung von 4,49 g 90%-iger m-Chlorperbenzoesäure in 100 ml Methylenchlorid wurde mit 11,3 g gemahlenem Kaliumcarbonat versetzt. Das Gemisch wurde bei 0 °C innert 5 Minuten tropfenweise mit einer Lösung von 5,93 g p-[trans-4- (3-Pentenyl) cyclohexyl] benzonitril in 20 ml Methylenchlorid versetzt und noch 2 Stunden bei Raumtemperatur gerührt. Anschliessend wurde das Gemisch nochmals mit 4,49 g 90%-iger m-Chlorperbenzoesäure versetzt und weitergerührt. Nach insgesamt 70 Stunden wurde das Reaktionsgemisch dreimal in Methylenchlorid/10%-iger Natriumthiosulfat-Lösung verteilt. Die organischen Extrakte wurden mit Natriumthiosulfat-Lösung und Wasser gewaschen, über Magnesiumsulfat getrocknet, filtriert und eingeengt. Chromatographische Trennung des zurückbleibenden, leicht gelblichen Öles (6,3 g) an Kieselgel mit Essigester/Petroläther (Vol. 10:90) ergab 6,27 g (99,5%) p-[trans-4-(3,4-Epoxypentyl)-cyclohexyl] benzonitril als farbloses Öl.

c) Eine Lösung von 7,4 g Triphenylphosphin in 80 ml Methylenchlorid wurde bei 0 °C unter Argonbegasung tropfenweise mit einer Lösung von 1,5 ml Brom in 20 ml Methylenchlorid versetzt, bis die Gelbfärbung bestehen blieb. Danach wurde das Gemisch am Rotationsverdampfer eingedampft und der Rückstand 1 Stunde am Hochvakuum getrocknet. Der gelbe, kristalline Rückstand wurde in 120 ml Benzol aufgeschlämmt. Die Suspension wurde mit 6,27 g p-[trans-4(3,4-Epoxypentyl) cyclohexyl] benzonitril versetzt und unter Rühren 1 Stunde zum Rückfluss erhitzt. Anschliessend wurde das Reaktionsgemisch an Kieselgel mit Toluol als Eluens filtriert. Einengen der produkthaltigen Fraktionen ergab schliesslich 9,54 g (99,1%) p-[trans-4- (erythro-3,4-Dibrompentyl) cyclohexyl] benzonitril als leicht bräunliches Öl.

Beispiel 14

Eine Lösung von Methylmangnesiumjodid in Diäthyläther (hergestellt aus 384 g Magnesium-Späne und 0,984 ml Methyljodid in 30 ml Diäthyläther) wurde bei Raumtemperatur tropfenweise mit einer Lösung von 2,0 g p-[trans-4-(trans-1-Pentenyl) cyclohexyl] benzonitril versetzt. Das Gemisch wurde 15 Minuten zum Rückfluss erhitzt. Anschliessend wurden 30 ml Toluol zum Reaktionsgemisch zugegeben, der Diäthyläther abdestilliert und das Gemisch nochmals 1,5 Stunden zum Rückfluss erhitzt. Danach wurde das Reaktionsgemisch bei 0 °C vorsichtig mit gesättigter Ammoniumchlorid-Lösung versetzt und dreimal in Diäthyläther/Wasser verteilt. Die organischen Extrakte wurden zweimal mit Wasser gewaschen, über Magnesiumsulfat getrocknet, filtriert und eingedampft. Chromatographische Trennung des gelben kristallinen Rückstandes (2,6 g) an Kieselgel mit Essigester/Petroläther (Vol. 5:95) ergab 1,85 g (87%) p-[trans-4-(trans-1-Pentenyl) cyclohexyl] acetophenon als leicht gelbe Kristalle.

Beispiel 15

Eine Lösung von 1,35 g p-[trans-4- (trans-1-Pentenyl)cyclohexyl]acetophenon in 16 ml Äthanol und 16 ml Diäthylenglykol wurde unter Argonbegasung mit 0,486 ml Hydrazinhydrat versetzt und dann unter Rühren 1,5 Stunden zum Rückfluss erhitzt (Badtemperatur 110 °C). Anschliessend wurde das Gemisch mit 550 mg festem Kaliumhydroxid versetzt, die Badtemperatur auf 210 °C erhöht und der Ähtnaol abdestilliert. Nach 2,5 Stunden bei 210 °C wurde die Reaktion abgebrochen und das Gemisch dreimal in Wasser/Petroläther verteilt. Die organischen Extrakte wurden zweimal mit Wasser gewaschen, über Magnesiumsulfat getrocknet und eingedampft. Chromatographische Trennung des Rückstandes (1,23 g) an Kieselgel mit Hexan als Eluens ergab 1,16 g (91%) 4-Äthyl-1- [trans-4- (trans-1-pentenyl) cyclohexyl] benzol als farbloses Öl; Smp. (C–I) −3,1 °C.

In analoger Weise wurden folgende Verbindungen hergestellt:

4-Propyl-1-[trans-4-(trans-1-pentenyl)cyclohexyl]-benzol; Smp. (C–I) 7,0 °C,

4-Äthyl-1-[2-(trans-4-(trans-1-propenyl) cyclohexyl)-äthyl-]benzol; Smp. (C–I) 1,7 °C, Klp. −26 °C.

Beispiel 16

Eine Suspension von 404,4 mg Diphosphortetrajodid in 5 ml Methylenc orid wurde unter Argonbegasung bei Raumtemperatur innert 5 Minuten tropfenweise mit einer Lösung von 205 mg

4-Äthoxy-1- [trans-4- (1,2-epoxypently) cyclohexyl] benzol und 0,703 ml Pyridin in 8 ml Methylenchlorid versetzt. Die Suspension wurde unter Rühren zum Rückfluss erhitzt, wobei nach 2 Stunden nochmals 404,4 mg Diphosphortetrajodid zugegeben wurden. Nach insgesamt 19 Stunden Rühren unter Rückfluss wurde die Reaktion abgebrochen und das Gemisch mit 1N Salzsäure versetzt und dreimal mit Diäthyläther extrahiert. Die organischen Extrakte wurden nacheinander mit 1N Salzsäure, Natriumthiosulfat-Lösung und Wasser gewaschen, über Magnesiumsulfat getrocknet, filtriert und eingedampft. Chromatographische Trennung des Rückstandes (0,2 g) an Kieselgel mit Essigester/Petroläther (Vol. 3:97) als Eluens ergab 120 mg 4-Äthoxy-1- [trans-4-(trans-1-pentenyl) cyclohexyl]benzol. Nach Umkristallisation aus 10 ml Methanol wurde das Produkt als weisse Kristalle mit Smp. (C–N) 32,2 °C und Klp. (N–I) 54,9 °C erhalten.

Das als Ausgangsmaterial verwendete 4-Äthoxy-1- [trans-4- (1,2-epoxypently) cyclohexyl] benzol wurde wie folgt hergestellt:

a) Eine Lösung von 2,63 g p-[trans-4- (trans-1-Pentenyl) -cyclohexyl] acetophenon in 90 ml Methylenchlorid wurde bei 0 °C unter Argobegasung nacheinander mit 7,46 g m-Chlorperbenzoesäure und 100 mg 2,6-Di-t-butyl-p-kresol versetzt. Das Gemisch wurde unter Lichtausschluss bei Raumtemperatur 40 Stunden gerührt. Anschliessend wurde das Reaktionsgemisch in Methylenchlorid/10%-iger Natriumthiosulfat-Lösung verteilt und die organische Phase einmal mit 10%-iger Natriumthiosulfat-Lösung und zweimal mit Natriumhydrogencarbonat-Lösung gewaschen. Die wässrigen Phasen wurden dreimal mit Methylenchlorid nachextrahiert. Die vereinigten organischen Phasen wurden über Magnesiumsulfat getrocknet, filtriert und eingedampft. Der gelbe, ölige Rückstand von 4-Acetoxy-1- [trans-4- (1,2-epoxypentyl) cyclohexyl) benzol wurde in 100 ml 1N methanolischer Kaliumhydroxid-Lösung gelöst und 1 Stunde bei Raumtemperatur gerührt. Anschliessend wurde das Gemisch mit 10 ml 25%-iger Salzsäure auf ca. pH 8 gestellt und in Diäthyläther/Wasser verteilt. Die wässrige Phase wurde noch dreimal mit Diäthyläther extrahiert. Die organischen Phasen wurden über Magnesiumsulfat getrocknet, filtriert und eingedampft. Chromatographische Trennung des zurückbleibenden braunen Öles (2,96 g) an Kieselgel mit Essigester/Petroläther (Vol. 10:90) ergab 2,16 g p-[trans-4- (1,2-epoxypentyl) cyclohexyl]phenol als gelbliche Kristalle.

b) Eine Lösung von 1,6 g p-[trans-4-(1,2-epoxypently)- cyclohexyl]phenol in 120 ml Aceton wurde unter Argonbegasung mit 1,89 ml Äthyljodid und 3,24 g gemahlenem Kaliumcarbonat versetzt. Das Gemisch wurde unter Rühren 24 Stunden zum Rückfluss erhitzt, dann am Rotationsverdampfer eingedampft und der Rückstand in Diäthyläther/Wasser verteilt. Die wässrige Phase wurde noch dreimal mit Diäthyläther extrahiert. Die organischen Phasen wurden zweimal mit Wasser gewaschen, über Magnesiumsulfat getrocknet, filtriert und eingedampft, Chromatographische Trennung des zurückbleibenden gelblichen Öles (1,76 g) an Kieselgel mit Essigester/Petroläther (Vol. 5:95) ergab 1,45 g (82%) 4-Äthoxy-1- [trans-4- (1,2-epoxypentyl) cyclohexyl]benzol als ölige Flüssigkeit.

Beispiel 17

Ein Gemisch von 1,00 g 2-(trans-1-Pentenyl)-1,3-propandiol 1,11 g p-Butyloxybenzaldehyd, 3 Tropfen 2N Schwefelsäure und 40 ml Toluol wurde 2 Stunden unter Wasserabscheidung zum Rückfluss erhitzt. Anschliessend wurde das Gemisch mit 7 Tropfen Triäthylamin versetzt, abkühlen gelassen, mit 5 ml gesättigter Natriumhydrogencarbonat-Lösung und dreimal mit je 10 ml Wasser gewaschen, über Natriumcarbonat getrocknet, filtriert und eingeengt. Der semikristalline Rückstand (1,86 g) wurde an Kieselgel mit Hexan/Diäthyläther (Vol. 97:3) chromatographiert. Die produkthaltigen Fraktionen wurden gesammelt (0,99 g) und zweimal bei −25 °C aus Hexan umkristallisiert. Es resultierten 0,44 g reines trans-2- (p-Butyloxyphenyl) -5- (trans-1-pentenyl) -m-dioxan; Smp. (C–N) 60,6 °C, Klp. (N–I) 61,9 °C.

Das als Ausgangsmaterial verwendete 2-(trans-1-Pentenyl) -1,3-propandiol wurde wie folgt hergestellt:

Zu einer Suspension von 5,3 g Lithiumaluminiumhydrid in 200 ml trockenem Tetrahydrofuran wurde unter Rühren in Inertgasatmosphäre innert 1 Stunde bei 5 °C eine Lösung von 16,1 g 2-(trans-1-Pentenyl) malonsäurediäthylester (Tetrahedron Lett. 1979, 861) in 75 ml Tetrahydrofuran zugetropft. Das Gemisch wurde noch 3,5 Stunden bei Raumtemperatur gerührt und dann nacheinander tropfenweise mit 15 ml Aceton und 20 ml gesättigter Natriumhydrogencarbonat-Lösung versetzt. Das Reaktionsgemisch wurde filtriert, das Filtrat eingeengt und der Rückstand (8,2 g) im Kugelrohr bei 150 °C/ca. 1 Torr destilliert. Hierbei wurden 6,5 g 2-(trans-1-Pentenyl)- 1,3-propandiol als farbloses Öl erhalten.

In analoger Weise wurden folgende Verbindungen hergestellt:

p-[trans-5-(trans-1-Propenyl)-m-dioxan-2-yl] benzonitril; Smp. (C–I) 97 °C, Klp. (N–I) 73 °C,

p-[trans-5-(trans-1-Butenyl)-m-dioxan-2-yl] benzonitril; Smp. (C–I) 92,2 °C,

p-[trans-5-(trans-1-Pentenyl)-m-dioxan-2-yl] benzonitril; Smp. (C–I) 67,2 °C, Klp. (N–I) 59,1 °C,

p-[trans-5-(trans-1-Hexenyl)-m-dioxan-2-yl] benzonitril; Smp. (C–I) 50,5 °C, Klp. (N–I) 37,0 °C,

p-[trans-5-(trans-1-Heptenyl)-m-dioxan-2-yl] benzonitril; Smp. (C–I) 49,3 °C, Klp. (N–I) 49,2 °C,

trans-2-(p-Äthoxyphenyl)-5-(trans-1-pentenyl)-m-dioxan; Smp. (C–I) 66,3 °C, Klp. (N–I) 64,4 °C,

trans-2-(p-Äthoxyphenyl)-5-(trans-1-hexenyl)-m-dioxan; Smp. (C–I) 56,7 °C, Klp. (N–I) 47,5 °C,

trans-2-(p-Butyloxyphenyl)-5-(trans-1-propenyl)-m-dioxan; Smp. (C–N) 58,5 °C, Klp. (N–I) 61,2 °C,
trans-2-(p-Butyloxyphenyl)-5-(trans-1-hexenyl)-m-dioxan; Smp. (C–N) 30,5 °C, $S_B$–N 30,3 °C, Klp. (N–I) 48,4 °C,
trans-2-(p-Propylphenyl)-5-(trans-1-propenyl)-m-dioxan; Smp. (C–I) 57,8 °C,
trans-2-(p-Propylphenyl)-5- (trans-1-pentenyl)-m-dioxan; Smp. (C–I) 45,2 °C, Klp. ($S_B$–I) 35,3 °C,
trans-2-(trans-4-Butylcyclohexyl)-5-(trans-1-propenyl)-m-dioxan; Smp. (C–N) 47,3 °C, Klp. (N–I) 48,4 °C,
trans-2-(trans-4-Propylcyclohexyl)-5-(trans-1-pentenyl)-m-dioxan; Smp. (C–$S_B$) 31,2 °C, Klp. ($S_B$–I) 85,7 °C.

**Patentansprüche**

1. Verbindungen der allgemeinen Formel

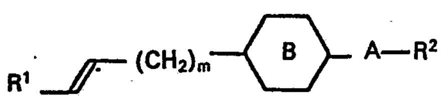

I

worin $R^1$ Wasserstoff oder geradkettiges Alkyl bedeutet; $R^2$ –CN, –R, –COR, –COOR oder an einem aromatischen Ring auch –OR, –OOCR oder Fluor darstellt und R Alkyl bezeichnet; A für eine Gruppe mit 1 bis 4 sechsgliedrigen Ringen steht, wobei diese Ringe untereinander und mit Ring B je über eine einfache Kovalenzbindung direkt verknüpft oder an einer oder gegebenenfalls 2 Stellen auch über –COO–, –OOC– oder $-CH_2CH_2-$ verknüpft sind; die sechsgliedrigen Ringe in A und Ring B 1,4-Phenylen oder trans-1,4-Cyclohexylen bedeuten oder einer dieser Ringe auch einen trans-2,5-disubstituierten m-Dioxanring, einen 2,5-disubstituierten Pyrimidinring oder einen 3,6-disubstituierten Pyridazinring bedeutet, und von benachbarten trans- 1,4- Cyclohexylen-Ringen höchstens je zwei über eine einfache Kovalenzbindung direkt verknüpft sind; um m die Zahl 2 oder, sofern Ring B trans-1,4-Cyclohexylen oder einen trans- 2,5- disubstituierten m-Dioxanring bedeutet, auch die Zahl 0 bezeichnet.

2. Verbindungen der allgemeinen Formel

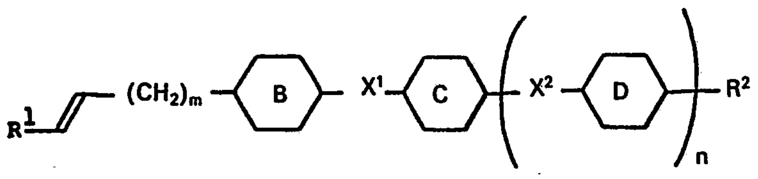

Ia

worin die Ringe B, C und D 1,4-Phenylen oder trans- 1,4-Cyclohexylen bedeuten oder einer dieser Ringe auch einen trans-2,5-disubstituierten m-Dioxanring, einen 2,5-disubstituierten Pyrimidinring oder einen 3,6-disubstituierten Pyridazinring bedeutet; eines der Symbole $X^1$ und $X^2$ eine einfache Kovalenzbindung und das andere –COO–, –OOC–, $-CH_2CH_2-$ oder, sofern mindestens einer der Ringe B, C und D von trans-1,4-Cyclohexylen verschieden ist, auch eine einfache Kovalenzbindung bezeichnet; n für die Zahl 0 oder 1 steht; und $R^1$, $R^2$, R und m die in Anspruch 1 gegebenen Bedeutungen haben.

3. Verbindungen nach Anspruch 1 oder 2, dadurch gekennzeichnet, dass Ring B trans-1,4-Cyclohexylen oder einen trans-2,5-disubstituierten m-Dioxanring bedeutet.

4. Verbindungen der allgemeinen Formel

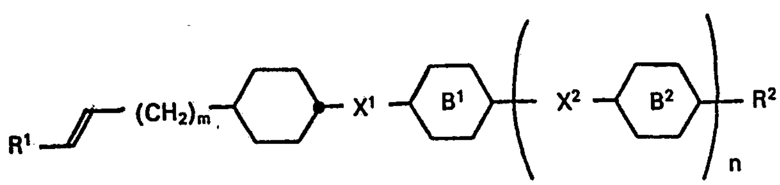

II

worin die Ringe $B^1$ und $B^2$ 1,4-Phenylen oder trans-1,4-Cyclohexylen bedeuten; eines der Symbole $X^1$ und $X^2$ eine einfache Kovalenzbindung und das andere –COO–, –OOC–, $-CH_2CH_2-$ oder, sofern mindestens einer der Ringe $B^1$ und $B^2$ von trans-1,4-Cyclohexylen verschieden ist, auch eine einfache Kovalenzbindung bezeichnet; n für die Zahl 0 oder 1 steht; und $R^1$, $R^2$, R und m die in Anspruch 1 gegebenen Bedeutungen haben.

5. Verbindungen nach Anspruch 4, dadurch gekennzeichnet, dass $X^1$ eine einfache Kovalenzbindung, –COO–, –OOC– oder $-CH_2CH_2-$ und $X^2$ eine einfache Kovalenzbindung bezeichnen und die Ringe $B^1$ und $B^2$ 1,4-Phenylen darstellen.

6. Verbindungen der allgemeinen Formel

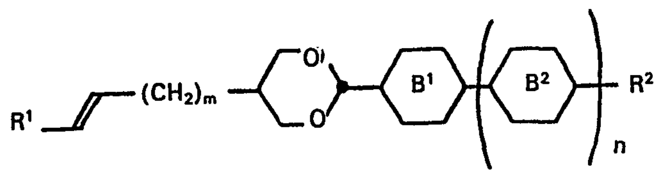

Ib

worin die Ringe $B^1$ und $B^2$ 1,4-Phenylen oder trans-1,4-Cyclohexylen bedeuten; n für die Zahl 0 oder 1 steht; und $R^1$, $R^2$, R und m die in Anspruch 1 gegebenen Bedeutungen haben.

7. Verbindungen nach Anspruch 6, dadurch gekennzeichnet, dass Ring $B^1$ 1,4-Phenylen bedeutet.

8. Verbindungen der allgemeinen Formel

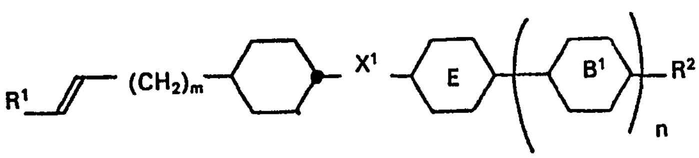

III

worin Ring E einen 2,5-disubstituierten Pyrimidinring und Ring$B^1$ 1,4-Phenylen oder trans-1,4-Cyclohexylen bedeutet; $X^1$ eine einfache Kovalenzbindung, –COO–, –OOC– oder $-CH_2CH_2-$ bezeichnet; n für die Zahl 0 oder 1 steht; und $R^1$, $R^2$, R und m die in Anspruch 1 gegebenen Bedeutungen haben.

9. Verbindungen nach Anspruch 8, dadurch gekennzeichnet, dass $X^1$ in Stellung 5 des Pyrimidinringes E steht und eine einfache Kovalenzbindung bedeutet.

10. Verbindungen nach einem der Ansprüche 2 bis 9, dadurch gekennzeichnet, dass n für die Zahl 0 steht.

11. Verbindungen nach einem der Ansprüche 1 bis 10, dadurch gekennzeichnet, dass R geradkettiges $C_1$–$C_{12}$-Alkyl bezeichnet.

12. Verbindungen nach einem der Ansprüche 1 bis 11, dadurch gekennzeichnet, dass $R^2$ Cyano, Alkyl oder an einem aromatischen Ring auch Alkoxy bedeutet.

13. Verbindungen nach Anspruch 12, dadurch gekennzeichnet, dass $R^2$ Cyano, $C_3$–$C_7$-Alkyl oder an einem aromatischen Ring auch $C_2$–$C_6$-Alkoxy bedeutet.

14. Verbindungen nach einem der Ansprüche 1 bis 13, dadurch gekennzeichnet, dass $R^1$ Wasserstoff oder geradkettiges $C_1$–$C_{10}$-Alkyl bedeutet, wenn m für die Zahl 0 steht, und $R^1$ Wasserstoff oder geradkettiges $C_1$–$C_8$-Alkyl bedeutet, wenn m für die Zahl 2 steht.

15. Verbindungen nach Anspruch 14, dadurch gekennzeichnet, dass $R^1$ Wasserstoff oder geradkettiges $C_1$–$C_5$-Alkyl bedeutet, wenn m für die Zahl 0 steht, und $R^1$ Wasserstoff oder geradkettiges $C_1$–$C_3$-Alkyl bedeutet, wenn m für die Zahl 2 steht.

16. Flüssigkristallines Gemisch mit mindestens 2 Komponenten, dadurch gekennzeichnet, dass mindestens eine Komponente eine Verbindung der in Anspruch 1 definierten Formel I ist.

17. Verfahren zur Herstellung der Verbindungen der in Anspruch 1 definierten Formel I, dadurch gekennzeichnet, dass man

a) zur Herstellung der Verbindungen der Formel I, worin $R^1$ primäres Alkyl mit mindestens 2 Kohlenstoffatomen bedeutet, $R^2$ –CN, –R oder an einem aromatischen Ring auch –OR oder Fluor darstellt und die in A vorkommenden Ringe untereinander und mit Ring B je über eine einfache Kovalenzbindung direkt verknüpft oder an einer oder gegebenenfalls 2 Stellen auch über $-CH_2CH_2-$ verknüpft sind, eine Verbindung der allgemeinen Formel

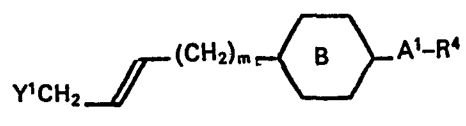

VI

worin $Y^1$ eine Abgangsgruppe bezeichnet; $R^4$ –CN, –R oder an einem aromatischen Ring auch –OR oder Fluor darstellt und R Alkyl bedeutet; $A^1$ für eine Gruppe mit 1 bis 4 sechsgliedrigen Ringen steht, wobei diese Ringe untereinander und mit Ring B je über eine einfache Kovalenzbindung direkt verknüpft oder an einer oder gegebenenfalls 2 Stellen auch über $-CH_2CH_2-$ verknüpft sind; die sechsgliedrigen Ringe in A und Ring B 1,4-Phenylen oder trans-1,4-Cyclohexylen bedeuten oder einer dieser Ringe auch einen trans-2,5-disubstituierten m-Dioxanring, einen 2,5-disubstituierten Pyrimidinring oder einen 3,6-disubstituierten Pyridazinring bedeutet, und von benachbarten trans- 1,4- Cyclohexylen-Ringen höchstens je zwei über eine einfache Kovalenzbindung direkt verknüpft sind; und m die Zahl 2 oder, sofern Ring B trans-1,4-Cyclohexylen oder einen trans-2,5-disubstituierten m-Dioxanring bedeutet, auch die Zahl 0 bezeichnet, in Gegenwart von Dilithiumtetrachlorkuprat oder Dilithiumtetrabromkuprat mit Alkylmagnesiumhalogenid umsetzt, oder

b) zur Herstellung der Verbindungen der Formel I, worin $R^1$ Methyl bedeutet, $R^2$ –CN, –R oder an einem aromatischen Ring auch –OR oder Fluor darstellt und die in A vorkommenden Ringe untereinander und mit Ring B je über eine einfache Kovalenzbindung direkt verknüpft oder an einer oder gegebenenfalls 2 Stellen auch über $-CH_2CH_2-$ verknüpft sind, eine Verbindung der Formel VI reduziert, oder

c) zur Herstellung der Verbindungen der Formel I, worin $R^2$ –COR darstellt und die in A vorkommenden Ringe untereinander und mit Ring B je über eine einfache Kovalenzbindung direkt verknüpft oder an einer oder gegebenenfalls 2 Stellen auch über $-CH_2CH_2-$ verknüpft sind, eine Verbindung der allgemeinen Formel

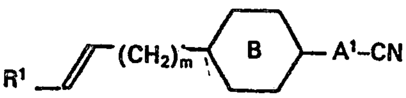 VII

worin $R^1$ Wasserstoff oder geradkettiges Alkyl bezeichnet und $A^1$, m und Ring B die in Formel VI gegebenen Bedeutungen haben, in Gegenwart von Dilithiumtetrachlorkuprat oder Dilithiumtetrabromkuprat mit Alkylmagnesiumhalogenid umsetzt, oder

d) zur Herstellung der Verbindungen der Formel I, worin A mindestens eine Gruppe –COO– oder –OOC– enthält und/oder $R^2$ –COOR, oder –OOCR darstellt, eine Verbindung der allgemeinen Formel

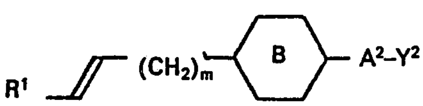 VIII

mit einer Verbindung der allgemeinen Formel

$Y^3-A^3-R^5$ IX

worin $R^1$ Wasserstoff oder geradkettiges Alkyl -edeutet; eine der Gruppen $Y^2$ und $Y^3$ –COOH oder –COCl und die andere –OH darstellt; $A^2$ und $A^3$ je für eine Gruppe mit 1 bis 4 sechsgliedrigen Ringen stehen oder eine der Gruppen $A^2$ und $A^3$ auch fehlen kann, mit der Massgabe, dass $A^2$ und $A^3$ zusammen höchstens 4 Ringe enthalten; Ring B gegebenenfalls mit in $A^2$ vorhandenen sechsgliedrigen Ringen und, sofern $A^2$ und/oder $A^3$ mehrere sechsgliedrige Ringe aufweisen, diese Ringe untereinander je über eine einfache Kovalenzbindung direkt verknüpft sind oder eine oder, sofern $A^3$ fehlt, gegebenenfalls auch 2 dieser Verknüpfungen über –COO–, –OOC oder $-CH_2CH_2-$ erfolgen; die sechsgliedrigen Ringe in $A^2$ und $A^3$ und Ring B 1,4-Phenylen oder trans-1,4-Cyclohexylen bedeuten oder einer dieser Ringe auch einen trans-2,5-disubstituierten m-Dioxanring, einen 2,5-disubstituierten Pyrimidinring oder einen 3,6-disubstituierten Pyridazinring bedeutet, und von benachbarten trans-1,4-Cyclohexylen-Ringen höchstens je zwei über eine einfache Kovalenzbindung direkt verknüpft sind; m die Zahl 2 oder, sofern Ring B trans-1,4-Cyclohexylen oder einen trans-2,5-disubstituierten m-Dioxanring bedeutet auch die Zahl 0 bezeichnet; und $R^5$, wenn $A^3$ für eine Gruppe mit 1 bis 4 sechsgliedrigen Ringen steht, –CN, –R, –COR, –COOR oder an einem aromatischen Ring auch –OR, –OOCR oder Fluor darstellt oder $R^5$, wenn $A^3$ fehlt, –R darstellt und R Alkyl bezeichnet, verestert, oder

e) zur Herstellung der Verbindungen der Formel I, worin $R^2$ –CN, –R oder an einem aromatischen Ring auch –OR oder Fluor darstellt und die in A vorkommenden Ringe untereinander und mit Ring B je über eine einfache Kovalenzbindung direkt verknüpft oder an einer oder gegebenenfalls 2 Stellen auch über $-CH_2CH_2-$ verknüpft sind, eine Verbindung der allgemeinen Formel

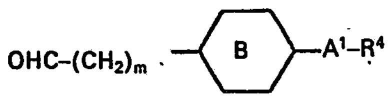 X

worin $A^1$, $R^4$, m und Ring B die in Formel VI gegebenen Bedeutungen haben, in Gegenwart einer Base mit Alkyltriphenylphosphoniumhalogenid umsetzt, oder

f) zur Herstellung der Verbindungen der Formel I, worin Ring B oder einer der sechsgliedrigen Ringe in A einen trans-2,5-disubstituierten m-Dioxanring bedeutet, eine Verbindung der allgemeinen Formel

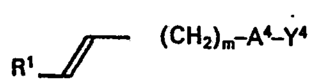 XXXV

mit einer Verbindung der allgemeinen Formel

$Y^5-A^5-R^2$ XXXVI

worin $R^1$ Wasserstoff oder geradkettiges Alkyl bedeutet; $R^2$ –CN, –R, –COR, –COOR oder an einem aromatischen Ring auch –OR, –OOCR oder Fluor darstellt und R Alkyl bezeichnet; eine der Gruppen $Y^4$ und $Y^5$ $-CH(CH_2OH)_2$ und die andere –CHO oder eine Acetalgruppe bedeutet; $A^4$ und $A^5$ je für eine Gruppe mit 1 bis 4 1,4-Phenylen- oder trans-1,4-Cyclohexylen-Ringen stehen oder eine der Gruppen $A^4$ und $A^5$ auch fehlen kann, mit der Massgabe, dass $A^4$ und $A^5$ zusammen höchstens 4 Ringe enthalten; $Y^4$ und $Y^5$ gegebenenfalls mit in $A^4$ bzw. in $A^5$ vorhandenen sechsgliedrigen Ringen und, sofern $A^4$ und/oder $A^5$ mehrere sechsgliedrige Ringe aufweisen, diese Ringe untereinander je über eine einfache Kovalenzbindung direkt verknüpft sind oder eine oder 2 dieser Verknüpfungen über –COO–, –OOC– oder $-CH_2CH_2-$ erfolgen; von benachbarten trans-1,4-

Cyclohexylen-Ringen höchstens je zwei über eine einfache Kovalenzbindung direkt verknüpft sind; und m die Zahl 2 bedeutet oder, wenn $A^4$ fehlt oder die Gruppe $R^1-CH=CH-(CH_2)_m-$ an einem trans-1,4-Cyclohexylen-Ring steht, auch die Zahl 0 bedeutet, umsetzt.

18. Verwendung der Verbindungen der in Anspruch 1 definierten Formel I für elektro-optische Zwecke.

**Revendications**

1. Composés de formule générale

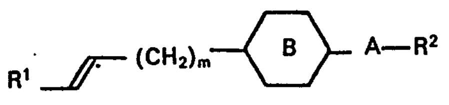

I

dans laquelle $R^1$ représente l'hydrogène ou un groupe alkyle à chaîne droite; $R^2$ représente –CN, –R, –COR, –COOR ou bien encore, sur un cycle aromatique, –OR, –OOCR ou le fluor et R représente un groupe alkyle; A représente un groupe contenant 1 à 4 cycles à 6 chaînons, ces cycles étant reliés entre eux et avec le cycle B dans chaque cas directement par une liaison covalente simple ou bien encore, en un ou le cas échéant, deux endroits, par l'intermédiaire de groupes –COO–, –OOC– ou $-CH_2CH_2-$; les cycles à 6 chaînons contenus dans A et le cycle B sont des cycles 1,4-phénylène ou trans-1,4-cyclohexylène, ou bien encore l'un de ces cycles peut consister en un cycle m-dioxanne trans-2,5-disubstitué, un cycle pyrimidine 2,5-disubstitué ou un cycle pyridazine 3,6-disubstitué, et dans le cas de cycles trans-1,4-cyclohexylène voisins, deux au maximum dans chaque cas sont reliés directement par une liaison covalente simple; et m est égal à 2 ou bien encore, lorsque le cycle B est un cycle trans-1,4-cyclohexylène ou un cycle m-dioxanne trans-2,5-disubstitué, à 0.

2. Composés de formule générale

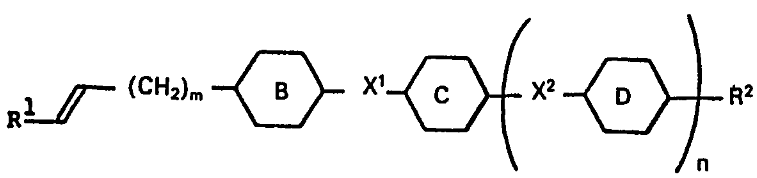

Ia

dans laquelle les cycles B, C et D sont des cycles 1,4-phénylène ou trans-1,4-cyclohexylène ou bien encore l'un de ces cycles est un cycle m-dioxanne trans-2,5-disubstitué, un cycle pyrimidine 2,5-disubstitué ou un cycle pyridazine 3,6-disubstitué; l'un des symboles $X^1$ et $X^2$ représente une liaison covalente simple et l'autre un groupe –COO–, –OOC–, $-CH_2CH_2-$ ou bien encore, lorsqu'au moins un des cycles B, C et D n'est pas un cycle trans-1,4-cyclohexylène, une liaison covalente simple; n est égal à 0 ou 1 et $R^1$, $R^2$, R et m ont les significations indiquées dans le rev. 1.

3. Composés selon la rev. 1 ou 2, caractérisés en ce que le cycle B est un cycle trans-1,4-cyclohexylène ou un cycle m-dioxanne trans-2,5-disubstitué.

4. Composés de formule générale

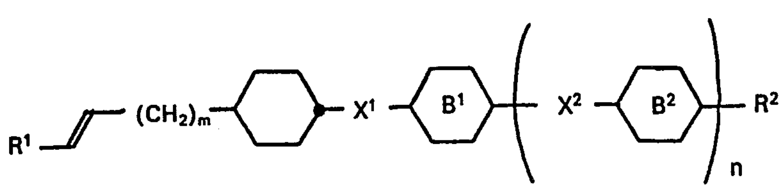

II

dans laquelle les cycles $B^1$ et $B^2$ sont des cycles 1,4-phénylène ou trans-1,4-cyclohexylène; l'un des symboles $X^1$ et $X^2$ représente une liaison covalente simple et l'autre un groupe –COO–, –OOC–, $-CH_2CH_2-$ ou bien encore, lorsqu'au moins un des cycles $B^1$ et $B^2$ n'est pas un cycle trans-1,4-cyclohexylène, une liaison covalente simple; n est égal à 0 ou 1 et $R^1$, $R^2$, R et m ont les significations indiquées dans la rev. 1.

5. Composés selon la rev. 4, caractérisés en ce que $X^1$ représente une liaison covalente simple, un groupe –COO–, –OOC– ou $-CH_2CH_2-$ et $X^2$ une liaison covalente simple, et les cycles $B^1$ et $B^2$ sont des cycles 1,4-phénylène.

6. Composés de formule générale

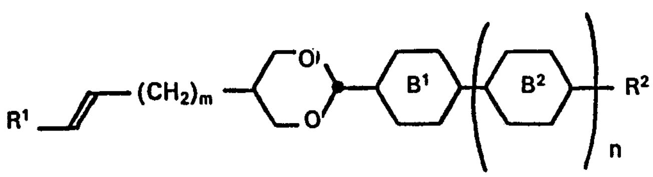

Ib

dans laquelle les cycles $B^1$ et $B^2$ sont des cycles 1,4-phénylène ou trans-1,4-cyclohexylène; n est égal à 0 ou 1, et $R^1$, $R^2$, R et m ont les significations indiquées dans la rev. 1.

7. Composés selon la rev. 6, caractérisé en ce que le cycle $B^1$ est un cycle 1,4-phénylène.

8. Composés de formule générale

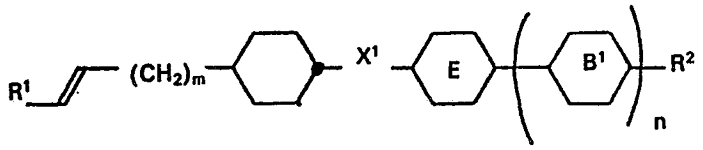

III

dans laquelle le cycle E est un cycle pyrimidine 2,5-disubstitué et le cycle $B^1$ un cycle 1,4-pheénylène ou trans-1,4-cyclohexylène; $X^1$ représente une liaison covalente simple, un groupe $-COO-$, $-OOC-$ ou $-CH_2CH_2-$; n est égal à 0 ou 1 et $R^1$, $R^2$, R et m ont les significations indiquées dans la rev. 1.

9. Composés selon la rev. 8, caractérisés en ce que $X^1$ est en position 5 du cycle pyrimidine E et représente une liaison covalente simple.

10. Composés selon l'une des rev. 2 à 9 caractérisés en ce que n est égal à 0.

11. Composés selon l'une des rev. 1 à 10, caractérisés en ce que R représente un groupe alkyle en C 1–C 12 à chaîne droite.

12. Composés selon l'une des rev. 1 à 11, caractérisés en ce que $R^2$ représente un groupe cyano, alkyle, ou bien encore, sur un cycle aromatique, un groupe alcoxy.

13. Composés selon la rev. 12, caractérisés en ce que $R^2$ représente un groupe cyano, alkyle en C 3–C 7, ou bien encore, sur un cycle aromatique, un groupe alcoxy en C 2–C 6.

14. Composés selon l'une des rev. 1 à 13, caractérisés en ce que $R^1$ représente l'hydrogène ou un groupe alkyle en C 1–C 10 à chaîne droite lorsque m est égal à 0, et $R^1$ représente l'hydrogène ou un groupe alkyle en C 1–C 8 à chaîne droite lorsque m est égal à 2.

15. Composés selon la rev. 14, caractérisés en ce que $R^1$ représente l'hydrogène ou un groupe alkyle à chaîne droite en C 1–C 5 lorsque m est égal à 0, et $R^1$ représente l'hydrogène ou un groupe alkyle à chaîne droite en C 1–C 3 lorsque m est égal à 2.

16. Mélange à cristaux liquides à au moins deux composants, caractérisé en ce que l'un au moins des composants consiste en un composé de formule I définie dans la rev. 1.

17. Procédé de préparation des composés de formule I définie dans la rev. 1, caractérisé en ce que:

a) pour préparer les composés de formule I dans laquelle $R^1$ représente un groupe alkyle primaire à au moins 2 atomes de carbone, $R^2$ représente $-CN$, $-R$ ou encore, sur un cycle aromatique, $-OR$ ou le fluor et les cycles présents dans A sont reliés entre eux et avec le cycle B dans chaque cas directement par une liaison covalente simple, ou bien, en un endroit ou le cas échéant, deux endroits, par l'intermédiaire d'un groupe $-CH_2CH_2-$, ou fait réagir un composé de formule générale

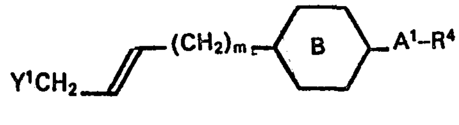

VI

dans laquelle $Y^1$ représente un groupe éliminable; $R^4$ représente $-CN$, $-R$ ou encore, sur un cycle aromatique, $-OR$ ou le fluor et R représente un groupe alkyle; $A^1$ représente un groupe contenant 1 à 4 cycles à 6 chaînons, ces cycles étant reliés entre eux et avec le cycle B dans chaque cas directement par une liaison covalente simple, ou bien encore, en un endroit ou les cas échéant, deux endroits, par l'intermédiaire d'un groupe $-CH_2CH_2-$; les cycles à 6 chaînons contenus dans A et le cycle B sont des cycles 1,4-phénylène ou trans-1,4-cyclohexylène, ou bien encore l'un de ces cycles est un cycle m-dioxanne trans-2,5-disubstitué, un cycle pyrimidine 2,5-disubstitué ou un cycle pyridazine 3,6-disubstitué, et dans le cas de cycles trans-1,4-cyclohexylène voisins, deux de ces cycles au maximum dans chaque cas peuvent être reliés directement par une liaison covalente simple; et m est égal à 2 ou bien encore, lorsque le cycle B est un cycle trans-1,4-cyclohexylène ou un cycle m-dioxanne trans-2,5-disubstitué, à 0, avec un halogénure d'alkyl-magnésium en présence de tétrachlorocuprate de dilithium ou de tétrabromocuprate de dilithium, ou bien

b) pour préparer les composés de formule I dans laquelle $R^1$ représente un groupe méthyle, $R^2$ représente $-CN$, $-R$ ou encore, sur un cycle aromatique, $-OR$ ou le fluor et les cycles présents dans A sont reliés entre eux et avec le cycle B dans chaque cas directement par une liaison covalente simple ou bien encore, en un endroit ou le cas échéant, deux endroits, par l'intermédiaire d'un groupe $-CH_2CH_2-$, on réduit un composé de formule VI, ou bien

c) pour préparer les composés de formule I dans laquelle $R^2$ représente $-COR$ et les cycles présents dans A sont reliés entre eux et avec le cycle B dans chaque cas directement par une liaison covalente simple ou bien encore, en un endroit ou le cas échéant, deux endroits, par l'intermédiaire d'un groupe $-CH_2CH_2-$, on fait réagir un composé de formule générale

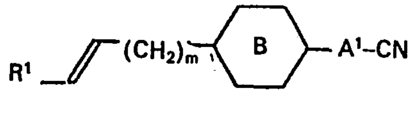

VII

dans laquelle $R^1$ représente l'hydrogène ou un groupe alkyle à chaîne droite et $A^1$, m et B ont les significations indiquées en référence à la formule VI, avec un halogénure d'alkyl-magnésium en présence de tétrachlorocuprate de dilithium ou de tétrabromocuprate de dilithium, ou bien

d) pour préparer les composés de formule I dans laquelle A contient au moins un groupe –COO– ou –OOC– et/ou $R^2$ représente –COOR ou –OOCR, on estérifie un composé de formule générale

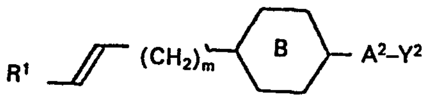
VIII

à l'aide d'un composé de formule générale

$Y^3–A^3–R^5$ IX

dans laquelle $R^1$ représente l'hydrogène ou un groupe alkyle à chaîne droite; l'un des symboles $Y^2$ et $Y^3$ représente –COOH ou –COCl et l'autre –OH; $A^2$ et $A^3$ représentent chacun un groupe contenant 1 à 4 cycles à 6 chaînons ou bien encore l'un des groupes $A^2$ et $A^3$ peut manquer, étant spécifié que $A^2$ et $A^3$ contiennent ensemble au maximum 4 cycles; le cycle B est éventuellement relié avec les cycles à 6 chaînons présents dans $A^2$ et, lorsque $A^2$ et/ou $A^3$ contiennent plusieurs cycles à 6 chaînons, ces cycles sont reliés entre eux dans chaque cas directement par une liaison covalente simple ou bien encore l'une de ces liaisons, ou bien, lorsque $A^3$ manque, éventuellement deux de ces liaisons, consistent en groupe –COO–, –OOC– ou $–CH_2CH_2–$; les cycles à 6 chaînons contenus dans $A^2$ et $A^3$ et le cycle B sont des cycles 1,4-phénylène ou trans-1,4-cyclohexylène ou bien encore l'un de ces cycles est un cycle m-dioxanne trans-2,5-disubstitué, un cycle pyrimidine 2,5-disubstitué ou un cycle pyridazine 3,6-disubstitué, et dans le cas de cycles trans-1,4-cyclohexylène voisins, deux au maximum dans chaque cas sont reliés directement par une liaison covalente simple; m est égal à 2 ou bien encore, lorsque le cycle B est un cycle 1,4-cyclohexylène ou un cycle m-dioxanne trans-2,5-disubstitué, à 0; et $R^5$ représente, lorsque $A^3$ est un groupe contenant 1 à 4 cycles à 6 chaînons, –CN, –R, –COR, –COOR ou bien encore, sur un cycle aromatique, –OR, –OOCR ou le fluor, ou bien $R^5$, lorsque $A^3$ manque, représente –R et R est un groupe alkyle, ou bien

e) pour préparer les composés de formule I dans laquelle $R^2$ représente –CN, –R ou bien encore, sur un cycle aromatique, –OR ou le fluor et les cycles présents dans A sont reliés entre eux et avec le cycle B dans chaque cas directement par une liaison covalente simple ou bien encore, en un endroit ou le cas échéant, en deux endroits, par l'intermédiaire d'un groupe $–CH_2CH_2–$, on fait réagir un composé de formule générale

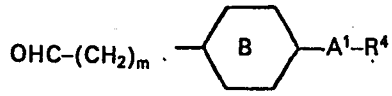
X

dans laquelle $A^1$, $R^4$, m et B ont les significations indiquées en référence à la formule VI, avec un halogénure d'alkyl-triphénylphosphonium en présence d'une base, ou bien

f) pour préparer les composés de formule I dans laquelle le cycle B ou l'un des cycles à 6 chaînons de A est un cycle m-dioxanne trans-2,5-disubstitué, on fait réagir un composé de formule générale

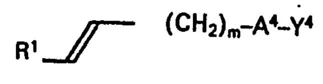
XXXV

avec un composé de formule générale

$Y^5–A^5–R^2$ XXXVI

dans laquelle $R^1$ représente l'hydrogène ou un groupe alkyle à chaîne droite; $R^2$ représente –CN, –R, –COR, –COOR ou bien encore, sur un cycle aromatique, fluor et R représente un groupe alkyle; l'un des groupes $Y^4$ et $Y^5$ est un groupe $–CH(CH_2OH)_2$ et l'autre un groupe –CHO ou un groupe acétal; $A^4$ et $A^5$ représentent chacun un groupe contenant 1 à 4 cycles 1,4-phénylène ou trans-1,4-cyclohexylène ou bien encore l'un des groupes $A^4$ et $A^5$ peut manquer, étant spécifié qu'$A^4$ et $A^5$ contiennent ensemble au maximum 4 cycles; $Y^4$ et $Y^5$ sont reliés éventuellement avec les cycles à 6 chaînons présents dans $A^4$ et/ou dans $A^5$ et, lorsque $A^4$ et/ou $A^5$ contiennent plusieurs cycles à 6 chaînons, ces cycles sont reliés entre eux dans chaque cas directement par une liaison covalente simple ou bien encore une ou deux de ces liaisons consistent en groupes –COO–, –OOC– ou $–CH_2CH_2–$; dans le cas de cycles trans-1,4-cyclohexylène voisins, deux au maximum dans chaque cas sont reliés directement par une liaison covalente simple; et m est égal à 2 ou bien encore, lorsque $A^4$ manque, ou lorsque le groupe $R^21–CH=CH–(CH_2)_m–$ est sur un cycle trans-1,4-cyclohexylène, à 0.

18. Utilisation des composés de formule I définie dans la rev. 1 dans des applications électro-optiques.

## Claims

1. Compounds of the general formula

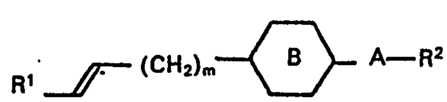
I

wherein $R^1$ signifies hydrogen or straight-chain alkyl; $R^2$ reprsents –CN, –R, –COR, –COOR or on an aromatic ring also –OR, –OOCR or fluorine and R denotes alkyl; A stands for a group with 1 to 4 six-membered rings, whereby these rings are directly linked with one another and with ring B in each case via a single covalent bond or are linked in one or optionally 2 positions also via –COO–, –OOC– or $-CH_2CH_2-$; the six-membered rings in A and ring B signify 1,4-phenylene or 1,4-cyclohexylene or one of these rings also signifies a trans-2,5-disubstituted m-dioxane ring, a 2,5-disubstituted pyrimidine ring or a 3,6-disubstituted pyridazine ring, and of adjacent trans-1,4-cyclohexylene rings a maximum of in each case two are directly linked via a single covalent bond; and m denotes the number 2 or, insofar as ring B signifies trans-1,4-cyclohexylene or a trans-2,5-disubstituted m-dioxane ring, also the number 0.

2. Compounds of the general formula

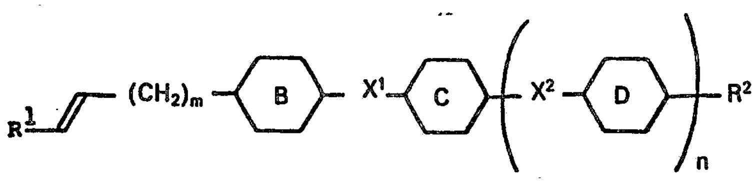

Ia

wherein the rings B, C and D signify 1,4-phenylene or trans-1,4-cyclohexylene or one of these rings also signifies a trans-2,5-disubstituted m-dioxane ring, a 2,5-disubstituted pyrimidine ring or a 2,6-disubstituted pyridazine ring; one of the symbols $X^1$ and $X^2$ denotes a single covalent bond and the other denotes –COO–, –OOC–, $-CH_2CH_2-$ or, insofar as at least one of the rings B, C and D is different from trans-1,4-cyclohexylene, also a single covalent bond; n stands for the number 0 or 1; and $R^1$, $R^2$, R and m have the significances given in claim 1.

3. Compounds according to claim 1 or 2, characterized in that ring B signifies trans-1,4-cyclohexylene or a trans-2,5-disubstituted m-dioxane ring.

4. Compounds of the general formula

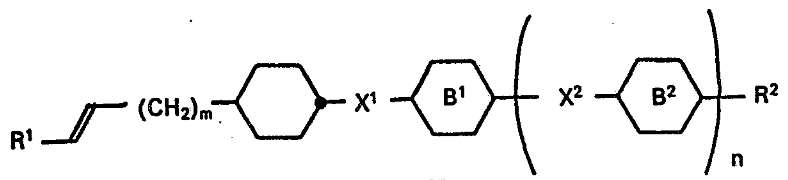

II

wherein the rings $B^1$ and $B^2$ signify 1,4-phenylene or trans-1,4-cyclohexylene; one of the symbols $X^1$ and $X^2$ denotes a single covalent bond and the other denotes –COO–, –OOC–, $-CH_2CH_2-$ or, insofar as at least one of the rings $B^1$ and $B^2$ is diffferent from trans-1,4-cyclohexylene, also a single covalent bond; n stands for the number 0 or 1; and $R^1$, $R^2$, R and m have the significances given in claim 1.

5. Compounds according to claim 4, characterized in that $X^1$ denotes a single covalent bond, –COO–, –OOC– or $-CH_2CH_2-$ and $X^2$ denotes a single covalent bond and the rings $B^1$ and $B^2$ represent 1,4-phenylene.

6. Compounds of the general formula

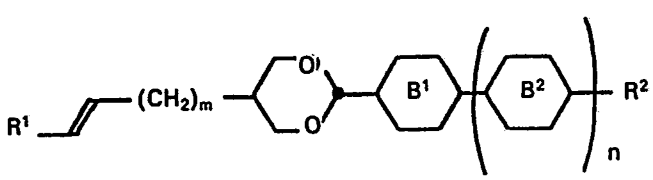

Ib

wherein the rings $B^1$ and $B^2$ signify 1,4-phenylene or trans-1,4-cyclohexylene; n stands for the number 0 or 1; and $R^1$, $R^2$, R and m have the significances given in claim 1.

7. Compounds according to claim 6, characaterized in that ring $B^1$ signifies 1,4-phenylene.

8. Compounds of the formula

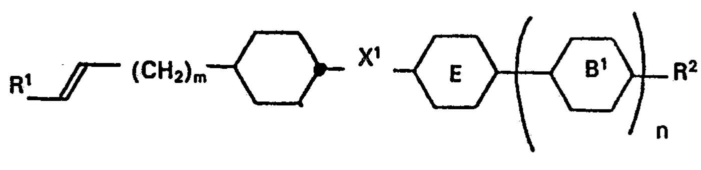

III

wherein ring E signifies 1 2,5-disubstituted pyrimidine ring and ring $B^1$ signifies 1,4-phenylene or trans-1,4--cyclohexylene; $X^1$ denotes a single covalent bond, –COO–, –OOC– or $-CH_2CH_2-$; n stands for the number 0 or 1; and $R^1$, $R^2$, R and m have the snigificances given in claim 1.

9. Compounds according to claim 8, characterized in that $X^1$ is present in position 5 of the pyrimidine ring and signifies a single covalent bond.

10. Compounds according to any one of claims 2 to 9, characterized in that n stands for the number 0.

11. Compounds according to any one of claims 1 to 10, characterized in that R denotes straight-chain $C_1$–$C_{12}$-alkyl

12. Compounds according to any one of claims 1 to 11, characterized in that $R^2$ signifies cyano, alkyl or on an aromatic ring also alkoxy.

13. Compounds according to claim 12, characterized in that $R^2$ signifies cyano, $C_3$–$C_7$-alkyl or on an aromatic ring also $C_2$–$C_6$-alkoxy.

14. Compounds according to any one of claims 1 to 13, characterized in that $R^1$ signifies hydrogen or straight-chain $C_1$–$C_{10}$-alkyl when m stands for the number 0 and $R^1$ signifies hydrogen or straight-chain $C_1$–$C_8$-alkyl when m stands for the number 2.

15. Compounds according to claim 14, characterized in that $R^1$ signifies hydrogen or straight-chain $C_1$–$C_5$-alkyl when m stands for the number 0 and $R^1$ signifies hydrogen or straight-chain $C_1$–$C_3$-alkyl when m stands for the number 2.

16. A liquid crystalline mixture with at least 2 components, characterized in that at least one component is a compound of formula I defined in claim 1.

17. A process for the manufacture of the compounds of formula I defined in claim 1, characterized by

a) for the manufacture of the compounds of formula I in which $R^1$ signifies primary alkyl with at least 2 carbon atoms, $R^2$ represents –CN, –R or on an aromatic ring also –OR or fluorine and the rings present in A are directly linked with one another and with ring B in each case via a single covalent bond or are linked in one or optionally 2 positions also via $-CH_2CH_2-$, reacting a compound of the general formula

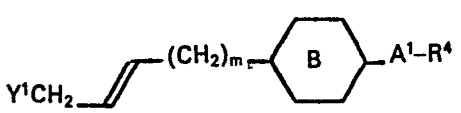

VI

wherein $Y^1$ denotes a leaving group; $R^4$ represents –CN, –R or on an aromatic ring also –OR or fluorine and R signifies alkyl; $A^1$ stands for a group with 1–4 six-membered rings, whereby these rings are directly linked with one another and with ring B in each case via a single covalent bond or are linked in one or optionally 2 positions also via $-CH_2CH_2-$; the six-membered rings in A and ring B signify 1-4-phenylene or trans-1,4-cyclohexylene or one of these rings also signifies a trans-2,5-disubstituted m-dioxane ring, a 2,5-disubstituted pyrimidine ring or a 3,6-disubstituted pyridazine ring, and of adjacent trans-1,4-cyclohexylene rings a maximum of in each case two are directly linked via a single covalent bond; and m denotes the number 2 or, insofar as ring B signifies trans-1,4-cyclohexylene or a trans-2,5-disubstituted m-dioxane ring, also the number 0, with alkylmagnesium halide in the presence of dilithium tetrachlorocuprate or dilithium tetrabromocuprate, or

b) for the manufacture of the compounds of formula I in which $R^1$ signifies methyl, $R^2$ represents –CN, –R or on an aromatic ring also –OR or fluorine and the rings present in A are directly linked with one another and with ringB in each case via a single covalent bond or are linked in one or optionally 2 positions also via $-CH_2CH_2-$, reducing a compound of formula VI, or

c) for the manufacture of the compounds of formula I in which $R^2$ represents –COR and the rings present in A are directly linked with one another and with ring B in each case via a single covalent bond or are linked in one or optionally 2 positions also via $-CH_2CH_2-$, reacting a compound of the general formula

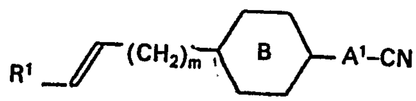

VII

wherein $R^1$ denotes hydrogen or straight-chain alkyl and $A^1$, m and ring B have the significances given in formula VI, with alkylmagnesium halide in the presence of dilithium tetrachlorocuprate or dilithium tetrabromocuprate, or

d) for the manufacture of the compounds of formula I in which A contains at least one group –COO– or –OOC– and/or $R^2$ represents –COOR or –OOCR, esterifying a compound of the general formula

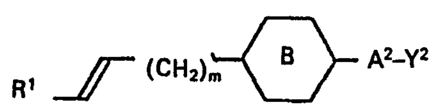

VIII

with a compound of the general formula

$Y^3$–$A^3$–$R^5$ IX

wherein $R^1$ signifies hydrogen or straight-chain alkyl; one of the groups $Y^2$ and $Y^3$ represents –COOH or –COCl and the other represents –OH; $A^2$ and $A^3$ each stand for a groupe with 1 to 4 six-membered rings or one of the groups $A^2$ and $A^3$ can also be absent, with the proviso that $A^2$ and $A^3$ together contain a maximum of 4 rings; ring B is linked optionally with six-membered rings present in $A^2$ and, insofar as $A^2$ and/or $A^3$ have more than one six-membered ring, these rings are linked with one another in each case directly via a

single covalent bond or one or, insofar as $A^3$ is absent, optionally also 2 of these linkages is/are effected via $-COO-$, $-OOC$ or $-CH_2CH_2-$; the six-membered rings in $A^2$ and $A^3$ and ring B signify 1,4-phenylene or trans-1,4-cyclohexylene or one of these rings also signifies a trans-2,5-disubstituted m-dioxane ring, a 2,5-disubstituted pyrimidine ring or a 3,6-disubstituted pyridazine ring, and of adjacent trans-1,4-cyclohexylene rings a maximum of in each case two are directly linked via a single covalent bond; m denotes the number 2 or, insofar as ring B signifies trans-1,4-cyclohexylene or a trans-2,5-disubstituted m-dioxane ring, also the number 0; and $R^5$ represents $-CN$, $-R$, $-COR$, $-COOR$ or on an aromatic ring also $-OR$, $-OOCR$ or fluorine when $A^3$ stands for a group with 1 to 4 six-membered rings or $R^5$ represents $-R$ when $A^3$ is absent and R denotes alkyl. or

e) for the manufacture of the compounds of formula I in which $R^2$ represents $-CN$, $-R$ or on an aromatic ring also $-OR$ or fluorine and the rings present in A are directly linked with one another and with ring B in each case via a single covalent bond or are linked in one or optionally 2 positions also via $-CH_2CH_2-$, reacting a compound of the general formula

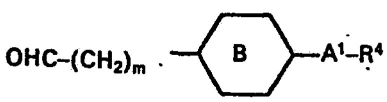

X

wherein $A^1$, $R^4$, m and ring B have the significances given in formula VI, with alkyltriphenylphosphonium halide in the presence of a base, or

f) for the manufacture of the compounds of formula I in which ring B or one of the six-membered rings in A signifies a trans-2,5-disubstituted m-dioxane ring, reacting a compound of the general formula

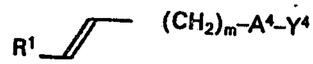

XXXV

with a compound of the general formula

$Y^5-A^5-R^2$ XXXVI

wherein $R^1$ signifies hydrogen or straight-chain alkyl; $R^2$ represents $-CN$, $-R$, $-COR$, $-COOR$ or on an aromatic ring also $-OR$, $-OOCR$ or fluorine and R denotes alkyl; one of the groups $Y^4$ and $Y^5$ signifies $-CH(CH_2OH)_2$ and the other signifies $-CHO$ or an acetal group; $A^4$ and $A^5$ each stand for a group with 1 to 4 1,4-phenylene or trans-1,4-cyclohexylene rings or one of the groups $A^4$ and $A^5$ can also be absent, with the proviso that $A^4$ and $A^5$ together contain a maximum of 4 rings; $Y^4$ and $Y^5$ are linked optionally with six-membered rings present in $A^4$ or in $A^5$ and, insofar as $A^4$ and/or $A^5$ have more than one six-membered ring, these rings are linked with one another in each case directly via a single covalent bond or one or 2 of these linkages is/are effected via $-COO-$, $-OOC-$ or $-CH_2CH_2-$; of adjacent trans-1,4-cyclohexylene rings a maximum of in each case two are directly linked via a single covalent bond; and m signifies the number 2 or, when $A^4$ is absent or the group $R^1-CH=CH-CH_2)_m-$ is present on a trans-1,4-cyclohexylene ring, also the number 0.

18. The use of the compounds of formula I defined in claim 1 for electro-optical purposes.